# EUROPEAN PATENT SPECIFICATION

(11) **EP 0 592 438 B1**
(45) Date of publication and mention of the grant of the patent: **27.08.1997**
(21) Application number: 91920239.0
(22) Date of filing: 08.10.1991
(51) Int. Cl.: C07D 403/06, A61K 31/40, C07D 401/06

(54) **INDOLE DERIVATIVES**
INDOLDERIVATE
DERIVES D'INDOLE

(30) Priority: 15.10.1990 US 597928
(43) Date of publication of application: 20.04.1994
(73) Proprietor: PFIZER INC., New York, N.Y. 10017 (US)
(72) Inventor: MACOR, John, Eugene, Mystic, CT 06355 (US); WYTHES, Martin James, Dover, Kent (GB)
(74) Representative: Ruddock, Keith Stephen
(86) International application number: US9107194
(87) International publication number: WO9206973

(56) References cited:
- GB-A- 851 780
- GB-A- 886 684
- GB-A- 893 707
- GB-A- 966 562
- GB-A- 2 081 717
- NL-C- 74 527
- NL-C- 74 786
- US-A- 2 773 875
- US-A- 3 037 031
- US-A- 4 803 218
- JOURNAL OF THE AMERICAN CHEMICAL SOCIETY, vol. 79, 1957, Columbus, OH (US); H. BADER et al.., pp. 5686-5689
- JOURNAL OF ORGANIC CHEMISTRY, vol. 23, 1958, Columbus, OH (US); A.P. GRAY, pp. 1453-1454
- JOURNAL OF ORGANIC CHEMISTRY, vol. 29, 1964, Columbus, OH (US); J.A. MOORE et al., pp. 2860-2864
- JOURNAL OF THE AMERICAN CHEMICAL SOCIETY, vol. 105, 1983, Columbus, OH (US); M. CAIN et al., pp. 907-913
- TETRAHEDRON, vol. 38, no. 1, 1982, Pergamon Press Ltd. (GB); N. MOHR et al., pp. 147-152, compounds 11, 12
- CHEMICAL ABSTRACTS, vol. 83, 1975, Columbus, OH (US); E. FRIDERICHS et al., p. 492, AN 28056k

## Description

### Background of the Invention

The present invention relates to indole derivatives, to processes and intermediates for their preparation, to pharmaceutical compositions containing them and to their medicinal properties. The active compounds of the present invention are useful in treating migraine and other disorders.

United States Patents 4,839,377 and 4,855,314 and European Patent Application Publication Number 313397 refer to 5-substituted 3-aminoalkyl indoles. The compounds are said to be useful for the treatment of migraine.

British Patent Application 040279 refers to 3-aminoalkyl-1H-indole-5-thioamides and carboxamides. The compounds are said to be useful in treating hypertension, Raymond's disease and migraine.

European Patent Application Publication Number 303506 refers to 3-poly:hydro-pyridyl-5-substituted-1H-indoles. The compounds are said to have 5HT1-receptor agonist and vasoconstrictor activity and to be useful in treating migraine.

European Patent Application Publication Number 354777 refers to N-piperidinyl:indolyl:ethyl-alkane sulfonamide derivatives. The compounds are said to have 5HT1-receptor agonist and vasoconstrictor activity and to be useful in treating cephalic pain.
NL-C-74786 discloses 3-(piperidin-2-ylmethyl)indole derivatives having pharmacological activity.
NL-C-74527 describes 3-(piperidin-1-ylmethyl)indole derivatives having oxytocic activity.
J.A.C.S., 79, 5686-9 (1957) discloses the preparation of 3-(piperidin-2-ylmethyl)indole derivatives.
J.Org.Chem., 23, 1453-4(1958) describes the preparation of 3-(piperidinylmethyl)indole derivatives.
J.Org.Chem., 29, 2860-4(1964) discloses the preparation of, inter alia, 5-hydroxy-3-(1-methylpiperidin-2-ylmethyl)indole and a picrate salt thereof.
J.A.C.S., 105, 907-913 (1983) relates to the preparation of 3-(piperidin-2-ylmethyl)indole and 3-(piperidin-2-ylcarbonyl) indole derivatives.
GB-A-851,780 describes the preparation of, inter alia, 3-(piperidin-2-ylmethyl)indole and a picrate salt thereof. US-A-2,773,875 relates to 3-(piperidin-2-ylmethyl)indole derivatives with oxytocic activity.
Tetrahedron, 38(1), 147-152(1982) describes the preparation of 3-(pyrrolidin-2-ylcarbonyl)indole derivatives. Chem.Abs., 83, 28056k(1975) describes the preparation of 5-hydroxy-3-(piperidinylmethyl)indole derivatives.

### Summary of the Invention

The present invention relates to compounds of the formula 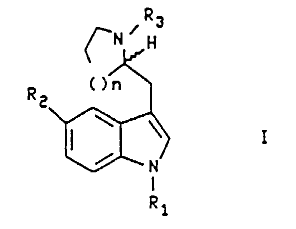 wherein n is 0, 1, or 2; R₁ is hydrogen; R₂ is selected from hydrogen, halogen (e.g., fluorine, chlorine, bromine or iodine), cyano, OR₄, -(CH₂)ₘ-(C=O) NR₅R₆, -(CH₂)ₘ-SO₂NR₅R₆, -(CH₂)ₘ-NR₇(C=O) R₈, -(CH₂)ₘ-NR₇SO₂R₈, -(CH₂)ₘ-S(O)ₓR₈, -(CH₂)ₘ-NR₇(C=O)NR₅R₆, -(CH₂)ₘ-NR (C=O)OR₉, and -CH=CH(CH₂)_{y}R₁₀; R₃ is selected from hydrogen and C₁ to C₆ linear or branched alkyl; R₄ is selected from hydrogen, C₁ to C₆ alkyl, and aryl; R₅ and R₆ are independently selected from hydrogen, C₁ to C₆ alkyl, aryl, and C₁ to C₃ alkyl-aryl or R₅ and R₆ taken together form a 4, 5, or 6 membered ring; R₇ and R₈ are independently selected from hydrogen, C₁ to C₆ alkyl, aryl, and C₁ to C₃ alkyl-aryl; R₉ is selected from hydrogen, C₁ to C₆ alkyl, aryl, and C₁ to C₃ alkyl-aryl; R₁₀ is selected from -(C=O)NR₅R₆ and -SO₂NR₅R₆, wherein R₅ and R₆ are defined as above, and -NR₇(C=O)R₈, -NR₇SO₂R₈, -NR₇(C=O)NR₅R₆, -S(O)ₓR₈ and -NR₇(C=O)OR₉, wherein R₇, R₈, and R₉ are as defined above; m is 0, 1, 2, or 3; y is 0, 1, or 2; x is 1 or 2; and the above aryl groups and the aryl moieties of the above alkylaryl groups are independently selected from phenyl and substituted phenyl, wherein said substituted phenyl may be substituted with one to three groups selected from C₁ to C₄ alkyl, halogen (e.g., fluorine, chlorine, bromine or iodine), hydroxy, cyano, carboxamido, nitro and C₁ to C₄ alkoxy and the pharmaceutically acceptable salts thereof with the provisos that:
(i) when R₁ and R₂ are hydrogen and n is 2, R₃ is not hydrogen or C₁-C₆ linear or branched alkyl when the compound of the formula (I) is a free base;
(ii) when R₁ and R₂ are hydrogen, R₃ is methyl and n is 2, the compound of the formula (I) is not a pharmaceutically acceptable acid addition salt thereof;
(iii) when R₁, R₂ and R₃ are hydrogen and n is 2, the pharmaceutically acceptable salt of the compound of the formula (I) is not the hydrochloride or picrate;
(iv) when R₁ is hydrogen, R₂ is hydroxy and R₃ is methyl, n is not 2 when the compound of the formula (I) is a free base or the pharmaceutically acceptable salt is the picrate or ½ oxalate; and
(v) when R₁ and R₃ are hydrogen and R₂ is hydroxy, n is not 2 when the compound of the formula (I) is a free base or the pharmaceutically acceptable salt is the picrate or hydrogensuccinate.

These compounds are useful in treating migraine and other disorders. Compounds of the formula I wherein R₂ is -CH=CH-R₁₀ are also useful as intermediates for preparing other compounds of the formula I.

The compounds of the invention include all optical isomers of formula I (e.g., R and S enantiomers) and their racemic mixtures. The R enantiomers at the designated chiral site in formula I are preferred.

Unless otherwise indicated, the alkyl groups referred to herein, as well as the alkyl moieties of other groups referred to herein (e.g. alkoxy), may be linear or branched, and they may also be cyclic (e.g., cyclopropyl, cyclobutyl, cyclopentyl or cyclohexyl) or be linear or branched and contain cyclic moieties.

Preferred compounds of the invention are compounds of the formula I wherein R₁ is hydrogen; R₂ is -(CH₂)ₘ-SO₂NR₅, -(CH₂)ₘ-NHSO₂R₈, -(CH₂)ₘ-SO₂R₈, -(CH₂)ₘ-(C=O)NHR₅, or -(CH₂)ₘ-NH(C=O)R₈; R₃ is hydrogen or methyl; and m, R₅ and R₈ are as defined above and the pharmaceutically acceptable salts thereof. Of the foregoing preferred compounds, the R enantiomers at the designated chiral site in formula I are more preferred.

The following compounds are particularly preferred:
(R)-5-methoxy-3-(N-methylpyrrolidin-2-ylmethyl)-1H-indole;
(R)-5-bromo-3-(N-methylpyrrolidin-2-ylmethyl)-1H-indole;
(R)-5-(2-ethylsulfonylethyl)-3-(N-methylpyrrolidin-2-ylmethyl) -1H-indole;
(R)-5-(2-methylaminosulfonylethyl)-3-(N-methylpyrrolidin-2-ylmethyl)-1H-indole;
(R)-5-(2-methylaminosulfonylethyl)-3-(pyrrolidin-2-ylmethyl)-1H-indole;
(R)-5-(methylaminosulfonylmethyl)-3-(N-methylpyrrolidin-2-ylmethyl)-1H-indole;
(R)-5-carboxamido-3-(N-methylpyrrolidin-2-ylmethyl)-1H-indole;
(R)-5-(2-methylsulfonylethyl)-3-(N-methylpyrrolidin-2-yl-methyl)-1H-indole;
(R)-5-(2-aminosulphonylethenyl)-3-(N-methylpyrrolidin-2-ylmethyl)-1H-indole;
(R)-5-(2-aminosulphonylethyl)-3-(N-methylpyrrolidin-2-ylmethyl)-1H-indole;
(R)-5-(2-N,N-dimethylaminosulphonylethyl)-3-(N-methylpyrrolidin-2-ylmethyl)-1H-indole;
(R)-5-(2-phenylsulphonylethyl)-3-(N-methylpyrrolidin-2-ylmethyl)-1H-indole;
(R)-5-(2-phenylsulphonylethyl)-3-(N-methylpyrrolidin-2-ylmethyl)-1H-indole hemisuccinate;
(R)-5-(2-ethylsulphonylethyl)-3-(N-methylpyrrolidin-2-ylmethyl)-1H-indole hemisuccinate;
(R) -5-(3-benzenecarbonylaminoprop-1-enyl)-3-(N-methylpyrrolidin-2-ylmethyl)-1H-indole;
(R)-5-(2-(4-methylphenylsulphonyl)ethyl)-3-(N-methylpyrrolidin-2-ylmethyl)-1H-indole;
(R)-5-(3-methylsulphonylaminoprop-1-enyl)-3-(N-methylpyrrolidin-2-ylmethyl)-1H-indole;
(R)-5-(2-ethylsulphonylethyl)-3-(N-2-propylpyrrolidin-2-ylmethyl)-1H-indole;
(R)-5-(2-ethylsulphonylethyl)-3-(pyrrolidin-2-ylmethyl)-1H-indole;
(R)-5-(2-(4-methylphenylsulphonyl)ethenyl)-3-(N-methylpyrrolidin-2-ylmethyl)-1H-indole;
(R)-5-(2-methylsulfonamidoethyl)-3-(N-methylpyrrolidin-2-ylmethyl)-1H-indole; and
(R)-5-(2-methylsulfonamidomethyl)-3-(N-methylpyrrolidin-2-ylmethyl)-1H-indole.

The following are other specific compounds of the present invention:
(R)-3-(N-methylpyrrolidin-2-ylmethyl)-1H-indole;
(R)-5-fluoro-3-(N-methylpyrrolidin-2-ylmethyl)-1H-indole;
(R)-5-acetylamino-3-(N-methylpyrrolidin-2-ylmethyl)-1H-indole;
(R)-5-benzyloxycarbonylamino-3-(N-methylpyrrolidin-2-ylmethyl)-1H-indole;
(R)-5-(2-aminocarbonylethyl)-3-(N-methylpyrrolidin-2-ylnethyl)-1H-indole;
(R)-5-aminocarbonylmethyl-3-(N-methylpyrrolidin-2-ylmethyl)-1H-indole;
(R)-5-methylsulfonamido-3-(N-methylpyrrolidin-2-ylmethyl)-1H-indole; and
(R)-5-aminosulfonyl-3-(N-methylpyrrolidin-2-ylmethyl)-1H-indole.

The present invention also relates to a pharmaceutical composition for treating a condition selected from hypertension, depression, anxiety, eating disorders, obesity, drug abuse, cluster headache, migraine, pain, and chronic paroxysmal hemicrania and headache associated with vascular disorders comprising an amount of a compound of the formula I or a pharmaceutically acceptable salt thereof effective in treating such a condition and a pharmaceutically acceptable carrier.

The present invention also relates to a pharmaceutical composition for treating disorders arising from deficient serotonergic neurotransmission *(e.g.,* depression, anxiety, eating disorders, obesity, drug abuse, cluster headache, migraine, pain, and chronic paroxysmal hemicrania and headache associated with vascular disorders) comprising an amount of a compound of the formula I or a pharmaceutically acceptable salt thereof effective in treating such a disorder and a pharmaceutically acceptable carrier.

The present invention also relates to the use of a compound of the formula (I) or of a pharmaceutically acceptable salt thereof for the manufacture of a medicament for treating a condition selected from hypertension, depression, anxiety, eating disorders, obesity, drug abuse, cluster headache, migraine, pain and chronic paroxysmal hemicrania and headache associated with vascular disorders.

The present invention also relates to the use of a compound of the formula (I) or of a pharmaceutically acceptable salt thereof for the manufacture of a medicament for treating disorders arising from deficient serotonergic neurotransmission (e.g., depression, anxiety, eating disorders, obesity, drug abuse, cluster headache, migraine, pain and chronic paroxysmal hemicrania and headache associated with vascular disorders).

The present invention also relates to a compound of the formula 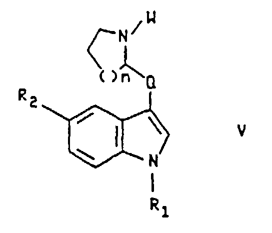 wherein W is -CO₂R₁₁ or R₃; Q is CH₂ or C=O; n, R₁, R₂ and R₃ are as defined for formula I; and R₁₁ is selected from C₁ to C₆ alkyl, benzyl and aryl, wherein aryl is as defined above, with the provisos that:
(i) when Q is CH₂, W is not R₃;
(ii) when R₁ and R₂ are hydrogen, Q is C=O and n is 2, W is not hydrogen or neo-pentyl; and
(iii) when R₁ and R₂ are hydrogen, Q is C=O and n is 1, W is not hydrogen or benzyloxycarbonyl.
The compounds of formula V are useful as intermediates in preparing compounds of the formula I.

Accordingly, one group of the foregoing intermediates comprises compounds of the formula 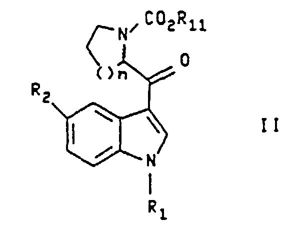 wherein n, R₁, R₂ and R₁₁ are as defined above and a second group of the foregoing intermediates comprises compounds of the formula 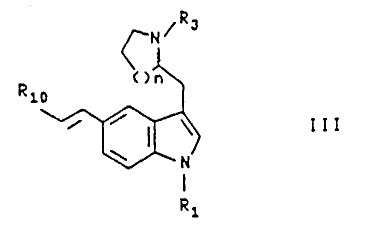 wherein n, R₁, R₃ and R₁₀ are as defined above.

### Detailed Description of the Invention

Compounds of formula I are prepared by hydride reduction of a compound of the formula 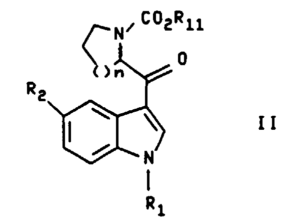 wherein R₁, R₂, n and R₁₁ are as defined above with a hydride reducing agent in an inert solvent. Suitable hydride reducing agents include lithium aluminum hydride, diborane, lithium borohydride and sodium borohydride. The preferred reagent is lithium aluminum hydride. Suitable solvents include ethers, such as diethyl ether, tetrahydrofuran, 1,4-dioxane and 1,2-dimethoxyethane. The preferred solvent is tetrahydrofuran. The reaction is conducted at a temperature of about 30°C to about 100°C., preferably about 65°C to about 70°C.

Compounds of formula I are also prepared by catalytic reduction of a compound of the formula 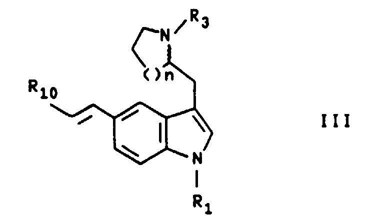 wherein R₁, R₃, n and R₁₀ are as defined above under an atmosphere of hydrogen, preferably at a pressure of about 1 to about 3 atmospheres, or using a hydrogen source such as ammonium formate or formic acid in an inert solvent. Suitable catalysts include palladium on carbon, Raney nickel, platinum oxide, rhodium, and ruthenium. The preferred catalyst is palladium on carbon. Suitable solvents include C₁ to C₆ alcohols, N,N-dimethylformamide, ethyl acetate, and acetonitrile. The preferred solvent is ethanol. The reaction is conducted at a temperature of about 0°C to about 60°C, most preferably at about 25°C.

Compounds of formula I are also prepared by alkylation of compounds of formula I where R₃=H and R₂ and R₁, are as defined for formula I with alkyl halides in the presence of a base in an inert solvent. Suitable alkyl halides include alkyl halides R³ - Halide where the halide is chloride, bromide and iodide. The preferred halide is iodide, or bromide in the presence of a suitable iodide source such as sodium iodide. Suitable bases include tertiary amines and inorganic bases. The preferred base is sodium carbonate. Suitable solvents include N,N-dimethylacetamide, N,N-dimethylformamide, dimethoxyethane, tetrahydrofuran, dichloromethane, acetonitrile. The preferred solvent is N,N-dimethylacetamide. The reaction is conducted at a temperature of about 0°C to about 150°C, preferably at about 120°C.

The compounds of formula II can be prepared by reacting a magnesium salt of an indole derivative of the formula 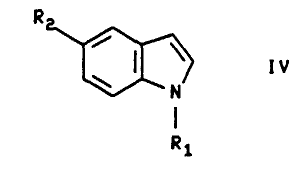 wherein R₁ and R₂ are defined above, with the acid chloride of an N-CO₂R₁₁-proline, N-CO₂R₁₁-azetidine-2-carboxylic acid, or N-CO₂R₁₁-pipecolinic acid (R, S, or racemate), wherein R₁₁ is defined as above. The indole magnesium salt is first prepared from the reaction of an indole of formula IV with an alkyl or aryl magnesium halide, preferably ethylmagnesium bromide. The reaction is generally conducted in an inert solvent at a temperature between about -30°C and about 65°C, preferably at about 25°C. Suitable solvents include diethyl ether, tetrahydrofuran, and other alkyl ethers. The preferred solvent is diethyl ether. The acid chloride of proline, azetidine-2-carboxylic acid, or pipecolinic acid is prepared in a separate reaction vessel by reaction of the N-CO₂R₁₁-proline, N-CO₂R₁₁-azetidine-2-carboxylic acid, or N-CO₂R₁₁-pipecolinic acid (R, S, or racemate), with oxalyl chloride in methylene chloride at about -10°C to about 25°C (Helv. Chim. Acta, 1920 (1976)). Suitable solvents include diethyl ether, tetrahydrofuran, other alkyl ethers, and methylene chloride. The proline, azetidine-2-carboxylic acid, or pipecolinic acid is N-substituted with a protecting group to avoid reaction of the nitrogen with the acid chloride when it is formed. Suitable protecting groups are substituted-aryl or substituted-alkyl carbamates (e.g. benzyloxycarbonyl). Preferably, a solution of the N-CO₂R₁₁-proline acid chloride in an inert solvent (e.g., diethyl ether) is added slowly to the solution of the magnesium salt of an indole of formula IV at a temperature of about -30°C to about 50°C, preferably at about 25°C.

The compounds of formula III can be prepared by reacting a compound of formula 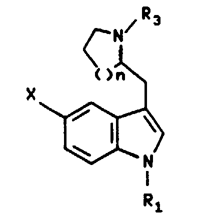 wherein R₁, R₃ and n are defined as above and X is chlorine, bromine or iodine (preferably bromine), with a compound containing a vinyl group (e.g. ethyl vinyl sulfone or N-methylvinylsulfonamide) in the presence of a palladium catalyst, a triarylphosphine and a base in an inert solvent. Suitable catalysts include palladium (II) salts, preferably palladium (II) acetate. Suitable solvents include acetonitrile, N,N-dimethylformamide, and tetrahydrofuran. The preferred solvent is acetonitrile. The preferred triarylphosphine is tri-o-tolylphosphine. Suitable bases include trisubstituted amines. The preferred base is triethylamine. The reaction is conducted at a temperature of about 25°C to 150°C, most preferably at about 80°C.

Compounds of formula I and intermediates to compounds of formula I can be prepared by hydride reduction of a compound of the formula 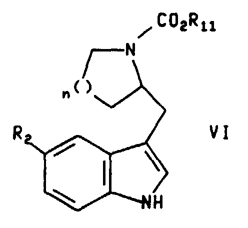 wherein R₂, n, and R₁₁ are as defined above with a hydride reducing agent in an inert solvent. Suitable hydride reducing agents include lithium aluminum hydride, diborane, lithium borohydride, and sodium amide. The preferred reagent is lithium aluminum hydride. Suitable solvents include ethers, such as diethyl ether, tetrahydrofuran, 1,4-dioxane and 1,2-dimethoxyethane. The preferred solvent is tetrahydrofuran. The reduction is conducted at a temperature of about 30°C to about 100°C, preferably about 65°C to about 70°C.

Compounds of formula I and intermediates to compounds of formula I can also be prepared by catalytic reduction of a compound of the formula 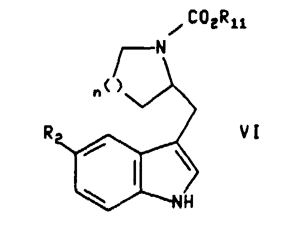 wherein R₂, n, and R₁₁ are as defined above under an atmosphere of hydrogen, preferably at a pressure of about 1 to 3 atmospheres, or using a hydrogen source such as ammonium formate of formic acid in an inert solvent. Suitable catalysts include palladium on carbon, Raney nickel, and platinum oxide. The preferred catalyst is palladium on carbon. Suitable solvents include C₁ to C₆ alcohols, N,N-dimethylformamide, ethyl acetate, and acetonitrile. The preferred solvent is ethanol. The reaction is conducted at a temperature of about 0°C to about 60°C, preferably at about 25°C.

Compounds of formula VI can be prepared by the transition metal catalyzed cyclization of a compound of the formula 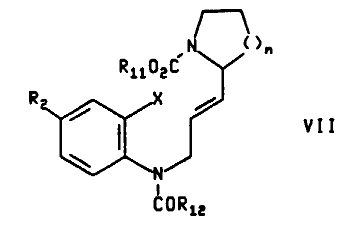 wherein R₂, n, and R₁₁ are as defined above, and X is chlorine, bromine, or iodine (preferably bromine or iodine), and R₁₂ is -OR₁₁ as defined above or alkyl, aryl, or trifluoromethyl (preferably trifluoromethyl) in a suitable inert solvent with a phase transfer catalyst and a base. Suitable catalysts include palladium salts such as palladium (II) acetate or palladium (II) chloride (preferably palladium acetate) and rhodium salts, such as tris(triphenyl)rhodium (I) chloride. Suitable solvents include N,N-dimethylformamide, acetonitrile, and N-methylpyrrolidine. The preferred solvent is N,N-dimethylformamide. Suitable phase transfer catalysts include tetraalkylammonium halides, preferably tetra-n-butylammonium chloride. Suitable bases include tertiary amines, sodium hydrogen carbonate, and sodium carbonate. The preferred base is triethylamine. The reaction is conducted at a temperature of about 80°C to about 180°C, preferably about 150°C to 160°C.

Compounds of formula VI can also be prepared by hydride reduction of a compound of the formula 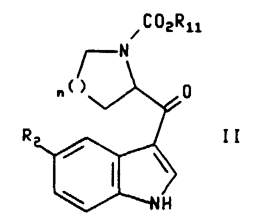 wherein R₂, n, and R₁₁ are as defined above with a hydride reducing agent in an inert solvent. Suitable hydride reducing agents include lithium borohydride, sodium borohydride, and sodium cyanoborohydride. The preferred reagent is lithium borohydride. Suitable solvents include ethers, such as diethyl ether, tetrahydrofuran, 1,4-dioxane and 1,2-dimethoxyethane. The preferred solvent is tetrahydrofuran. The reduction is conducted at a temperature of about 30°C to about 100°C, preferably about 65°C to about 70°C.

Compounds of formula VII can be prepared by the Mitsunobu coupling reaction of compounds of formulas 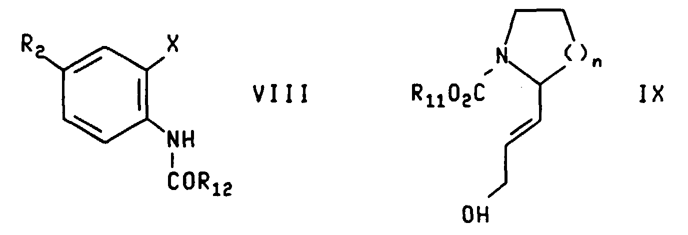 wherein R₂, n, R₁₁, and R₁₂ are as defined above using a phosphine and an azodicarboxylate in a suitable solvent. Suitable phosphines include trialkylphosphines and triarylphosphines, preferably triphenylphosphine. Suitable azodicarboxylates include dialkyl azodicarboxylates, preferably diethyl diazodicarboxylate. Suitable solvents include methylene chloride, ethers, including tetrahydrofuran, diethyl ether, and 1,4-dioxane, N-N-dimethylformamide and acetonitrile. The preferred solvent is tetrahydrofuran. The reaction is conducted at a temperature of about 0°C to about 65°C, most preferably at about 25°C.

Compounds of formula VIII, if not available commercially, can be prepared by reacting a compound of formula X 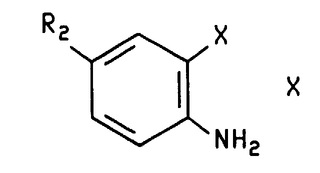 wherein R₂ and X are as defined above with the acid chloride or the symmetrical anhydride of R₁₂CO₂H in a suitable solvent with an suitable base. The preferred acid chloride or anhydride is trifluoroacetic anhydride. Suitable solvents include ethers, including tetrahydrofuran, diethyl ether and 1,4-dioxane, methylene chloride, and chloroform. The preferred solvent is methylene chloride. Suitable bases include triethylamine, pyridine, and sodium hydrogen carbonate. The preferred base is pyridine. The reaction is conducted at a temperature of about 0°C to about 65°C, preferably at about 25°C.

Compounds of formula X, if not available commercially, can be prepared by reacting a compound of formula XI 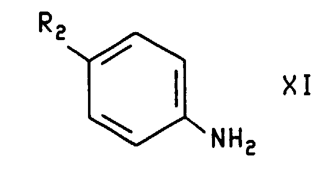 wherein R₂ is as defined above with either chloride, bromine, or iodine in a suitable solvent with a suitable base. Reaction with bromine is preferred. Suitable solvents include C₁-C₆ alcohols, methylene chloride, chloroform, or carbon tetrachloride. The preferred solvent is methanol. Suitable bases include triethylamine, pyridine, sodium carbonate, and sodium hydrogen carbonate. The preferred base is sodium hydrogen carbonate. The reaction is conducted at a temperature of about 0°C to about 65°C, preferably at about 25°C.

Compounds of the formula IX can be prepared from hydride reduction of a compound of formula XII 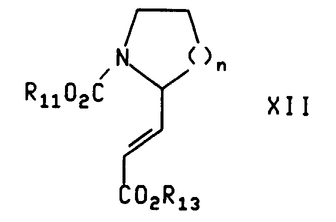 wherein R₁₁ is defined as above and R₁₃ is C₁-C₆, alkyl, aryl, or alkylaryl with a hydride reducing agent in an inert solvent. Suitable hydride reducing agents include lithium aluminum hydride, lithium borohydride, sodium borohydride, and diisobutylaluminum hydride. The preferred reagent is diisobutylaluminum hydride. Suitable solvents include ethers, such as diethyl ether, tetrahydrofuran, 1,4-dioxane and 1,2-dimethoxyethane. The preferred solvent is tetrahydrofuran. The reduction is conducted at a temperature of about -100°C to about 0°C, preferably at about -80°C to about -70°C.

Compounds of the formula XII can be prepared from the Wittig reaction in a suitable solvent involving compounds of the formulas 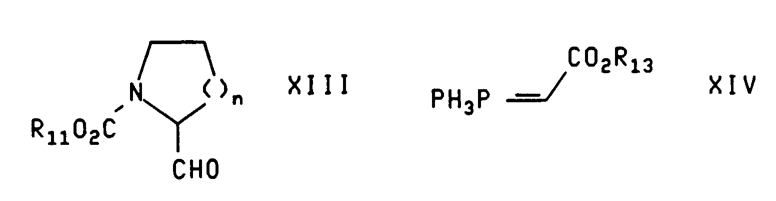 wherein R₁₁ and R₁₃ are defined as above. Suitable solvents include ethers such as diethyl ether, tetrahydrofuran, and 1,4-dioxane. Tetrahydrofuran is the preferred solvent. The reaction is conducted at a temperature of about -78°C to about 30°C, preferably at about -78°C.

Compounds of the formula XIII can be prepared as outlined in S. Kiyooka, et al., J. Org. Chem., 5409 (1989) and Y. Hamada, et al., Chem. Pharm. Bull., 1921 (1982).

Compounds of the formula XIV are either commercially available or can be prepared as outlined in L. Fieser and M. Fieser, Reagents for Organic Synthesis, John Wiley and Sons, New York, Vol. 1, p. 112 (1967).

Unless indicated otherwise, the pressure of each of the above reactions is not critical. Generally, the reactions will be conducted at a pressure of about one to about three atmospheres, preferably at ambient pressure (about one atmosphere).

The compounds of the formula I which are basic in nature are capable of forming a wide variety of different salts with various inorganic and organic acids. Although such salts must be pharmaceutically acceptable for administration to animals, it is often desirable in practice to initially isolate a compound of the formula I from the reaction mixture as a pharmaceutically unacceptable salt and then simply convert the latter back to the free base compound by treatment with an alkaline reagent, and subsequently convert the free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent such as methanol or ethanol. Upon careful evaporation of the solvent, the desired solid salt is obtained.

The acids which are used to prepare the pharmaceutically acceptable acid addition salts of the base compounds of this invention are those which form non-toxic acid addition salts, i.e., salts containing pharmacologically acceptable anions, such as hydrochloride, hydrobromide, hydroiodide, nitrate, sulfate or bisulfate, phosphate or acid phosphate, acetate, lactate, citrate or acid citrate, tartrate or bitartrate, succinate, maleate, fumarate, gluconate, saccharate, benzoate, methanesulfonate and pamoate [i.e., 1,1'-methylene-bis-(2-hydroxy-3-naphthoate)] salts.

Those compounds of the formula I which are also acidic in nature, e.g., where R₂ contains a carboxylate, are capable of forming base salts with various pharmacologically acceptable cations. Examples of such salts include the alkali metal or alkaline-earth metal salts and particularly, the sodium and potassium salts. These salts are all prepared by conventional techniques. The chemical bases which are used as reagents to prepare the pharmaceutically acceptable base salts of this invention are those which form non-toxic base salts with the herein described acidic compounds of formula I. These non-toxic base salts include those derived from such pharmacologically acceptable cations as sodium, potassium calcium and magnesium, etc. These salts can easily be prepared by treating the corresponding acidic compounds with an aqueous solution containing the desired pharmacologically acceptable cations, and then evaporating the resulting solution to dryness, preferably under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction of maximum product of yields of the desired final product.

The compounds of the formula I and the pharmaceutically acceptable salts thereof (hereinafter, also referred to as the active compounds of the invention) are useful psychotherapeutics and are potent serotonin (5-HT₁) agonists and may be used in the treatment of depression, anxiety, eating disorders, obesity, drug abuse, cluster headache, migraine, chronic paroxysmal hemicrania and headache associated with vascular disorders, pain, and other disorders arising from deficient serotonergic neurotransmission. The compounds can also be used as centrally acting antihypertensives and vasodilators.

The active compounds of the invention are evaluated as anti-migraine agents by testing the extent to which they mimic sumatriptan in contracting the dog isolated saphenous vein strip (P.P.A. Humphrey et al., Br. J. Pharmacol., 94, 1128 (1988)). This effect can be blocked by methiothepin, a known serotonin antagonist. Sumatriptan is known to be useful in the treatment of migraine and produces a selective increase in carotid vascular resistance in the anaesthetized dog. It has been suggested (W. Fenwick et al., Br. J. Pharmacol., 96, 83 (1989)) that this is the basis of its efficacy.

The compositions of the present invention may be formulated in a conventional manner using one or more pharmaceutically acceptable carriers. Thus, the active compounds of the invention may be formulated for oral, buccal, intranasal, parenteral (e.g., intravenous, intramuscular or subcutaneous) or rectal administration or in a form suitable for administration by inhalation or insufflation.

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium phosphate); lubricants (e.g. magnesium stearate, talc or silica); disintegrants (e.g. potato starch or sodium starch glycollate) ; or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, methyl cellulose or hydrogenated edible fats); emulsifying agents (e.g. lecithin or acacia); non-aqueous vehicles (e.g. almond oil, oily esters or ethyl alcohol) ; and preservatives (e.g. methyl or propyl p-hydroxybenzoates or sorbic acid).

For buccal administration the composition may take the form of tablets or lozenges formulated in conventional manner.

The active compounds of the invention may be formulated for parenteral administration by injection, including using conventional catheterization techniques or infusion. Formulations for injection may be presented in unit dosage form e.g. in ampules or in multi-dose containers, with an added preservative. The compositions may take such forms as suspensions, solutions or emulsions in oily or aqueous vehicles, and may contain formulating agents such as suspending, stabilizing and/or dispersing agents. Alternatively, the active ingredient may be in powder form for reconstitution with a suitable vehicle, e.g. sterile pyrogen-free water, before use.

The active compounds of the invention may also be formulated in rectal compositions such as suppositories or retention enemas, e.g., containing conventional suppository bases such as cocoa butter or other glycerides.

For intranasal administration or administration by inhalation, the active compounds of the invention are conveniently delivered in the form of a solution or suspension from a pump spray container that is squeezed or pumped by the patient or as an aerosol spray presentation from a pressurized container or a nebulizer, with the use of a suitable propellant, e.g. dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. The pressurized container or nebulizer may contain a solution or suspension of the active compound. Capsules and cartridges (made, for example, from gelatin) for use in an inhaler or insufflator may be formulated containing a powder mix of a compound of the invention and a suitable powder base such as lactose or starch.

A proposed dose of the active compounds of the invention for oral, parenteral or buccal administration to the average adult human for the treatment of the conditions referred to above (e.g., migraine) is 0.1 to 200 mg of the active ingredient per unit dose which could be administered, for example, 1 to 4 times per day.

Aerosol formulations for treatment of the conditions referred to above (e.g., migraine) in the average adult human are preferably arranged so that each metered dose or "puff" of aerosol contains 20 µg to 1000 µg of the compound of the invention. The overall daily dose with an aerosol will be within the range 100 µg to 10 mg. Administration may be several times daily, for example 2, 3, 4 or 8 times, giving for example, 1, 2 or 3 doses each time.

The following Examples illustrate the preparation of the compounds of the present invention. Melting points are uncorrected. NMR data are reported in parts per million (6) and are referenced to the deuterium lock signal from the sample solvent. Specific rotations were measured at room temperature using the sodium D line (589 nm).

Commercial reagents were utilized without further purification. Chromatography refers to column chromatography performed using 32-63 µm silica gel and executed under nitrogen pressure (flash chromatography) conditions. Room temperature refers to 20 - 25°C.

### EXAMPLE 1

### General Procedure for the Reduction of Benzyloxy-carbonyl-pyrrolidin-2-ylcarbonyl-1H-indole, N-Benzyloxy-carbonyl-azetidin-2-ylcarbonyl-1H-indoles, or N-Benzyloxy-carbonyl-piperidin-2-ylcarbonyl-1H-indoles Forming 3-(N-Methyl-pyrrolidin-2-ylmethyl)-1H-indoles, 3-(N-Methyl-azetidin-2-ylmethyl)-1H-indoles, or 3-(N-Methylpiperidin-2-ylmethyl)-1H-indoles,respectively.

To a stirred solution of (R)- or (S)-(N-benzyloxycarbonylpyrrolidin-2-ylcarbonyl)-1H-indole, (R)-, (S), or (R,S)-(N-benzyloxycarbonylazetidin-2-ylcarbonyl)-1H-indole, or (R)-, (S)-, or (R,S)-(N-benzyloxycarbonylpiperidin-2-ylcarbonyl)-1H-indole, (5.00 mmol) in anhydrous tetrahydrofuran (20mL) at room temperature under nitrogen was carefully added lithium aluminum hydride (0.57 g, 15.0 mmol, 3.0 eq) as a powder, and the resulting mixture was stirred at room temperature under nitrogen for 1 hour. The mixture was then heated at reflux (66°C) under nitrogen for 12 hours. The reaction was then quenched with successive additions of water (0.5 mL), aqueous sodium hydroxide (20%, 0.5 mL), and then additional water (1.0 mL), and the resulting mixture filtered through diatomaceous earth (Celite (trademark)). The solids were then washed with copious amounts of ethyl acetate (50 mL). The combined filtrate was then washed with water (20 mL), dried (MgSO₄), and evaporated under reduced pressure. The residue was then column chromatographed using silica gel (50 g) and elution with the appropriate solvent system to afford the 3-(N-methylpyrrolidin-2-ylmethyl)-1H-indole, 3-(N-methylazetidin-2-ylmethyl)-1H-indole, or 3-(N-methylpiperidin-2-ylmethyl)-1H-indole. Following this procedure the following compounds were prepared:
A. **(S)-5-Methoxy-3-(N-methylpyrrolidin-2-ylmethyl)-1H-indole**
   (S)-(N-Benzyloxycarbonylpyrrolidin-2-ylcarbonyl)-5-methoxy-1H-indole was used. The chromatographic eluent was 8% triethylamine in ethyl acetate to afford the title compound (yields ranged from 22 to 57%) as an oil: IR (CHC1₃) 3475, 1625, 1585, 1480, 1455 cm⁻¹; ¹H NMR (CDC1₃) δ 8.13 (br s, 1H), 7.23 (d, J=8.8 Hz, 1H), 7.04 (d, J=2.4 Hz, 1H), 6.97 (d, J=2.2 Hz, 1H), 6.84 (dd, J=2.4 and 8.8 Hz, 1H), 3.86 (s, 3H), 3.17-3.10 (m, 2H), 2.58 (dd, J=9.9 and 13.9 Hz, 1H), 2.50-2.40 (m, 1H), 2.47 (s, 3H), 2.26-2.17 (m, 1H), 1.89-1.72 (m, 2H), 1.70-1.52 (m, 2H);¹³C NMR (CDC1₃) δ 153.8, 131.4, 128.2, 122.7, 113.9, 111.8, 111.7, 101.1, 66.6, 57.5, 56.0, 40.8, 31.5, 30.0, 21.9; LRMS, m/z (relative intensity) 244 (M⁺, 7), 160 (20), 145 (16), 117 (21), 84 (100); HRMS: calculated for C₁₅H₂₀N₂O: 244.1573; found: 244.1575; [α]²⁵= -96° (CHC1₃, c = 1.0).
B. **(R)-5-Methoxy-3-(N-methylpyrrolidin-2-ylmethyl)-1H-indole**
   (R)-(N-Benzyloxycarbonylpyrrolidin-2-ylcarbonyl)-5-methoxy-1H-indole was used. The chromatographic eluent was 8% triethylamine in ethyl acetate to afford the title compound (yields ranged from 13 to 61%) as an oil whose spectral and physical properties were identical with the spectral and physical properties of the title compound of Example 1A with the exception of specific rotation of plane polarized light: [α]²⁵ = + 100° (CHC1₃, c = 1.0). HRMS: calculated for C₁₅H₂₀N₂O: 244.1573; found: 244.1547.
C. **(R)-5-Dibenzylamino-3-(N-methylpyrroidin-2-ylmethyl)-1H-indole**
   (R)-3-(N-Benzyloxycarbonylpyrrolidin-2-ylcarbonyl)-5-dibenzylamino-1H-indole was used. Column chromatography using elution with methylene chloride/methanol/ammonium hydroxide [9:1:0.1] afforded the title compound as a pale green foam: ¹H NMR (CDCl₃) δ 7.82 (br s, NH), 7.35-7.19 (m, 10 H), 7.20 (d, J=8.6 Hz, 1H), 6.95 (d, J=2.1 Hz, 1H), 6.85 (dd, J=2.3 and 8.7 Hz, 1H), 6.80 (d, J=2.2 Hz, 1H), 4.65 (s, 4H), 3.25-3.02 (m, 2H), 2.52 (dd, J=9.5 and 13.9 Hz, 1H), 2.39-2.15 (m, 2 H), 2.30 (s, 3H), 1.85-1.40 (m, 4H);¹³C NMR (CDCl₃) δ 143.2, 139.7, 130.5, 128.5, 128.2, 127.3, 126.8, 122.9, 112.5, 112.2, 111.8, 103.4, 67.0, 57.4, 56.4, 40.6, 31.4, 29.7, 21.9. HRMS: calculated for C₂₈H₃₁N₃ 409.2520. Found 409.2475.
D. **(R)-5-Methoxy-3-(N-methylpiperid-2-ylmethyl)-1H-indole**
   (R)-3-(N-Benzyloxycarbonylpiperid-2-ylcarbonyl)-5-methoxy-1H-indole was used. Column chromatography using elution with 6% triethylamine in ethyl acetate afforded the title compound as a white foam: ¹³C NMR (CDCl₃) δ 153.7, 131.4, 128.3, 123.3, 113.2, 111.7, 111.6, 101.2, 64.4, 57.2, 55.9, 43.4, 31.0, 28.8, 25.9, 24.1; [α]²⁵= +67° (CDCl₃, c=1.0); HRMS: calculated for C₁₆H₂₂N₂O: 258.1734. Found: 258.1710.
E. **(S)-5-Methoxy-3-(N-methylazetidin-2-ylmethyl-1H-indole**
   (S)-3-(N-Benzyloxycarbonylazetidinyl-2-ylcarbonyl)-5-methoxy-1H-indole was used. The chromatographic eluet was 8% triethylamine in ethyl acetate to afford the title compound as a white solid: mp, 118.0-120.0°C; ¹³C NMR (CDCl₃) δ153.8, 131.6, 128.0, 122.9, 112.3, 111.9, 111.8, 101.0, 68.5, 56.0, 53.1, 44.7, 32.4, 25.0; [α]²⁵ = -44° (CHCl₃, c=1.0). Anal. calcd for C₁₄H₁₈N₂O: C, 73.01; H, 7.88, N, 12.16. Found: C, 72.65; H, 7.91; N, 12.06.
F. **(R,S)-5-Methoxy-3-(N-methylazetidin-2-ylmethyl)-1H-indole**
   (R,S)-3-(N-Benzyloxycarbonylazetidinyl-2-ylcarbonyl)-5-methoxy-1H-indole was used. The chromatographic eluet was 10% triethylamine in ethyl acetate to afford the title compound as a white solid: mp, 116.0-119.0°C; Anal. calcd for C₁₄H₁₈N₂O: C, 73.01; H, 7.88; N, 12.16. Found: C, 72.61; H, 7.99; N, 12.10.

### EXAMPLE 2

### General Method for the Hydrogenation of 5-(2-Sulfonylethenyl)-3-(N-methylpyrrolidin-2-ylmethyl)-1H-indoles to Form 5-(2-sulfonylethyl)-3-(N-methylpyrrolidin-2-ylmethyl)-1H-indoles

A solution of 5-(2-sulfonylethenyl)-3-(N-methylpyrrolidin-2-yl)-1H-indole (0.47 mmol) and 10% Pd/C (0.150 g) in ethanolic hydrogen chloride [prepared from absolute ethanol (10 mL) and acetyl chloride (43 µL) and N, N-dimethylformamide (7.5 mL) was shaken under a hydrogen atmosphere (15 psi) at room temperature for 20 hours. The resultant reaction mixture was filtered through diatomaceous earth (Celite (trademark)), washed with absolute ethanol, and the combined filtrates were evaporated under reduced pressure. The residue was partitioned between ethyl acetate and water. The organic phase was separated, washed with water (3x), brine (1x), dried (Na₂SO₄), and evaporated under reduced pressure to afford a yellow oil. Column chromatography of this oil using silica gel and elution with methylene chloride/absolute ethanol/ammonia (90:10:1) afforded the appropriate 5-(2-Ethylsulfonylethyl)-3-(N-methylpyrrolidin-2-ylmethyl)-1H-indole. Following this procedure, the following compounds were prepared:
A. **(R)-5-(2-Ethylsulfonylethyl)-3-(N-methylpyrrolidin-2-ylmethyl)-1H-indole**
   (R)-5-trans-(2-Ethylsulfonylethenyl)-3-(N-methylpyrrolidin-2-ylmethyl)-1H-indole (Example 4A) was reduced as described above. Chromatography afforded the title compound (0.33 mmol, 70%) as a gum: TLC (CH₂Cl₂:EtOH:NH₃, 90:10:1): R_{f} = 0.3; [α]²⁵ = + 62° (methanol, c = 0.10). Anal. Calcd for C₁₈H₂₆N₂O₂S • 0.05 CH₂Cl₂: C, 63.21; H, 7.67; N, 8.17; found: C, 63.55; H, 7.61; N, 8.41.
B. **(R)-5-(2-Methylaminosulfonylethyl)-3-(N-methyl-pyrrolidin-2-ylmethyl)-1H-indole**
   (R)-5-trans-(2-Methylaminosulfonylethenyl)-3-(N-methylpyrrolidin-2-ylmethyl)-1H-indole (Example 4B) was reduced as described above. Chromatography afforded the title compound (65%) as a foam. Anal. Calcd for C₁₇H₂₅N₃O₂S • 0.1 CH₂Cl₂: C, 59.71; H, 7.39; N, 12.12; found: C, 59.66; H, 7.14; N, 11.90.

### EXAMPLE 3

### General Synthesis of 3-(N-Benzyloxycarbonylpyrrolidin-2-ylcarbonyl)-1H-indoles 3-(N-Benzyloxycarbonylazetidin-2-ylcarbonyl)-1H-indoles, or 3-(N-Benzyloxycarbonylpiperidin-2-ylcarbonyl)-1H-indoles.

Two solutions containing the reactants were prepared separately as follows. To a stirred solution of N-carbobenzyloxyproline (D or L, 3.10 g, 12.4 mmol, 1 eq) or N-carbobenzyloxyazetidine-2-carboxylic acid (R or S or racemate, 12.4 mmol) or N-carbobenzyloxypipecolinic acid (R or S or racemate, 12.4 mmol) in anhydrous methylene chloride (7 mL) with one drop dimethylformamide was added oxalyl chloride (1.60 mL, 18.4 mmol, 1.5 eq), and the resulting effervescing solution was stirred at room temperature under nitrogen for 1.5 hours. The solution was then evaporated under reduced pressure, and any remaining solvent was removed from the residual oil using high vacuum to afford the N-benzyloxycarbonylproline acid chloride. At the same time, a solution of ethylmagnesium bromide (3.0 M in ether, 4.13 mL, 12.4 mmol, 1 eq) was added to a stirred solution of the indole (12.4 mmol) in anhydrous ether (50 mL), and this cloudy solution was heated at reflux under nitrogen for 1.5 hours to form the indolemagnesium bromide salt. The proline acid chloride was then dissolved in methylene chloride or ethyl ether (3 mL), and this solution was added dropwise to the stirred solution of the indolemagnesium bromide salt at room temperature, and the resultant reaction mixture was stirred at room temperature under nitrogen for 1 hour. A saturated solution of sodium hydrogen carbonate (25 mL) and ethyl acetate (50 mL) was then added to the reaction mixture, and this mixture was vigorously stirred for 15 minutes. The resulting mixture was filtered through diatomaceous earth (Celite (trademark)), the solids washed with copious amounts of ethyl acetate, and the ethyl acetate layer was separated from the aqueous layer which was extracted with ethyl acetate (2 x 25 mL). All ethyl acetate extracts were combined, dried, and evaporated under reduced pressure. The residual oil/solid was flash chromatographed using silica gel (250 g) and eluted with an appropriate solvent system to afford the desired 3-(N-benzyloxycarbonylpyrrolidin-2-ylcarbonyl)indole,3-(N-benzyloxycarbonylazetidin-2-ylcarbonyl)-1H-indole, or 3-(N-benzyloxycarbonylpiperidin-2-ylcarbonyl)-1H-indole.

### A. (S)-3-(N-Benzyloxycarbonylpyrrolidin-2-ylcarbonyl)-5-methoxy-1H-indole

N-Carbobenzyloxy-L-proline was used. Chromatography using 40-60% ethyl acetate gradient in hexanes afforded the title compound (yields ranged from 27 to 43%) as a white powder. Recrystallization in ethyl acetate/hexanes afforded an analytical sample as a white crystalline solid: mp, 164.0-165.0°C; IR (KBr) 3250, 1695, 1660, 1585, 1520, 1485, 1450, 1425 cm⁻¹; ¹H NMR (CDCl₃) [Note: the spectrum of the title compound appears as a 1:3 mixture of diastereomers due to slow inversion of the amide nitrogen on an NMR time scale. Therefore, the ¹H NMR will be interpreted for each compound separately with the more abundant conformer quoted first] δ [more abundant conformer] 9.83 (br s, 1H), 7.53 (d, J=3.4 Hz, 1H), 7.42-7.30 (m, 6H), 7.00 (d, J=8.9 Hz, 1H), 6.69 (dd, J=2.4 and 9.0 Hz, 1H), 5.25 (d, J=12.9 Hz, 1H), 5.14 (d, J=12.5 Hz, 1H), 5.07-4.99 (m, 1H), 3.74 (s, 3H), 3.78-3.55 (m, 2H), 2.28-1.84 (m, 4H) and δ [less abundant conformer] 9.28 (br s, 1H), 7.90 (d, J=2.3 Hz, 1H), 7.59 (d, J=3.4 Hz, 1H), 7.24 (d, J=9.0 Hz, 1H), 7.06-6.90 (m, 5H), 6.88 (dd, J=2.7 and 9.0 Hz, 1H), 5.07-4.99 (m, 2H), 4.96-4.88 (m, 1H), 3.86 (s, 3H), 3.78-3.55 (m, 2H), 2.28-1.84 (m, 4H); LRMS, m/z (relative intensity) 379 (8), 378 (M⁺,33), 204 (31), 174 (64), 160 (41), 146 (10), 91 (100). Analysis: calculated for C₂₂H₂₂N₂O₄: C, 69.83; H, 5.86; N, 7.40; found: C, 69.81; H, 5.67; N, 7.40.

### B. (R)-3-(N-Benzyloxycarbonylpyrrolidin-2-ylcarbonyl)-5-methoxy-1H-indole

N-Carbobenzyloxy-D-proline was used. Chromatography using 40-60% ethyl acetate gradient in hexanes afforded the title compound (yields ranged from 25 to 36%) as a white powder. Recrystallization in ethyl acetate/hexanes afforded an analytical sample as a white crystalline solid: mp, 165.0-166.0°C. The spectral and physical data for the title compound was identical in all respects with the spectral and physical data of its enantiomer (the title compound of Example 3A); HRMS: calculated for C₂₂H₂₂N₂O₄: 378.1582; found: 378.1573.

### C. (R)-3-(N-Benzyloxycarbonylpyrrolidin-2-ylcarbonyl)-5-dibenzylamino-1H-indole

N-Carbobenzyloxy-D-proline was used. Trituration of the extraction residue with diethyl ether afforded the title compound as a solid: mp, 176.0-177.0°C; LRMS (m/z, relative intensity) 543 (100, M⁺), 453 (10), 407 (7), 339 (40), 307 (10), 247 (10), 154 (38); [α]²⁵=+112° (THF, c=1.0); Anal. calcd for C₃₅H₃₃N₃O₃: C, 77.32; H, 6.12; N, 7.73. Found: C, 77.35; H, 6.30; N, 7.66.

### D. (R)-3-(N-Benzyloxycarbonylpiperid-2-ylcarbonyl)-5-methoxy-1H-indole

N-Carbobenzyloxy-D-pipecolinic acid was used. Column chromatography using elution with 10% ether in methylene chloride afforded the title compound as a tan foam: LRMS (m/z, relative intensity) 392 (90, M⁺), 348 (27), 284 (13), 273 (12), 258 (15), 237 (47), 217 (58), 173 (100), HRMS: calculated for C₃₅H₃₃N₃O₂: C, 69.22; H, 5.53; N, 7.69. Found: C, 69.35; H, 5.33; N, 7.64.

### E. (S)-3-(N-Benzyloxycarbonylazetidinyl-2-ylcarbonyl)-5-methoxy-1H-indole

(S)-N-Carbobenzyloxyazetidine-2-carboxylic acid was used. Trituration of the extract residue with absolute methanol afforded the title compound as a white solid: mp, 199.0-200.0°C. Anal. calcd for C₂₁H₂₀N₂O₄: C, 69.22; H, 5.53; N, 7.69. Found: C, 69.35; H, 5.33; N, 7.64.

### F. (R,S)-3-(N-Benzyloxycarbonylazetidinyl-2-ylcarbonyl)-5-methoxy-1H-indole

(R,S)-N-Carbobenzyloxyazetidine-2-carboxylic acid was used. Trituration of the extract residue with absolute methanol afforded the title compound as a white solid: mp, 199.0-200.0°C. Anal. calcd for C₂₁H₂₀N₂O₄: C, 69.22; H, 5.53; N, 7.69. Found: C, 68.85; H, 5.47; N, 7.57.

### EXAMPLE 4

### General Method for the Synthesis of 5-trans-(2-Sulfonylethenyl)-3-(N-methylpyrrolidin-2-ylmethyl)-1H-indoles

A mixture of the appropriate vinyl sulfone (1.17 mmol, 1.4 eq), tri-o-tolylphosphine (0.075 g, 0.25 mmol, 0.33 eq), palladium (II) acetate (0.013 g), triethylamine (0.25 mL, 1.79 mmol, 2 eq), and (R)-5-bromo-3-(N-methylpyrrolidinylmethyl)-1H-indole (0.25 g, 0.85 mmol) in anhydrous acetonitrile (3 mL) was heated at reflux under nitrogen for 17 hours. The resultant reaction mixture was evaporated under reduced pressure, and the residue was column chromatographed using silica gel and elution with methylene chloride/absolute ethanol/ammonia (90:8:1) to afford the title compound.

### A. (R)-5-trans-(2-Ethylsulfonylethenyl)-3-(N-methylpyrrolidin-2-ylmethyl)-1H-indole

Ethyl vinyl sulfone was used, and chromatography afforded the title compound (65%) as a white foam: TLC (CH₂Cl₂/EtOH/NH₃, 90:10:1):R_{f}= 0.5. Analysis: calculated for C₁₈H₂₄N₂O₂S • 0.2 CH₂Cl₂: C, 62.55; H, 7.04; N, 8.02; found: C, 62.65; H, 6.94; N, 7.92.

### B. (R)-5-trans-(2-Methylaminosulphonylethenyl)-3-(N-methyl-pyrrolidin-2-ylmethyl)-1H-indole

N-methylvinylsulfonamide was used, and chromatography afforded the title compound (71%) as a white foam. Analysis: calculated for C₁₇H₂₃N₃O₂S • 0.1 CH₂Cl₂ : C, 60.06; H, 6.84; N, 12.29; found: C, 59.74; H, 6.77; N, 11.97.

### EXAMPLE 5

### General Procedure for the Hydride Reduction of 3-(N-Benzyloxycarbonyl-pyrrolidin-2-ylmethyl-1H-indoles and 3-(N-Benzyloxycarbonylpiperid-2-ylmethyl)-1H-indoles Forming 3-(N-Methylpyrrolidin-2-ylmethyl)-1H-indoles and 3-(N-Methylpiperid-2-ylmethyl)-1H-indoles

To a stirred mixture of lithium aluminum hydride (0.152 g, 4.00 mmol,2 eq) in anhydrous tetrahydrofuran (10 mL) at 0°C was added rapidly a solution of the 3-(N-benzyloxycarbonylpyrrolidin-2-ylmethyl)-1H-indole or the 3-(N-benzyloxycarbonylpiperid-2-ylmethyl)-1H-indole (2.00 mmol) in anhydrous tetrahydrofuran (5 mL). The resulting mixture is heated at reflux under a nitrogen atmosphere for 3 hours. The reaction mixture is cooled, and water (0.25 mL), 15% aqueous sodium hydroxide (0.25mL), and then more water (0.75 mL) were added sequentially. The resulting mixture was stirred at 25°C for 30 minutes, filtered, and the filtrate was evaporated under reduced pressure. The residue was column chromatographed using silica gel (approximately 50 g) and elution with a solution methylene chloride: methanol: ammonium hydroxide [9:1:0.1] or other appropriate solvent system to afford the corresponding 3-(N-methylpyrrolidin-2-ylmethyl)-1H-indole or 3-(N-methylpiperid-2-ylmethyl)-1H-indole.

Following this procedure the following compounds were prepared:
A. **(R)-5-(Methylaminosulfonylmethyl)-3-(N-methylpyrrolidin-2-ylmethyl)-1H-indole**
   (R)-3-(N-Benzyloxycarbonylpyrrolidin-2-ylmethyl)-5-(methylaminosulfonylmethyl)-1H-indole was used. The reaction residue after aqueous work-up as described above was triturated with absolute methanol to afford the title compound as a white solid: mp, 213.0-214.0°C; ¹H NMR (DMSO-d₆) δ 10.9 (br s, indole NH), 7.51 (be d, 1H), 7.31 (d, J=8.3 Hz, 1H), 7.16 (br d, 1H), 7.08 (br dd, J=8.3 Hz, 1H), 6.82 (br q, sulfonamide NH), 4.35 (s, 2H), 3.07-2.95 (m, 2H), 2.54 (d, J=4.7 Hz, 3H), 2.52-2.38 (m, 2H), 2.35 (s, 3H), 2.10 (br, q, J=8.2 Hz, 1H), 1.75-1.40 (m, 4H); [α]²⁵=+89° (DMSO-d6_{,} c=1.0); Anal. calcd for C₁₆H₂₃N₃SO₂: C, 59.79; H, 7.21; N, 13.07. Found: C, 59.66; H, 7.29; N, 12.81.
B. **(R)-5-Aminomethyl-3-(N-methylpyrrolidin-2-ylmethyl)-1H-indole**
   (R)-3-(N-Benzyloxycarbonylpyrrolidin-2-ylmethyl)-5-cyano-1H-indole was used. Column chromatography using elution with 9:1:0.1 [methylene chloride:methanol: ammonium hydroxide] afforded the title compound as a white foam: ¹³C NMR δ 135.6, 132.3, 127.5, 123.0, 122.8, 121.4, 117.1, 112.8, 111.5, 66.8, 57.2, 46.4, 40.5, 31.2, 29.2, 21.5; HRMS: calculated for C₁₅H₂₁N₃ 243.1737, found 243.1732.
C. **(R,S)-5-(Methylaminosulfonylmethyl)-3-(N-methylpiperid-2-ylmethyl)-1H-indole**
   (R,S)-3-(N-Benzyloxycarbonylpiperidin-2-ylmethyl)-5-(methylaminosulfonylmethyl)-1H-indole was used. Column chromatography using elution with 10% triethylamine in ethyl acetate afforded the title compound as a clear, colorless oil: ¹³C NMR (DMSO-d₆) δ 135.9, 127.7, 124.0, 123.6, 121.0, 119.7, 111.9, 111.1, 63.9, 56.7, 56.3, 43.2, 30.5, 29.0, 27.9, 25.5, 23.7; LRMS (m/z, relative intensity) 336 (1, M⁺), 241 (5), 143 (31), 142 (13), 99 (34), 98 (100), 70 (16); HRMS calculated for C₁₇H₂₅N₃O₂S: 336.1745; found: 336.1756.

### EXAMPLE 6

### General Procedure for the Catalytic Reduction of 3-(N-Benzyloxycarbonylpyrrolidin-2-ylmethyl)-1H-indoles and 3-(N-Benzyloxycarbonylpiperid-2-ylmethyl)-1H-indoles Forming 3-(Pyrrolidin-2-ylmethyl)-1H-indoles and 3-(Piperid-2-ylmethyl)-1H-indoles

A mixture of the 3-(N-benzyloxycarbonylpyrrolidin-2-ylmethyl)-1H-indole or the 3-(N-benzyloxycarbonylpiperid-2-ylmethyl)-1H-indole (2.00 mmol), 10% palladium on carbon (0.20 g), and ammonium formate (1.26 g, 20 mmol, 10 eq) in absolute ethanol (15 mL) was stirred under a nitrogen atmosphere for 4 hours. The resulting reaction mixture was filtered through diatomaceous earth, and the filtrate was evaporated under reduced pressure. The residue was column chromatographed using silica gel (approximately 50 g) and elution with a solution of methylene chloride: methanol: ammonium hydroxide [8:2:0.2] or other appropriate solvent system to afford the corresponding 3-(pyrrolidin-2-ylmethyl)-1H-indole or 3-(piperid-2-ylmethyl)-1H-indole.

Following this procedure the following compounds were prepared:
A. **(R)-5-(Methylaminosulfonylmethyl)-3-(pyrrolidin-2-ylmethyl)-1H-indole**
   (R)-3-(N-Benzyloxycarbonylpyrrolidin-2-ylmethyl)-5-(methylaminosulfonylmethyl)-1H-indole was used. Column chromatography as described above afforded the title compound as an off-white gum: ¹³C NMR (DMSO-d₆) δ 135.9, 127.5, 123.8, 123.7, 120.9, 119.7, 112.4, 111.1, 59.2, 56.6, 45.7, 31.1, 31.0, 29.0, 24.6; [α]²⁵ = +4° (DMSO-d₆, c=1.0); [α]²⁵=-14° (EtOH/CHCl₃ [1:1], c=1.0); HRMS: calculated for [C₁₅H₂₁N₃O₂S•H⁺]: 308.1433; found: 308.1467.
B. **(R)-5-Cyano-3-pyrrolidin-2-ylmethyl)-1H-indole**
   (R)-3-(N-Benzyloxycarbonlpyrrolidin-2-ylmethyl)-5-cyano-1H-indole was used. Column chromatography as described above afforded the title compound as an off-white gum: ¹³C NMR (CDCl₃/CD₃OD) δ 138.1, 127.2, 125.0, 124.4, 124.2, 121.0, 113.4, 112.2, 101.5, 59.5, 50.1, 45.7, 31.3, 30.3, 24.7; LRMS (M/z, relative intensity) 225 (M+,3), 179 (3), 155 (10), 70 (100) ; HRMS: calculated for C₁₄H₁₅N₃ 225.1268, found 225.1245.
C. **(R)-3-(Pyrrolidin-2-ylmethyl)-1H-indole**
   (R)-3-(N-Benzyloxycarbonylpyrrolidin-2-ylmethyl)-1H-indole was used. Evaporation of the filtrate residue directly afforded the title compound as a white foam: ¹H NMR (CDCl₃) δ 9.05 (br s, indole NH), 7.50 (d, J=8.6 Hz, 1H), 7.23 (d, J=8.6 Hz, 1H), 7.12-6.98 (m, 2H), 6.90 (s, 1H), 4.0 (br s, amine NH), 3.36-3.24 (m, 1H), 2.95-2.75 (m, 3H), 2.70-2.58 (m, 1H), 1.85-1.50 (m, 3H), 1.45-1.29 (m, 1H); [α]²⁵ = +18° (CHCl₃, c-1.0).
D. **(R)-5-Methoxy-3-(Pyrrolidin-2-ymethyl)-1H-indole**
   (R)-3-(N-Benzyloxycarbonylpyrrolidin-2-ylmethyl)-5-methoxy-1H-indole was used. Evaporation of the filtrate residue directly afforded the title compound as a gum: LRMS (m/z, relative intensity) 231 (100, M+), 161 (10), 155 (17), 135 (11), 119 (32); [α]²⁵=-12° (CHCl₃, c=1.0); Anal, calcd for C₁₄H₁₈N₂O•0.75 C₂H₄O₂ [acetic acid salt]: C, 67.61; H, 7.69; N, 10.17. Found: C, 67.74; H, 7.53; N, 9.90.
E. **(R,S)-5-(Methylaminosulfonylmethyl)-3-(piperid-2-ylmethyl)-1H-indole**
   (R,S)-3-(N-Benzyloxycarbonylpiperid-2-ylmethyl)-5-(methylaminosulfonylmethyl)-1H-indole was used. Column chromatography as described above afforded the title compound as a clear, colorless oil: ¹³C NMR (DMSO-d₆) δ 136.0, 127.5, 124.2, 123.8, 121.0, 119.8, 111.2, 110.9, 56.8, 56.7, 45.8, 31.7, 31.4, 29.0, 25.0, 23.9; LRMS (m/z, relative intensity) 321 (19, M+), 238 (43), 227 (21), 144 (99), 143 (100); HRMS: calculated for C₁₆H₂₃N₃O₂S: 321.1513; found: 321.1501.

### EXAMPLE 7

### General Procedure for the Formulation of 3-(N-Benzyloxycarbonylpyrrolidin-2-ylmethyl)-1H-indoles and 3-(N-Benzyloxycarbonylpiperid-2-ylmethyl)-1H-indole Via the Palladium Catalyzed Cyclization of 1-(N-Benzyloxycarbonylpyrrolidin-2-yl)-3-(N-(2-halophenyl)-N-trifluoroacetylamino)propenes and 1-(N-Benzyloxycarbonylpiperid-2-yl)-3-(N-(2-halophenyl)-N-trifluoroacetylamino)propenes

A mixture of the 1-(N-benzyloxycarbonylpyrrolidin-2-yl)-3-(N-(2-halophenyl)-N-trifluoroacetylamino)propene or the1-(N-benzyloxycarbonylpiperid-2-yl)-3-(N-(2-halophenyl)-N-trifluoroacetylamino)propene (2.00 mmol), tetrabutylammonium chloride (2.00 mmol), and palladium(II) acetate (0.089 g, 0.40 mmol, 0.2 eq) in a solution of triethylamine (8 mL) and anhydrous N,N-dimethylformamide (4 mL) was heated at reflux under nitrogen for 2 hours. The resulting reaction mixture was evaporated under reduced pressure, and the residue was partitioned between ethyl acetate (25 mL) and water (25 mL). The ethyl acetate layer was removed, and the aqueous layer was extracted with additional ethyl acetate (25 mL). The organic extracts were combined, dried (MgSO₄), and evaporated under reduced pressure. The residue was column chromatographed using silica gel (approximately 50 g) amd elution with either a diethyl ether gradient in methylene chloride or an acetone gradient in methylene chloride to afford the corresponding 3-(N-benzyloxycarbonylpyrrolidin-2-ylmethyl)-1H-indole or the 3-(N-benzyloxycarbonylpiperid-2-ylmethyl)-1H-indole.

Following this procedure the following compounds were prepared:
A. **(R)-3-(N-Benzyloxycarbonylpyrrolidin-2-ylmethyl)-1H-indole**
   (R)-1-(N-Benzyloxycarbonylpyrrolidin-2-yl)-3-(N-(2-iodophenyl)-N-trifluoroacetyl-amino)propene was used. Column chromatography afforded the title compound as a clear, pale brown oil: ¹H NMR (CDCl₃) δ 8.05 (br s, indole NH), 7.49-7.34 (m, 7H), 7.17 (br t, 1H), 7.02 (br s, 1H), 6.95 (br s, 1H), 5.24 (s, 2H), 4.28-4.14 (br m, 1H), 3.52-3.41 (m, 2H), 3.28 (br d, 1H), 2.79-2.63 (m, 1H), 1.90-1.70 (m, 4H); LRMS (m/z, relative intensity) 334 (10, M+), 204 (16), 160 (39), 130 (39), 91 (100).
B. **(R)-3-(N-Benzyloxycarbonylpyrrolidin-2-ylmethyl)-5-(methylaminosulfonylmethyl)-1H-indole**
   (R)-1-(N-Benzyloxycarbonylpyrrolidin-2-yl)-3-(N-(2-bromo-4-methylaminosulfonylmethylphenyl)-N-trifluoroacetylamino)propene was used. Column chromatography afforded the title compound as an off-white foam: IR (CHCl₃) 1673, 1410, 1358, 1324, 1118, 1092 cm^{-l}; LRMS (m/z, relative intensity) 441 (9, M+), 237 (29), 204 (77), 160 (97), 143 (73), 91 (100); HRMS: calculated for C₁₃H₂₇N₃O₄S: 441.1724; found: 441.1704.
C. **(R)-3-(N-Benzyloxycarbonylpyrrolidin-2-ylmethyl-5-cyano-1H-indole**
   (R)-1-(N-Benzyloxycarbonylpyrrolidin-2-yl)-3-(N-(2-bromo-4-cyanophenyl)-N-trifluoroacetylamino)propene was used. Column chromatography afforded the title compound as a white foam: IR (1% solution in CHCl₃) 2215, 1687 cm⁻¹; ¹³C NMR [Note: due to slow nitrogen inversion two conformers of the products are seen by NMR spectroscopy] (CDCl₃) δ 155.1, 137.9, 137.0, 128.8, 128.5, 128.4, 128.0, 127.8, 124.9, 124.6, 121.0, 114.0, 113.9, 112.1, 102.3, 67.2, 66.7, 58.5, 57.6, 47.0, 46.7, 30.3, 30.0, 29.6, 28.8, 23.6, 22.7. Anal. calcd for C₂₂H₂₁N₃O₂•0.25 C₂H₄O₂ [acetic acid]: C, 72.17; H, 5.92; N, 11.22. Found: C, 72.28; H, 5.76; N, 10.95.
D. **(R,S)-3-(N-Benzyloxycarbonylpiperid-2-ylmethyl)-5-(methylaminosulfonylmethyl-1H-indole**
   (R,S)-1-(N-Benzyloxycarbonylpiperid-2-yl)-3-(N-(2-bromo-4-methylaminosulfonyl-methylphenyl)-N-trifluoroacetylamino)propene was used. Column chromatography afforded the title compound as an off-white foam: ¹³C NMR [Note: due to slow nitrogen inverion two conformers of the products are since by NMR spectroscopy] (CHCl₃) δ 162.5, 136.9, 136.2, 128.4, 127.6, 124.5, 123.3, 120.8, 120.3, 111.5, 66.8, 57.4, 39.5, 36.5, 31.4, 29.8, 25.8, 25.5, 18.8; LRMS (m/z, relative intensity) 445 (5, M+), 361 (4), 238 (40), 218 (80), 174 (100), 143 (53); HRMS calculated for C₂₄H₂₉N₃O₄S: 455.1880; found: 455.1899.

### EXAMPLE 8

### (R)-3-(N-Benzyloxycarbonylpyrrolidin-2-ylmethyl)-5-methoxy-1H-indole

To a stirred mixture of lithium borohydride (0.092 g, 4.22 mmol, 2 eq) in anhydrous tetrahydrofuran (5 mL) at 0°C was added a solution of the (R)-3-(N-benzyloxycarbonylpyrrolidin-2-ylcarbonyl)-5-methoxy-1H-indole (0.80 g, 2.11 mmol) in anhydrous tetrahydrofuran (8 mL). The resultant mixture was heated at reflux under nitrogen for 1 hour. The reaction mixture was cooled, and water (1 mL) was added carefully, followed by ethyl acetate (20 mL). The resultant mixture was stirred at room temperature for 30 minutes, dried (MgSO₄), filtered through diatomaceous earth, and the filtrate was evaporated under reduced pressure. The residue was column chromatographed using silica gel (approximately 50 g) and elution with ethyl acetate/hexanes [1:1] afforded (R)-3-(N-benzyloxycarbonylpyrrolidin-2-ylmethyl)-5-methoxy-1H-indole as a colorless gum: ¹³C NMR [Note: due to slow nitrogen inversion two conformers of the products are seen by NMR spectroscopy] (CDCl₃) 6 162.5, 136.9, 1.36.2, 128.4, 127.8, 127.6, 124.5, 123.3, 120.8, 120.3, 111.5, 66.8, 57.4, 39.5, 36.5, 31.4, 29.8, 25.8, 25.5, 18.8; LRMS (m/z, relative intensity) 364 (30, M+), 204 (17), 160 (92), 145 (17), 117 (13), 91 (100). Anal. calcd for C₂₂H₂₄N₂O₃•0.5 H₂O: C, 70.76; H, 6.75; N, 7.50. Found: C, 70.70; H, 6.94; N, 7.15.

### EXAMPLE 9

### General Procedure for the Formation of 1-(N-Benzyloxycarbonylpyrrolidin-2-yl)-3-(N-(2-halophenyl)-N-trifluoroacetylamino)propenes and 1-(N-Benzyloxycarbonylpiperid-2-yl)-3-(N-(2-halophenyl)-N-trifluoroacetylamino)propenes from the Mitsunobu Coupling of 2-Halo-N-trifluoroacetylanilines with 1-(N-Benzyloxycarbonylpyrrolidin-2-yl)-3-hydroxypropene or 1-(N-Benzyloxycarbonylpiperid-2-yl)-3-hydroxypronene

To a stirred mixture of 1-(N-benzyloxycarbonylpyrrolidin-2-yl)-3-hydroxypropene or 1-(N-benzyloxycarbonylpiperid-2-yl)-3-hydroxy-propene (R, or S, or racemate 2.00 mmol), the 2-halo-N-trifluoroacetylaniline (2.5 mmol, 1.25 eq), and triphenylphosphine (0.655 g, 2.50 mmol, 1.25 eq) in anhydrous tetrahydrofuran at 0°C under a nitrogen atmosphere was added diethyl azodicarboxylate (0.39 mL, 2.48 mmol, 1.25 eq) dropwise. The reaction solution was slowly warmed to 25°C over the course of 2 hours, and then stirred at 25°C under a nitrogen atmosphere for an additional 12 hours. The resulting reaction solution was evaporated under reduced pressure, and the residue was column chromatographed using silica gel (approximately 50 g) and elution with either a diethyl ether gradient in hexanes or an ethyl acetate gradient in hexanes to afford the corresponding 1-(N-benzyloxycarbonylpyrrolidin-2-yl)-3-(N-(2-halophenyl)-N-trifluoroacetylamino)propene or 1-(N-benzyloxycarbonylpiperid-2-yl)-3-(N-(2-halophenyl)-N-trifluoroacetylamino)propene.

Following this procedure the following compounds were prepared:
A. **(R)-1-(N-Benzyloxycarbonylpyrrolidin-2-yl)-3-(N-(2-iodophenyl)-N-trifluoroacetylamino)propene**
   (R)-1-(N-Benzyloxycarbonylpyrrolidin-2-yl)-3-hydroxypropene and 2-iodo-N-trifluoro-acetylaniline were used. Column chromatography afforded the title compound as a clear, colorless oil: ¹H NMR (CDCl₃) δ 7.88 (br d, 1H), 7.43-6.89 (m, 10H), 5.70-5.35 (m, 2H), 5.13 (br s, 2H), 5.00-4.75 (m, 1H), 4.40-4.29 (m, 1H), 3.60-3.42 (m, 3H), 2.05-1.45 (m, 4H); LRMS (FAB, m/z, relative intensity) 559 (100, [MH+]), 515 (52), 451 (15), 244 (7).
B. **(R)-1-(N-Benzyloxycarbonylpyrrolidin-2-yl)-3-(N-(2-bromo-4-methylaminosulfonylmethylphenyl)-N-trifluoro acetylamino)propene**
   (R)-1-(N-Benzyloxycarbonylpyrrolidin-2-yl)-3-hydroxypropene and 2-bromo-4-methylaminosulfonylmethyl-N-trifluoroacetylaniline were used. Column chromatography using elution with 4% acetone in methylene chloride afforded the title compound as a white foam (44%): FAB LRMS (m/z, relative intensity) 620 ([MH+ with ⁸¹Br], 618 ([MH+ with ⁷⁹Br], 98), 576 (50), 574 (63), 512 (17), 484 (33).
C. **(R)-1-(N-Benzyloxycarbonylpyrrolidin-2-yl)-3-(N-(2-bromo-4-cyanophenyl)-N-trifluoroacetylamino)propene**
   (R)-1-(N-Benzyloxycarbonylpyrrolidin-2-yl)-3-hydroxypropene and 2-bromo-4-cyano-N-trifluoroacetylaniline were used. Column chromatography using elution with a gradient of diethyl ether (5% - 100%) in methylene chloride afforded the title compound as a clear, colorless oil: IR (CHCl₃) 2231, 1702, 1157 cm⁻¹; LRMS (m/z, relative intensity) 537 ([MH+ with ⁸¹Br], 13), 535 ([MH+ with ⁷⁹Br], 13), 402 (29), 400 (30), 294 (55), 292 (57), 244 (80), 213 (89), 91 (100); Anal. calcd for C₂₄BRF₃H₂₁N₃O₃•0.2 H₂O: C, 53.39; H, 3.99; N, 7.78. Found: C, 53.25; H, 3.95; N, 7.98.
D. **(R,S)-1-(N-Benzyloxycarbonylpiperid-2-yl)-3-(N-(2-bromo-4-methylaminosulfonylmethylphenyl)-N-trifluoroacetylamino)propene**
   (R,S)-1-(N-Benzyloxycarbonylpiperid-2-yl)-3-hydroxypropene and 2-bromo-4-methylaminosulfonylmethyl-N-trifluoroacetylaniline were used. Column chromatography using elution with 20% acetonitrile in methylene chloride afforded the title compound as a white foam: FAB LRMS (m/z, relative intensity) 634 ([MH+ with ⁸¹Br], 26), 632 ([MH+ with ⁷⁹Br], 22), 590 (35), 588 (43), 401 (33), 327 (48), 281 (75), 207 (90), 147 (100); FAB HRMS: calculated for C₂₆H₂₉BrF₃N₃O₅S•[H+] 632.1043, found 632.1047 [for ⁷⁹Br and ³²S].

### EXAMPLE 10

### General Synthesis of 2-Halo-N-trifluoroacetylanilines from Reaction of 2-Haloanilines and Trifluoroacetic Anhydride

To a stirred solution of the 2-haloaniline (2.00 mmol) and pyridine (0.18 mL, 2.22 mmol, 1.1 eq) in anhydrous methylene chloride (10 mL) at 0°C under a nitrogen atmosphere was added dropwise trifluoroacetic anhydride (0.31 mL, 2.19 mmol, 1.1 eq). The resultant reaction mixture was stirred at 0°C under a nitrogen atmosphere for 3 hours. A saturated solution of sodium hydrogen carbonate was added (15 mL), and this aqueous mixture was extracted with ethyl acetate (3 x 15 mL). The extracts were combined, dried (MgSO₄), and evaporated under reduced pressure. The residue was column chromatographed using silica gel (approximately 50 g) and elution with an ethyl acetate gradient in hexanes to afford the corresponding 2-halo-N-trifluoroacetylaniline.

Following this procedure the following compounds were prepared:
A. **2-Iodo-N-trifluoroacetylaniline**
   2-Iodoaniline was used. Evaporation of the ethyl acetate extracts afforded the title compound directly as a white solid: mp, 105.0-106.5°C; FAB LRMS (m/z relative intensity) 316 ([MH+], 8), 155 (80), 135 (26), 119 (100); ¹³C NMR (acetone-d₆) δ 206.2, 140.4, 130.2, 130.1, 128.2.
B. **2-Bromo-4-methylaminosulfonylmethyl-N-trifluoroacetylaniline**
   2-Bromo-4-methylaminosulfonylmethylaniline was used. Evaporation of the ethyl acetate extracts afforded the title compound directly as a white solid: mp, 164.0-166.0°C. Anal. calcd for C₁₀H₁₀BrF₃N₂O₃S: C, 32.02; H, 2.69; N, 7.47. Found: C, 32.18; H, 2.67; N, 7.30.
C. **2-Bromo-4-cyano-N-trifluoroaocetylaniline**
   2-Bromo-4-aminocarbonylaniline was used. Dehydration of the carboxamide also occurred in this reaction. Column chromatography using ethyl acetate/hexanes afforded the title compound as a white solid: mp, 125-130°C; ¹H NMR (DMSO-d₆) δ 11.6 (br s, NH), 8.37 (d, J=1.8 Hz, 1H), 7.96 (dd, J=1.8 and 8.2 Hz, 1H), 7.71 (d, J=8.2 Hz, 1H).

### EXAMPLE 11

### General Procedure for the Bromination of Anilines to Form 2-Bromoanilines

To a stirred solution of the aniline (2.00 mmol) and sodium hydrogen carbonate (0.21 g, 2.50 mmol, 1.25 eq) in methanol (10 mL) at 0°C was added dropwise bromine (0.113 mL, 2.19 mmol, 1.1 eq). The resulting reaction mixture was then stirred at 25°C for 30 minutes. The reaction mixture was then evaporated under reduced pressure, and the residue was placed in a saturated solution of sodium hydrogen carbonate (10 mL). This aqueous mixture was extracted with ethyl acetate (3 x 15 mL). The extracts were combined, dried (MgSO₄), and evaporated under reduced pressure. The residue was column chromatographed using silica gel (approximately 50 g) and elution with an appropriate solvent system to afford the corresponding 2-bromoaniline.

Following this procedure the following compounds were prepared:
A. **2-Bromo-4-methylaminosulfonylmethylaniline**
   4-Methylaminosulfonylmethylaniline (M.D. Dowle, et al. Eur. Pat. Appl. EP225,726) was used. Column chromatography using elution with 40% ethyl acetate in hexanes afforded the title compound as a white solid: mp, 104.0-107.0°C. Anal. calcd for C₈H₁₁BrN₂O₂S: C, 34.42; H, 3.97; N, 10.04. Found: C, 34.66; H, 3.96; N, 9.96.
B. **4-Aminocarbonyl-2-bromoaniline**
   4-Aminobenzamide was used. Column Chromatography using elution with a ethyl acetate gradient (25-50%) in methylene chloride afforded the title compound as a white solid: mp, 144.5-146.0°C; ¹H NMR (DMSO-d₆) δ 7.93 (d, J=2.0 Hz, 1H), 7.70 (br s, amide NH), 7.62 (dd, J=2.0 and 8.5 Hz, 1H), 7.05 (br s, amide NH), 6.77 (d, J=8.5 Hz, 1H), 5.85 (s, aniline NH₂).

### EXAMPLE 12

### 1-(N-Benzyloxycarbonylpyrrolidin-2-yl)-3-hydroxypropene or 1-(N-Benzyloxycarbonylpiperid-2-yl)-3-hydroxypropene

To a stirred solution of either ethyl 3-(N-benzyloxycarbonylpyrrolidin-2-yl)-2-propenoate or ethyl-3-(N-benzyloxycarbonylpiperid-2-yl)-2-propenoate (R, or S, or racemate, 10.00 mmol) in anhydrous tetrahydrofuran (75 mL) at -78°C under nitrogen was added dropwise a solution of diisobutylaluminium hydride (1.0 M in hexanes, 12.0 mL, 22.0 mmol, 2.2 eq). The resulting solution was stirred at -78°C under nitrogen for 30 minutes. The reaction solution was then allowed to warmed to room temperature over the course of 2 hours. A saturated solution of sodium hydrogen carbonate (50 mL) was added, and the aqueous mixture was extracted with ethyl acetate (3 x 50 mL). The extracts were combined, dried (MgSO₄) , and evaporated under reduced pressure. Column chromatography of the residue with diethyl ether/hexanes [1:1] afforded either 1-(N-benzyloxycarbonylpyrrolidin-2-yl)-3-hydroxypropene or 1-(N-benzyloxycarbonyl-piperid-2-yl)-3-hydroxypropene.

Following the procedure the following compounds were prepared:
A. **(R)-1-(N-Benzyloxycarbonylpyrrolidin-2-yl)-3-hydroxypropene**
   (R)-Ethyl 3-(N-benzyloxycarbonylpyrrolidin-2-yl)-2-propenoate was used. Chromatography of the extraction residue afforded the title compound as a clear, colorless oil: ¹H NMR (CDCl₃) δ 7.40-7.25 (m, 5H), 5.75-5.53 (m, 2H), 5.20-5.00 (m, 2H), 4.38 (br m, 1H), 4.06 (br d, J=13.7 Hz, 2H), 3.45 (br t, J=7.0 Hz, 1H), 2.03-1.68 (m, 4H); [α²⁵ = +34° (MeOH, c=1.0); HRMS: calculated for C₁₅H₁₉NO₃ 261.1365, found 261.1356.
B. **(R,S)-1-(N-Benzyloxycarbonylpiperid-2-yl)-3-hydroxypropene**
   (R,S)-Ethyl 3-(N-benzyloxycarbonylpiperid-2-yl)-2-propenoate was used. Chromatography of the extraction residue afforded the title compound as a clear, colorless oil: LRMS (m/z, relative intensity) 257 (3), 212 (12), 193 (8), 175 (65), 173 (100), 145 (27), 109 (24), 91 (87); ¹H NMR (CDCl₃) δ7.40-7.20 (m, 5H), 5.70-5.61 (m, 2H), 5.14 (d, J=17.6 Hz, 1H), 5.10 (d, J=17.5 Hz, 1H), 4.88 (br m, 1H), 4.14-4.00 (m, 3H), 2.91 (br t, J=12.7 Hz, 1H), 1.78-1.47 (m, 6H). Anal. calcd for C₁₆H₂₁NO₃•0.1 H₂O: C, 69.34; H, 7.71; N, 5.05. Found: 69.38; H, 7.84; N, 5.16.

### EXAMPLE 13

### Synthesis of Ethyl 3-(N-Benzyloxycarbonylpyrrolidin-2-yl)-2-propenoate or Ethyl 3-(N-Benzyloxycarbonylpiperid-2-yl)-2-propenoate

To a stirred solution of N-carbobenzyloxypyrrolidine-2-carboxaldehyde or N-carbobenzyloxypiperidine-2-carboxaldehyde (5.00 mmol) [S. Kiyooka, et al., J. Org. Chem., 5409 (1989) and Y. Hamada, et al., Chem. Pharm. Bull., 1921 (1982)] in anhydrous tetrahydrofuran at -78°C was added (carbethoxymethylene) triphenylphosphorane (2.09 g, 6.00 mmol. 1.2 eq) as a solid portionwise. The resulting reaction mixture was stirred at room temperature under nitrogen for 2 hours, and then heated at reflux under nitrogen for 1 hour. The reaction mixture was evaporated under reduced pressure and the residue was column chromatographed using silica gel (approximately 100 g) and elution with 20% diethyl ether in hexanes to afford either ethyl 3-(N-benzyloxycarbonylpyrrolidin-2-yl)-2-propenoate or ethyl 3-(N-benzyloxycarbonylpiperid-2-yl)-2-propenoate.

### A. (R)-Ethyl 3-(N-Benzyloxycarbonylpyrrolidin-2-yl)-2-propenoate

(R)-N-Carbobenzyloxypyrrolidine-2-carboxaldehyde was used. Chromatography as described above afforded the title compound as a clear, colorless oil: ¹H NMR (CDCl₃-d₆) δ 7.34-7.25 (m, 5H), 6.89-6.76 (m, 1H), 5.88-5.74 (m, 1H), 5.18-5.05 (m, 2H), 4.60-4.43 (m, 1H), 4.17 (q, J=7.1 Hz, 2H), 3.55-3.40 (m, 2H), 2.11-2.00 (m, 1H), 1.90-1.75 (m, 3H), 1.28 (t, J=7.1 Hz, 3H); ¹³C NMR (CDCl₃) [Note: due to slow nitrogen inversion two conformers of the products are seen by NMR spectroscopy] δ 166.3, 154.7, 147.9, 147.4, 136.6, 128.4, 127.9, 120.9, 66.9, 65.8, 60.4, 58.1, 57.7, 46.8, 46.4, 31.6, 30.8, 23.6, 22.8, 22.6, 15.3, 14.2.

### B. (R,S)-Ethyl 3-(N-Benzyloxycarbonlpiperid-2-yl)-2-propenoate

(R,S)-N-Carbobenzyloxypiperidine-2-carboxaldehyde was used. Chromatography as described above afforded the title compound as a clear, colorless oil: ¹H NMR (CDCl₃-d₆) δ 7.36-7.27 (m, 5H), 6.85 (dd, J=4.4 and 16.3 Hz, 1H), 5.80 (dd, J-2.4 and 16.3 Hz, 1H), 5.11 (s, 2H), 5.01 (br m, 1H), 4.17 (q, J=6.7 Hz, 2H), 4.05 (br d, J=12.6 Hz, 1H), 2.87 (br t, 1H), 1.80-1.35 (m, 6H), 1.27 (t, J=6.6 Hz, 3H); FAB LRMS (m/z, relative intensity) 318 ([MH+], 100), 274 (86), 228 (14), 210 (21), 182 (43), 138 (32).

### EXAMPLE 14

### (R)-5-Amino-3-(N-methylpyrrolidin-2ylmethyl)indole

A mixture of (R)-5-dibenzylamino-3-(N-methylpyrrolidin-2-ylmethyl)indole (1.08 g, 2.64 mmol) and palladium [II] hydroxide on carbon (0.6 g) in absolute ethanol (25 mL) was shaken under a hydrogen atmoshpere (3 atm) at 40°C for 4 hours. The resulting mixture was filtered through diatmaceous earth, and the filtrate was evaporated under pressure to afford the title compound (0.60 g, 2.62 mmol, 99%) as a white foam: ¹H NMR (DMSO-d₆) δ 10.65 (br s, NH), 7.14 (d, J=2.2 Hz, 1H), 7.12 (d, J=8.6 Hz, 1H), 6.85 (d, J=1.6 Hz, 1H), 6.60 (dd, J=2.0 and 8.6 Hz, 1H), 3.63-2.83 (m, 7H), 2.78 (s, 3H), 2.05-1.67 (m, 4H); [α]²⁵=+9° (MeOH, c=1.0); HRMS: calculated for C₁₄H₁₉N₃: 229.1575; found 229.1593.

### EXAMPLE 15

### General Synthesis of 5-Carbonylamino-3-(N-methylpyrrolidin-2-ylmethyl)-1H-indoles and 5-Sulfonylamino-3-(N-methylpyrrolidin-2-ylmethyl)-1H-indoles

To a stirred solution of (R)-5-amino-3-(N-methylpyrrolidin-2-ylmethyl)indole (0.229 g, 1.00 mmol) and triethylamine (0.21 mL, 1.5 mmol, 1.5 eq) in anhydrous acetonitrile (3 mL) at 0°C under nitrogen was added the appropriate carbonyl chloride or sulfonyl chloride (1.5 mmol, 1.5 eq). The resulting reaction mixture was stirred at room temperature for 12 hours. The reaction mixture was then evaporated under reduced pressure, and the residue was column chromatographed using silica gel (approximately 25 g) and elution with an appropriate solvent system afforded the appropriate 5-carbonylamino-3-(N-methylpyrrolidin-2-ylmethyl)-1H-indole or 5-sulfonylamino-3-(N-methylpyrrolidin-2-ylmethyl)-1H-indole.

Following this procedure the following compounds were prepared:
A. **(R)-5-Benzyloxycarbonylamino-3-(N-methylpyrrolidin-2-ylmethyl)-1H-indole**
   Benzyl chloroformate was used. Column chromatography using elution with triethylamine/acetone/ethyl acetate [2:10:88] afforded the title compound as an off-white foam: ¹³C NMR (CDCl₃) δ 163.3, 136.4, 133.6, 129.8, 128.6, 128.2, 127.9, 126.0, 123.2, 113.8, 111.4, 110.1, 66.8, 66.5, 57.5, 40.8, 31.5, 29.8, 21.8; LRMS (m/z, relative intensity) 363 (M+, 12), 279 (7), 184 (7), 171 (33), 108 (100); HRMS: calculated for C₂₂H₂₅N₃O₂ 363.1949, found 363.1926. Anal. calcd for C₂₂H₂₅N₃O₂•0.4 C₄H₈O₄ [ethyl acetate]: C, 71.09; H, 7.13; N, 10.54. Found: C, 70.82; H, 7.03; N, 10.58.
B. **(R)-3-(N-Methylopyrrolidin-2-ylmethyl)-5-methylsulfonamido-1H-indole**
   Methanesulfonyl chloride was used. Column chromatography using elution with triethylamine/acetone/ethyl acetate [1:3:6] afforded the title compound as a white foam: ¹³C NMR (CDCl₃) δ 134.9, 128.3, 128.2, 123.6, 119.3, 115.0, 113.9, 112.0, 66.7, 57.3, 40.7, 38.7, 31.3, 29.4, 21.7; HRMS: calculated for C₁₅H₂₁N₃O₂S [with ³²S] 307.1356, found 307.1323.
C. **(R)-5-Acetylamino-3-(N-methylpyrrolidin-2-ylmethyl)-1H-indole**
   Acetyl chloride was used. Column chromatography using elution with triethylamine/acetone/ethyl acetate [1:3:6] afforded the title compound as a white foam: ¹³C NMR (acetone-d₆) δ 168.3, 134.4, 132.2, 128.7, 124.1, 115.7, 113.8, 111.6, 110.2, 67.3, 58.0, 40.9, 31.9, 30.5, 24.1, 22.5; LRMS (m/z, relative intensity) 271 (M+, 39), 241 (4), 207 (5), 187 (20), 144 (20), 84 (100); HRMS: calculated for C₁₆H₂₁N₃O 271.1686, found 271.1693. Anal. calcd for C₁₆H₂₁N₃O•1.15 H₂O: C, 65.80; H, 8.04; N, 14.39. Found: C, 65.99; H, 7.90; N, 13.99.
D. **(R)-5-N,N-Dimethylaminocarbonylamino-3-(N-methylpyrrolidin-2-ylmethyl)-1H-indole**
   Dimethylcarbamyl chloride was used. Column chromatography using elution with methylene chloride/methanol/ ammonium hydroxide [9:1:0.1] afforded the title compound as an off white foam: ¹H NMR (CDCl₃) δ 8.95 (br s, 1H), 7.49 (br s, 1H), 7.15-7.06 (m, 2H), 6.82 (d, J=1.9 Hz, 1H), 6.44 (br s, 1H), 3.12-3.05 (m, 2H), 3.00 (s, 6H), 2.58-2.40 (m, 2H), 2.40 (s, 3H), 2.18 (br q, J=8.1 Hz, 1H), 1.83-1.47 (m, 4H); ¹³C NMR (CDCl₃) δ 157.2, 133.8, 130.5, 127.7, 123.2, 117.8, 113.0, 112.0, 111.3, 66.5, 57.4, 40.6, 36.4, 31.4, 29.8, 21.7; LRMS (m/z, relative intensity) 300 (M+, 50), 217 (10), 171 (20), 84 (100); HRMS: calculated for C₁₇H₂₄N₄O 300.1952, found 300.1957.
E. **(R)-5-Trifluoroacetylamino-3-(N-methylpyrrolidin-2-ylmethyl)-1H-indole**
   Trifluoroacetic anhydride was used. Column chromatography using elution with methylene chloride/methanol/ammonium hydroxide [9:1:0-.1] afforded the title compound as an off white foam: ¹H NMR (CDCl₃) δ 8.99 (br s, 1H), 7.80 (br s, 1H), 7.27-7.19 (m, 2H), 6.95 (d, J=1.4 Hz, 1H0, 3.16-3.08 (m, 2H) , 2.58 (dd, J=9.4 and 13.5 Hz, 1H). 2.57-2.43 (m, 1H), 2.43 (m, 1H), 2.43 (s, 3H), 2.22 (dd, J=9.2 and 17.5 Hz, 1H), 1.85-1.46 (m, 4H); ¹³C NMR (CDCl₃) δ 134.5, 127.7, 126.9, 123.8, 116.1, 113.9, 111.9, 111.6, 104.1, 66.6, 57.3, 40.6, 31.3, 29.5, 21.7; HRMS: calculated for C₁₆H₁₈F₃N₃O 325.1403, found 325.1378.

### EXAMPLE 16

### (R)-3-(N-Methylpyrrolodin-2-ylmethyl)-5-(2-methylsulfonamidomethyl)-1H-indole

To a stirred mixture of (R)-5-aminomethyl-3-(N-methylpyrrolidin-2-ylmethyl)-1H-indole (0.113 g, 0.46 mmol) and pyridine (50µL, 0.93 mmol, 2.0 eq) in a solution of dimethylformamide and acetontrile (1:3, respectively, 2 mL total) at 0°C under nitrogen was added methanesulfonyl chloride dropwise (44µL, 0.56 mmol, 1.3 eq). The resulting reaction solution was stirred at room temperature under nitrogen for 1 hour, and then it was evaporated under reduced pressure. The residual oil was column chromatographed using silica gel (6 g) and elution with methylene chloride/methanol/ammonium hydroxide [9:1:0.1] to afford the title compound (0.044 g, 0.14 mmol, 30%) as a white foam: ¹H NMR (CDCl₃) δ 8.25 (br s, NH), 7.54 (br s, 1H), 7.35 (d, J=8.4 Hz, 1H), 7.17 (dd, J=1.6 and 8.4 Hz, 1H), 7.06 (d, J=1.8 Hz, 1H), 4.78 (br s, NH), 4.42 (s, 2H), 3.20-3,12 (m, 2H), 2.87 (s, 3H), 2.64 (dd, J=9.4 and 13.9 Hz, 1H). 2.54-2.43 (m, 1H), 2.47 (s, 3H), 2.25 (dd, J=9.3 and 17.3, 1H), 1.86-1.52 (m, 4H); ¹³C NMR (CDCl₃) δ 135.8, 127.8, 127.3, 123.0, 122.0, 118.5, 113.7, 111.6, 66.7, 57.4, 47.9, 40.9, 40.7, 31.3, 29.5, 21.7; LRMS (m/z relative intensity) 321 (28), 320 (M+, 26), 237 (51), 157 (100), 143 (64), 129 (78) ; HRMS: calculated for C₁₆H₂₂N₃O₂S 320.1435, found 320.1453.

### EXAMPLE 17

### General Synthesis of Allylsulphonamides

A. **Allylsulphonamid**
   The title compound was prepared by the method of M. A. Belous and I. Ya. Postouski, Zhur. Obschei. Khim., 1950, 20, 1701.
B. **N-Methylallylsulphonamide**
   The title compound was prepared by an analogous procedure to above by using methylamine instead of ammonia. Anal. Calcd for C₅H₁₁NO₂S: C,40.25; H,7.43; N,9.38. Found: C,40.51; H,7.37; N,9.70.

### EXAMPLE 18

### Preparation of Ethylallylsulphone

The title compound was prepared by the method of R. J. Palmer and C. J. M. Stirling., J. Amer. Chem. Soc., 1980, 102, 7888.

### EXAMPLE 19

### General Synthesis of Vinyl Sulphonamides

Where the required vinylsulphonamide was not commercially available, they were prepared by the following procedure based on the procedure described in Zhur. Obschei. Khim., 1959, 29, 1494.
A. **N,N-Dimethylvinylsulphonamide**
   To a stirred solution of chloroethylsulphonyl chloride (25 g, 153 mmol) in dry diethyl ether (150 mL) at -10°C, was added dropwise a solution of dimethylamine (30.5 mL, 460 mmol) in dry diethyl ether (100 mL) over 5 minutes. After stirring for 90 minutes at -10°C the solution was filtered and evaporated in vacuo. The residue was distilled to give the title compound (9.5 g, 46%): b.p. 120-122°C (20 mm Hg). Anal. Calcd for C₄H₉NO₂S: C,35.54; H,6.71; N,10.36%. Found: C,35.36; H,6.37; N,10.19.
B. The following examples were prepared by the general procedure above, using the appropriate amine starting material. Purification was by distillation or column chromatography.

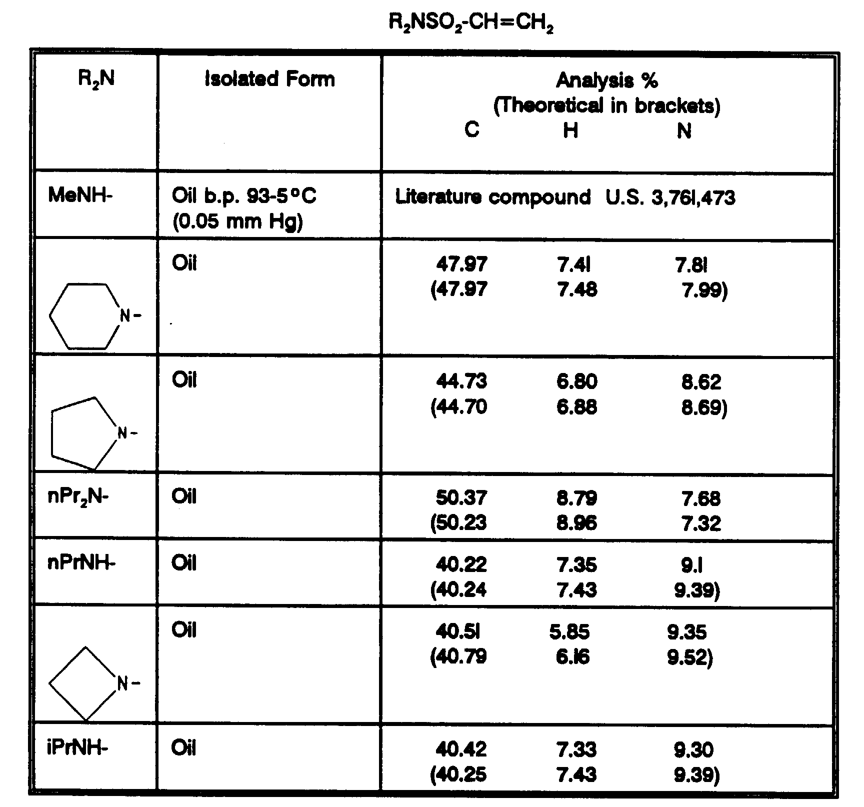

### EXAMPLE 20

### General Syhthesis of Vinyl Sulphones

Where the required vinyl sulphone was not commercially available, they were prepared from the corresponding thiols using the procedure described by J. M. Gaillot, Y Gelas-Mialhe and R. Vessiere Can. J. Chem., 1979, 57, 1958. The following examples are representative.

| RS-CH₂CH₂-OH | | | |
|---|---|---|---|
| R | Isolated Form | Analysis % (Theoretical in brackets) | |
| | | C | H |
| nPr | Oil 1/16 EtOAc | 48.68 | 9.79 |
| | 1/5 H₂0 | (48.76 | 10.06) |
| | | | |
| nBu | Oil | T.l.c - Rf. 0.26 | |
| | | (SiO₂, Ether/Hexane 1/1) | |

| RS-CH₂CH₂-Cl | | | |
|---|---|---|---|
| R | Isolated Form | Analysis % (Theoretical in brackets) | |
| | | C | H |
| nPr | Oil 1/5 H₂O 1/30 | 41.63 | 7.60 |
| | CH₂Cl₂ | (41.65 | 7.69) |
| nBu | Oil 1.0 H₂0 | 42.31 | 7.84 |
| | | (42.21 | 8.27) |

| RSO₂-CH₂CH₂Cl | | | |
|---|---|---|---|
| R | Isolated Form | Analysis % (Theoretical in brackets) | |
| | | C | H |
| nPr | Oil | 34.75 | 6.68 |
| | | (35.19 | 6.50) |
| nBu | Oil 1/15 CH₂Cl₂ | 38.41 | 7.01 |
| | | (38.27 | 6.95) |

| RSO₂-CH=CH₂ | | | |
|---|---|---|---|
| R | Isolated Form | Analysis % (Theoretical in brackets) | |
| nBu | Oil | 48.95 | 8.07 |
| | | (48.62 | 8.16) |

### EXAMPLE 21

### General Synthesis of indoles with 5-alkenyl substituents

A. **(R)-5-trans-(2-N,N-Dimethylaminocarbonylethenyl)**-**3-(N-methylpyrrolidin-2-ylmethyl)-1H-indole**
A mixture of N,N-dimethylacrylamide (134 ML, 1.3 mmol), tri-o-tolylphosphine (91 mg, 0.3 mmol), palladium (II) acetate (15 mg, 0.07 mmol), triethylamine (280 µL, 2 mmol) and (R)-5-bromo-3-(N-methylpyrrolidin-2-ylmethyl)-1H-indole was dissolved in anhydrous acetonitrile (5 mL) and refluxed for 24 hours under nitrogen. The reaction was partitioned between ethylacetate and aqueous sodium carbonate. The dried (Na₂SO₄) organic phase was evaporated and the residue purified by column chromatography on silica gel, eluting with CH₂Cl₂: MeOH: NH₄OH 96:3.5:0.5 to afford the title compound as a white foam (145 mg, 47%). Anal. Calcd for C₁₉H₂₃N₃O•1/9 CH₂Cl₂: C,71.56; H,7.87; N,13.10%. Found: C,71.29; H,8.15; N,13.05.
B. The following examples were prepared using the above procedure with the appropriate alkene starting material (available commercially, or prepared by routes outlined in this patent).

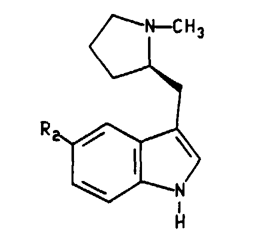

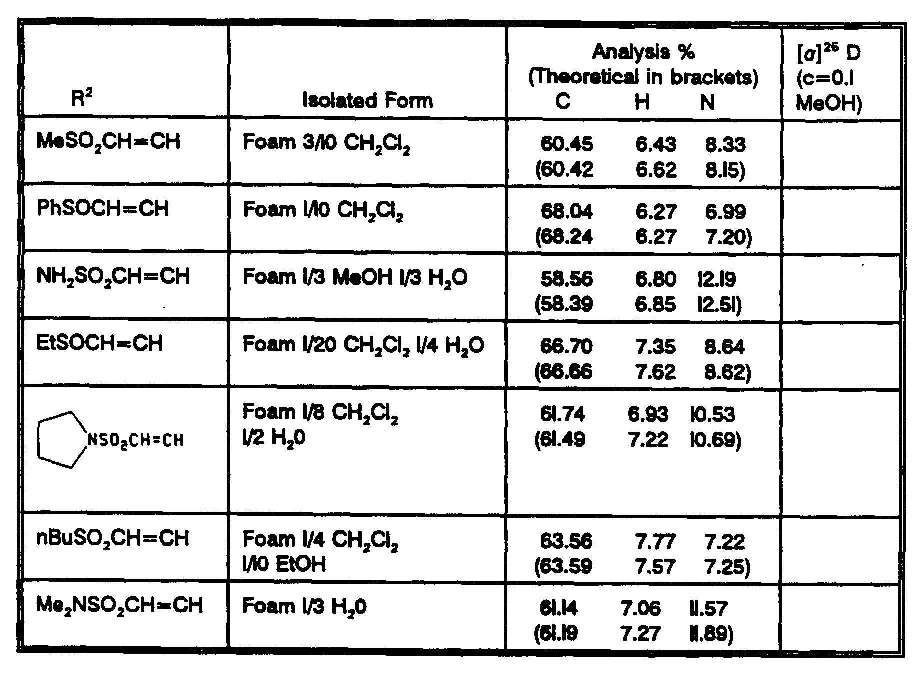

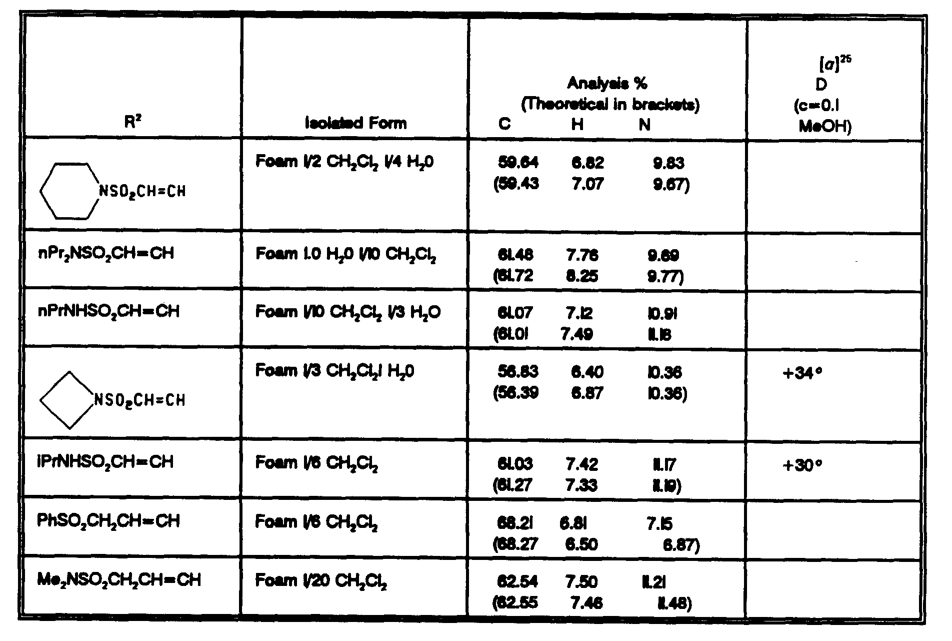

| R² | Isolated Form | Analysis % (Theoretical in brackets) | | | [α]²⁵ D (c=0.1 MeOH) |
|---|---|---|---|---|---|
| | | C | H | N | |
| NH₂SO₂CH₂CH=CH | Foam 0.1 CH₂Cl₂ 1.0 MeOH | 58.50 (58.13 | 6.93 7.30 | 11.22 11.24) | |
| EtSO₂CH₂CH=CH | Form | 65.56 (65.86 | 7.47 7.56 | 8.00 8.08) | |
| PhCONHCH₂CH=CH | Foam 0.1 CH₂Cl₂ | 75.00 (75.78 | 6.97 7.18 | 10.76 11.00 ) | +70° |
| MeSO₂NHCH₂CH=CH | Foam 0.1 CH₂Cl₂ | 61.05 (61.07 | 7.31 7.14 | 11.12 11.80) | |

C) The following compounds could be prepared by the procedure a) above but using the corresponding betachloroethylsulphone as starting material instead of an alkene. These reactions were preferably carried out in the presence of 3-6 equivalents of triethylamine.

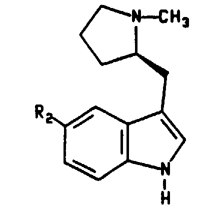

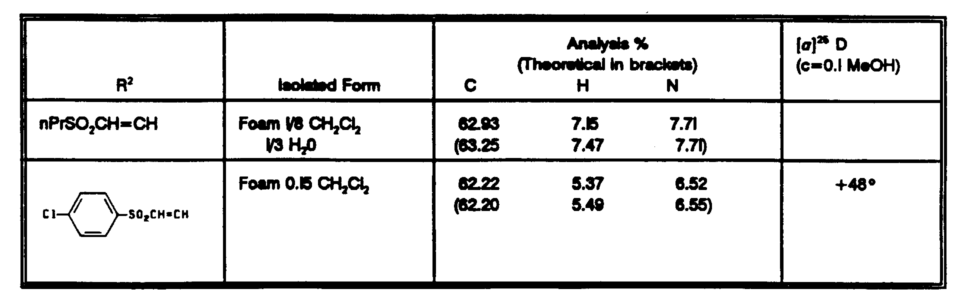

### EXAMPLE 22

### General Procedure for Hydrogenation of 5-alkenylindoles

A typical procedure is as follows:
A. **(R)-5-(2-Aminosulphonylethyl)-3-(N-methylpyrrolidin-2-ylmethyl-1H-indole**
   (R)-5-(2-Aminosulphonylethenyl)-3-(N-methyl-pyrrolidin-2-ylmethyl)-1H-indole (157 mg, 0.5 mmol) was dissolved in absolute ethanol (10 mL) and added to a solution of ethanolic hydrogen chloride 25 ml (prepared from acetyl chloride (38 ML, 0.53 mmol) and absolute ethanol (25 mL)). 10% palladium-on-carbon (125 mg) was added. This solution was hydrogenated under a hydrogen atmosphere (15 p.s.i.) at room temperature for 18 hours. The resultant reaction mixture was filtered through diatomaceous earth (Celite trademark or Arbacell-trademark)) washed with absolute ethanol and the combined filtrates evaporated in vacuo. The residue was purified by column chromatography on silica gel, eluting with a gradient solvent mixture up to CH₂Cl₂: MeOH:NH₄OH 93:7:1 to give the title compound as a colourless oil (80 mg, 51%). Anal. Calcd for C₁₆H₂₃N₂O•1/4 MeOH. 1/3 H₂O: C,58.21; H,7.36; N,12.54. Found: C,58.60; H,7.40; N,12.57. [α]²⁵ = +69° (c=0.1, MeOH).
B. The following examples were prepared by an analogous procedure to a) above.

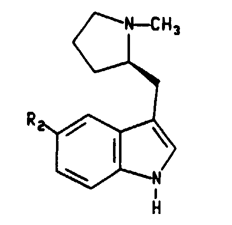

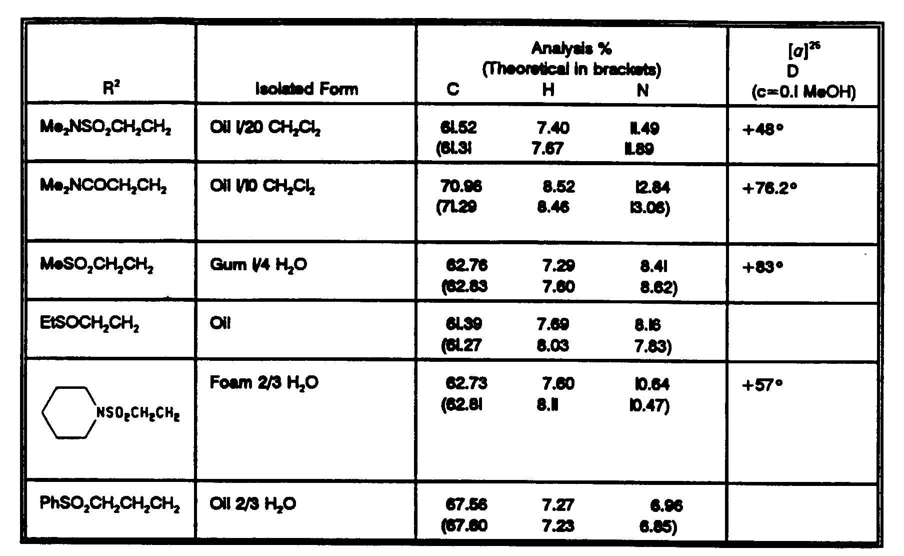

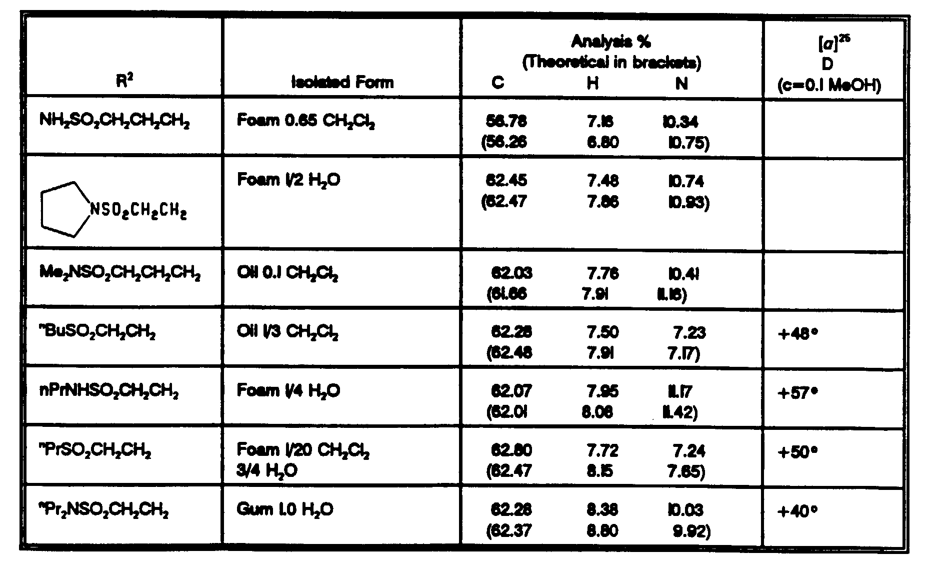

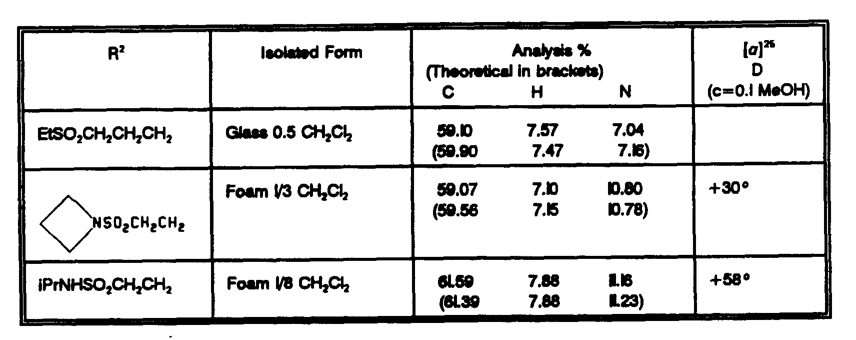

### EXAMPLE 23

### General Synthesis of (R)-5-(2-Ethylsulphonylethyl)-3-(pyrrolidin-2-ymethyl)-1H-indole

A. **(R)-3-(N-Benzyloxycarbonylpyrrolidin-2-yl-methyl)**-**5-bromo-1H-indole**
   (R)-3-(N-Benzyloxycarbonylpyrrolidin-2-yl-carbonyl)-5-bromo-1H-indole (0.67 g, 1.57 mmol) was dissolved in anhydrous tetrahydrofuran (20 mL) and at room temperature under nitrogen was added lithium borohydride (2 molar in tetrahydrofuran) (1.2 mL, 2.4 mmol). The reaction mixture was stirred at room temperature for 3 hours and warmed to reflux for 16 hours. After cooling to room temperature, 2NHCl (10 mL) was added dropwise and the reaction mixture partitioned between ethyl acetate and water. The separated organic phase was washed with saturated aqueous sodium hydrogen carbonate (2x), brine (1x), dried (Na₂SO₄), and evaporated in vacuo to give a colourless oil. Purification by column chromatography on silica gel, eluting with dichloromethane gave the title compound as an oil (0.32 g). TLC (SiO₂:CH₂Cl₂): R_{f}=0.2.
B. **(R)-5-(Ethylsulphonylethenyl)-3-(N-Benzyloxycarbonylpyrrolidin-2-ylmethyl)-1H-indole**
   The compound from procedure a) above was coupled with ethyl vinylsulphone under standard conditions described above, to give the title compound as a foam. Anal. Calcd for C₂₅H₂₈N₂O₄S•1/8 CH₂Cl₂: C,65.15; H,6.15; N,6.05. Found: C,65.16; H,6.17; N,5.97. [α]²⁵= -50° (0.1, MeOH).
C. **(R)-5-(2-Ethylsulphonylethyl)-3 (pyrrolidin-2-ylmethyl)-1H-indole**
   The compound prepared in procedure b) above, was hydrogenated under the standard condition described above, to give the title compound as a foam. Anal. Calcd for C₁₇H₂₄N₂O₂S•1/2 CH₂Cl₂: C,63.07; H,7.48; N,8.63. Found: C,62.90; H,7.25; N,8.58. [α]²⁵ = -11° (c=0.1, MeOH).

### EXAMPLE 24

### General Synthesis of (R)-3-(N-alkyl-pyrrolidin-2-ylmethyl)indoles

A. **(R)-3-(N-Ethylpyrrolidin-2-ylmethyl)-5-(2-ethyl****sulphonylethyl)-1H-indole**
To a solution of (R)-3-(pyrrolidin-2-ylmethyl)-5-(2-ethylsulphonylethyl)-1H-indole (0.27 g, 0.8 mmol) in dimethylformamide (dried over 4A sieves) (5 mls), was added sodium carbonate (90 mgs) and ethyl iodide (0.07 mls, 0.88 mmol) at room temperature. The mixture was heated at 120°C under nitrogen for 16 hours. After cooling to room temperature the reaction mixture was partitioned between ethyl acetate and water. The separated organic phase was washed with water (3x), dried (Na₂SO₄) and evaporated in vacuo to give an oil. Purification by column chromatography on silica gel, eluting with CH₂Cl₂: EtOH: NH₄OH (90:10:0.5) gave the title compound as a gum (100 mgs). Anal. Calcd for C₁₉H₂₈N₂O₂S•1/4 CH₂Cl₂. 1/2 H2O: C,61.04; H,7.85; N,7.40. Found: C,60.80; H,7.69; N,7.48.[α]²⁵ + 60° (c=0.1, MeOH).
B. The following examples were prepared using the procedure described in a) above but with the appropriate alkyl halide in place of ethyl iodide. The alkyl halide could be iodide or bromide with the optional presence of sodium iodide. Solvents used were either dimethylformamide or dimethylacetamide.

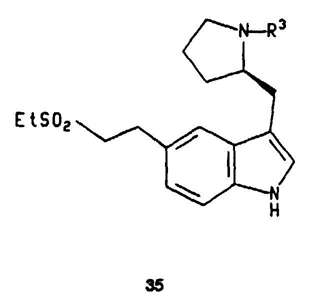

| R³ | Isolated Form | Analysis % (Theoretical in brackets) | | | [α]²⁵ D c=0.1 MeOH |
|---|---|---|---|---|---|
| | | C | H | N | |
| iPr | Gum 1/10 CH₂Cl₂ 1/4 H₂O | 64.16 (64.29 | 8.17 8.24 | 7.55 7.46) | +24° |
| * CH₃CH(CH₂CH₃) (isomer 1- R.f. 0.40 SiO₂, CH₂Cl₂:MeOH: NH₃ (90:10:1) | Gum 1/2 CH₂Cl₂ 1/4 H₂O | 60.68 (60.97 | 7.91 7.97 | 7.08 6.62) | -3° |
| * CH₃CH(CH₂CH₃) (isomer 2 - R.f. 0.38 SiO₂, CH₂Cl₂:MeOH:NH₃(90:10:1)) | Gum 1/8 CH₂Cl₂ | 65.19 (65.53 | 8.13 8.40 | 7.45 7.24) | +26° |
| nPr | Gum 1/20 CH₂Cl₂ 3/5 H₂O | 64.04 (63.77 | 8.19 8.36 | 7.52 7.42) | +62° |
| (CH₃)₂CHCH₂ | Gum 1/2 H₂O | 65.32 (65.40 | 8.49 8.63 | 6.87 7.26) | +80° |
| * CH₃(CH₃CH₂)CHCH₂ (S-isomer) | Gum 2/3 H₂O | 65.72 (65.63 | 8.82 8.85 | 7.10 6.96) | +65° |

### EXAMPLE 25

### (R)-3-(N-methylpyrrolidin-2-ylmethyl)-1H-indole

(R)-5-Bromo-3-(N-methylpyrrolidin-2-yl-methyl)-1H-indole (60 mg, 0.2 mmol) was dissolved in ethanol (1 mL) and hydrogenated over 10% palladium on carbon (45 mg) at 60 p.s.i. of hydrogen pressure at room temperature for 16 hours. The reaction mixture was evaporated to dryness, and the residue partitioned between ethyl acetate and 10% aqueous sodium carbonate. The organic phase was dried (Na₂SO₄), and evaporated in vacuo. The resulting residue was purified by column chromatography on silica gel (eluting with 89:10:1 CH₂Cl₂:MeOH:NH₄OH) to give the title compound (28 mg). Anal. Calcd for C₁₄H₁₈N₂•1/8 CH₂Cl₂ C,75.42; H,8.18; N,12.46. Found: C,75.50; H,8.51; N,12.09. [α]²⁵ = + 60.2° (c=0.088, CHCl₃).

### EXAMPLE 26

### (R)-3-(N-Benzyloxycarbonylpyrrolidin-2-ylcarbonyl)-5-bromo-1H-indole

Two solutions containing the reactants were prepared separately as follows: To a stirred solution of N-benzyloxycarbonyl-D-proline (1.0 g) in anhydrous dichloromethane (2 ml) and N,N-dimethylformamide (1 drop) was added oxalyl chloride (0.5 mL), and the resulting solution was stirred at room temperature for 1.5 hours. The solution was evaporated under reduced pressure, and remaining solvent was removed under high vacuum to give the N-benzyloxycarbonyl-D-proline acid chloride. At the same time, a solution of ethyl magnesium bromide (1.4 mL of a 3M solution in ether) was added dropwise over 5 minutes to a stirred solution of 5-bromoindole (0.75 g) in dry ether (18 mL). The mixture was stirred at room temperature for 10 minutes, heated under reflux for 2 hours, then cooled to -30°C. A solution of the above N-benzyloxycarbonyl-D-proline acid chloride in dry ether (4 mL) was added dropwise with stirring, and stirring was continued for a further 1 hour. Ether (12.5 mL) and saturated aqueous sodium bicarbonate (6.5 mL) were added, and the temperature was allowed to rise to room temperature. Stirring was continued for a further 10 minutes and the mixture was filtered. The solid was washed well with ethyl acetate, and the combined filtrate and washings were washed with water, brine and dried (MgSO₄). Evaporation of the solvent gave an oil which was chromatographed on silica gel. Elution with ethyl acetate gave the title compound as a foam (0.82 g): LRMS, m/z (relative intensity) 428 (M+ with ⁸¹Br,5), 426 (M+ with ⁷⁹Br, 5), 224 (19), 222 (21), 204 (62), 160 (68), 91 (100). Anal Calcd for CH₂₁H₁₉BrN₂O₃: C,59.02; H,4.48; N,6.56. Found: C,58.85; H,4.51; N,6.38%.

### EXAMPLE 27

### (R)-5-Bromo-3-(N-methylpyrrolidin-2-ylmethyl)-1H-indole

A solution of (R)-3-(N-benzyloxycarbonyl-pyrrolidin-2-ylcarbonyl)-5-bromo-1H-indole (1.04 g) in dry tetrahydrofuran (20 mL) was added dropwise to a stirred suspension of lithium aluminium hydride (0.27 g) in dry tetrahydrofuran (15 mL) at room temperature under an atmosphere of dry nitrogen. The mixture was heated under reflux with stirring for 18 hours and then cooled. Additional lithium aluminium hydride (50 mg) was added and refluxing was continued for an additional 3 hours. The mixture was again cooled, lithium aluminium hydride (40 mg) was added, and refluxing was continued for a further 18 hours. The mixture was cooled and water (0.44 mL) was carefully added with stirring, followed by 20% aqueous sodium hydroxide (0.44 mL), followed by more water (1.33 mL). The mixture was diluted with ethyl acetate and filtered through Celite (trademark) filter aid. The filtrate was washed with water, brine and then dried (Na₂SO₄). Evaporation of the solvent gave an oil which was chromatographed on silica gel. Elution with dichloromethane/ethanol/concentrated aqueous ammonia (90:10:0.5) gave the title compound as a solid (0.51 g), m.p. 137-140°C (from dichloromethane/hexane); IR (KBr) 1620, 1595, 1570, 1480, 1450, 1435 cm⁻¹; ¹H NMR (DMSO-d₆) δ 11,05 (br s, 1H), 7.65 (br d, 1H), 7.31 (d, J=8.6 Hz, 1H), 7.21 (br d, 1H), 7.16 (dd, J=1.8 and 8.6 Hz, 1H), 3.03-2.94 (m, 2H), 2.47 (dd, J=9.2 and 14.0 Hz, 1H), 2.36-2.26 (m, 1H), 2.33 (s, 3H), 2.09 (dd, J=8.7 and 17.3 Hz, 1H), 1.73-1.38 (m,4H); ¹³C NMR (DMSO-d₆ δ 134.8, 129.5, 124.7, 123.2, 120.7, 113.4, 112.1, 110.9, 66.1, 57.0, 40.5, 30.9, 29.1, 21.6; LRMS, m/z (relative intensity) 294 (M+ with ⁸¹Br, 1), 293 (2), 292 (M+ with ⁷⁹Br, 1), 210 (14), 208 (15), 154 (8), 129 (42), 128 (19), 101 (26), 85 (57), 84 (100), 83 (30); [α]²⁵ = + 62° (methanol, c = 0.10). Anal Calcd for C₁₄H₁₇N₂Br. 0.25 H₂O: C, 56.48; H, 5.93; N, 9.41. Found: C, 56.65; H, 5.69; N,9.23.

### EXAMPLE 28

### (R)-5-(2-Ethylsulphonylethenyl)-3-(N-methyl-pyrrolidin-2-ylmethyl)-1H-indole

A mixture of (R)-5-bromo-3-(N-methylpyrrolidin-2-ylmethyl)-1H-indole (0.25 g), ethyl vinyl sulphone (0.14 g), tri-o-tolylphosphine (0.075 g), palladium (II) acetate (0.013 g), triethylamine (0.25 mL) and acetonitrile (3 mL) was heated under reflux for 17 hours in an atmosphere of nitrogen. The mixture was evaporated and the residue was chromatographed on silica gel. Elution with dichloromethane/ethanol/ concentrated aqueous ammonia (90:8:1) gave the title compound as a foam (0.185 g): TLC (dichloromethane/-ethanol/ concentrated aqueous ammonia, 90:10:1): R_{f} = 0.5. Anal. Calcd for C₁₈H₂₄N₂O₂S. 0.2 CH₂Cl₂: C,62.55; H,7.04; N,8.02. Found: C,62.65; H,6.94; N,7.92.

### EXAMPLE 29

### (R)-5-(2-Ethylsulphonylethyl)-3-(N-methylpyrrolidin-2-ylmethyl)1H-indole

(R)-5-(2-Ethylsulphonylethenyl)-3-(N-methyl-pyrrolidin-2-ylmethyl)-1H-indole (157 mg) was dissolved in a mixture of ethanolic hydrogen chloride [prepared by addition of acetyl chloride (0.043 mL) to ethanol (10 mL)], N,N-dimethylformamide (7.5 mL) and water (0.1 mL) and the solution was shaken under a hydrogen atmosphere (15 psi) at room temperature for 18 hours in the presence of 10% palladium on carbon (150 mg). The mixture was filtered through Arbacel (trade mark) filter aid and the residue was washed well with ethanol. The combined filtrate and washings were evaporated under reduced pressure and the residual oil was partitioned between ethyl acetate and 2M aqueous sodium carbonate solution. The organic layer was separated, washed three times with water followed by brine and then dried (Na₂SO₄). Evaporation of the solvent gave an oil which was chromatographed on silica gel. Elution with dichloromethane/methanol/ concentrated aqueous ammonia (90:10:1) gave the title compound as a gum (110 mg): TLC (CH₂Cl₂/C₂H₅OH/NH₃; 90:10:1): R_{f} = 0.3; [α]²⁵ = +62° (methanol, c = 0.10). Anal. Calcd for C₁₈H₂₆N₂O₂S. 0.05 CH₂Cl₂: C,63.21; H,7.67; N,8.17. Found: C,63.55; H,7.61; N,8.41%.

### EXAMPLE 30

### (R)-5-(2-Ethylsulphonylethyl)-3-(N-methylpyrrolidin-2-ylmethyl)-1H-indole hemisuccinate

A solution of succinic acid (69 mg) in hot ethanol (3.5 mL) was added slowly with stirring to a solution of (R)-5-(2-ethylsulphonylethyl)-3-(N-methylpyrrolidin-2-ylmethyl)-1H-indole free base (390 mg) in ethanol (3.5 mL). The solution was evaporated and the residue was triturated first with ether and then with ethyl acetate to give the title compound as a solid (375 mg): mp 59-62°C: [α]²⁵ = + 36° (methanol, c = 0.10). Anal. Calcd for C₁₈N₂₆N₂O₂S. 0.5 C₄H₆O₄. 0.25 CH₃CO₂C₂H₅. 0.5 H₂O: C,59.00; H,7.42; H,6.68. Found: C,59.17; H,7.37; N,6.73.

### EXAMPLE 31

### (R)-5-(2-Benzenesulphonylethenyl)-3-(N-methyl-pyrrolidin-2-ylmethyl)-1H-indole hydrobromide

A mixture of (R)-5-bromo-3-(N-methylpyrrolidin-2-ylmethyl)-1H-indole (0.25 g), phenylvinylsulphone (0.19 g), tri-o-tolylphosphine (0.075 g), palladium (II) acetate (0.0125 g), triethylamine (0.25 mL) and acetonitrile (2.5 mL) was heated under reflux for 42 hours in an atmosphere of nitrogen. The solvent was evaporated and the residue was chromatographed on silica gel. Elution with dichloromethane/methanol/ concentrated aqueous ammonia (90:10:1) gave the title compound as a foam (0.24 g): Anal. Calcd for C₂₂N₂₄N₂O₂S. HBr. 1/3 CH₂Cl₂: C,54.77; H,5.29; N,5.72. Found: C,55.00; H,4.85; N,5.58.

### EXAMPLE 32

### (R)-5-(2-Benzenesulphonylethyl)-3-(N-methylpyrrolidin-2-ylmethyl)-1H-indole

A solution of (R)-5-(2-benzenesulphonylethenyl)-3-N-methylpyrrolidin-2-ylmethyl)-1N-indole hydrobromide (0.214 g) and 10% palladium on carbon (0.15 g) in a mixture of absolute ethanol (10 mL), N,N-dimethylformamide (1 mL) and water (2 drops) was shaken under a hydrogen atmosphere (15 psi) at room temperature for 18 hours. The mixture was filtered through Celite (trademark) filter aid and the residue was washed well with ethanol. The combined filtrate and washings were evaporated under reduced pressure and the residue was partitioned between ethyl acetate and 2M aqueous sodium carbonate solution. The organic layer was separated, washed three times with water, followed by brine and dried (Na₂SO₄). Evaporation of the solvent gave a gum which was chromatographed on silica gel. Elution with dichloromethane/methanol/concentrated aqueous ammonia (90:10:0.5) gave the title compound as a foam (0.096 g). Anal. Calcd for C₂₂H₂₆N₂O₂S. H₂O: C,65.97; H,7.05; N,7.00. Found: C,65.51; H,6.77; N,7.45.

### EXAMPLE 33

### (R)-5-(2-Benzenesulphonylethyl)-3-(N-methylpyrrolidin-2-ylmethyl)-1H-indole hemisuccinate

A solution of succinic acid (95 mg) in ethanol (5 mL) was added to a solution of (R)-5-(2-benzenesulphonylethyl)-3-(N-methylpyrrolidin-2-ylmethyl)-1H-indole free base (620 mg) in ethanol (5 mL). The solution was evaporated to give the title compound as a foam (680 mg): [α]²⁵ = + 29° (methanol, c = 0.10). Anal. Calcd for C₂₂N₂₆N₂O₂S. 0.5 C₄H₆O₄. 0.33 C₂H₅OH. 0.5 H₂O; C,63.59; H,6.92; N,6.01. Found: C,63.52; H,6.91; N,6.12.

### EXAMPLE 34

### (R)-5-[2-(4-Methylphonylsulphonyl)ethenyl]-3-(N-methylpyrrolidin-2-ylmethyl)-1H-indole

A mixture of (R)-5-bromo-3-(N-methylpyrrolidin-2-ylmethyl)-1H-indole (0.40 g), 4-methylphenylvinyl-sulphone (0.273 g), tri-o-tolylphosphine (0.085 g), palladium (II) acetate (0.031 g), triethylamine (0.42 g), and acetonitrile (20 mL) was heated under reflux for 16 hours in an atmosphere of nitrogen. The mixture was cooled and partitioned between ethyl acetate and 10% aqueous sodium bicarbonate solution. The organic layer was washed with brine, dried (Na₂SO₄) and evaporated. The residual orange oil was chromatographed on silica gel. Elution was commenced with dichloromethane/methanol (90:10), followed by dichloromethane/methanol/concentrated aqueous ammonia (90:10:0.25), gradually increasing the concentration of concentrated aqueous ammonia to 1%. The later product-containing fractions were evaporated to give the title compound as a foam (226 mg): [α]²⁵ = + 71° (methanol, c = 0.10). Anal. Calcd for C₂₃H₁₆N₂O₂S. 0.15 CH₂Cl₂: C,68.27; H,6.51; N,6.88. Found: C,68.26; H,6.54; N,6.99.

### EXAMPLE 35

### (R)-5-[2-(4-Methylphenylsulphonyl)ethyl-3-(N-methylpyrrolidin-2-ylmethyl)-1H-indole

A solution of (R)-5-[2-(4-methylphenyl-sulphonyl)ethenyl]-3-(N-methylpyrrolidin-2-ylmethyl)-1H-indole (0.18 g) and 10% palladium on carbon (0.20 g) in ethanolic hydrogen chloride [prepared from absolute ethanol (25 mL) and acetyl chloride (35 µL)] was shaken under a hydrogen atmosphere (15 psi) at room temperature for 16 hours. The reaction mixture was filtered through Celite (trademark) filter aid and the residue was washed well with ethanol. The combined filtrate and washings were evaporated under reduced pressure and the residue was partitioned between ethyl acetate and 2M aqueous sodium carbonate solution. The organic layer was saturated, washed three times with water, followed by brine and dried (Na₂SO₄). Evaporation of the solvent gave a gum which was chromatographed on silica gel. Elution with dichloromethane/methanol/concentrated aqueous ammonia (90:10:0.25) gave the title compound as a foam (108 mg) : [α]²⁵ = + 30° (methanol, c = 0.10). Anal. Calcd for C₂₃H₂₈N₂O₂S. 0.05 CH₂Cl₂. 0.5 H₂O: C,67.55; H,7.15; N,6.84. Found: C,67.51; H,7.04; N,6.98.

## Claims

1. A compound of the formula 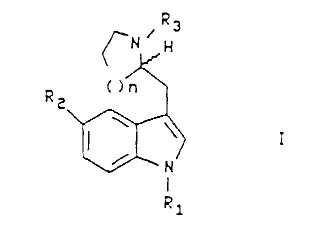 wherein n is 0, 1, or 2; R₁ is hydrogen; R₂ is selected from hydrogen, halogen, cyano, OR₄, -(CH₂)ₘ-(C=O)NR₅R₆, -(CH₂)ₘ-SO₂NR₅R₆, -(CH₂)ₘ-NR₇(C=O)R₈, -(CH₂)ₘ-NR₇SO₂R₈, -(CH₂)ₘ-S(O)ₓR₈, -(CH₂)ₘ-NR₇(C=O)NR₅R₆, -(CH₂)ₘ-NR₇(C=O) OR₉, and -CH=CH(CH₂)_{y}R₁₀; R₃ is selected iron hydrogen and C₁ to C₆ linear or branched alkyl; R₄ is selected from hydrogen, C₁ to C₆ alkyl, and aryl; R₅ and R₆ are independently selected from hydrogen, C₁ to C₆ alkyl, aryl, and C₁ to C₃ alkyl-aryl or R₅ and R₆ taken together form a 4, 5, or 6 membered ring; R₇ and R₈ are independently selected from hydrogen, C₁ to C₆ alkyl, aryl, and C₁ to C₃ alkyl-aryl; R₉ is selected from hydrogen, C₁ to C₆ alkyl, aryl, and C₁ to C₃ alkyl-aryl; R₁₀ is selected from -(C=O)NR₅R₆ and -SO₂NR₅R₆, wherein R₅ and R₆ are defined as above, and -NR₇(C=O)R₈, -NR₇SO₂R₈, -NR₇(C=O)NR₅R₆, -S(O)ₓR₈ and -NR₇(C=O)OR₉, wherein R₇, R₈, and R₉ are as defined above; y is 0, 1, or 2; x is 1 or 2; m is 0, 1, 2, or 3; and the above aryl groups and the aryl moieties of the above alkylaryl groups are independently selected from phenyl and substituted phenyl, wherein said substituted phenyl may be substituted with one to three groups selected from C₁ to C₄ alkyl, halogen, hydroxy, cyano, carboxamido, nitro, and C₁ to C₄ alkoxy, and the phamaceutically acceptable salts thereof, with the provisos that;
(i) when R₁ and R₂ are hydrogen and n is 2, R₃ is not hydrogen or C₁-C₆ linear or branched alkyl when the compound of the formula (I) is a free base;
(ii) when R₁ and R₂ are hydrogen, R₃ is methyl and n is 2, the compound of the formula (I) is not a pharmaceutically acceptable acid addition salt thereof;
(iii) when R₁, R₂ and R₃ are hydrogen and n is 2, the pharmaceutically acceptable salt of the compound of the formula (I) is not the hydrochloride or picrate;
(iv) when R₁ is hydrogen, R₂ is hydroxy and R₃ is methyl, n is not 2 when the compound of the formula (I) is a free base or the pharmaceutically acceptable salt is the picrate or ½ oxalate; and
(v) when R₁ and R₃ are hydrogen and R₂ is hydroxy, n is not 2 when the compound of the formula (I) is a free base or the pharmaceutically acceptable salt is the picrate or hydrogensuccinate.

2. The R enantiomer of a compound according to claim 1.

3. A compound according to claim 1 wherein R₁ is hydrogen; R₂ is -(CH₂)ₘ-SO₂NHR₅, -(CH₂)ₘ-NHSO₂R₈, -(CH₂)ₘ-SO₂R₈, -(CH₂)ₘ-(C=O)NHR₅, or -(CH₂)ₘ-NH(C=O)R₈; R₃ is hydrogen or methyl; and m, R₅ and R₈ are as defined in claim 1.

4. A compound according to claim 1, said compound being selected from:
(R)-5-methoxy-3-(N-methylpyrrolidin-2-ylmethyl)-1H-indole;
(R)-5-bromo-3-(N-methylpyrrolidin-2-ylmethyl)-1H-indole;
(R)-5-(2-ethylsulfonylethyl)-3-(N-methylpyrrolidin-2-ylmethyl)-1H-indole ;
(R)-5-(2-methylaminosulfonylethyl)-3-(N-methylpyrrolidin-2-ylmethyl)-1H-indole ;
(R)-5-(2-methylaminosulfonylethyl)-3-(pyrrolidin-2-ylmethyl)-1H-indole ;
(R)-5-(methylaminosulfonylmethyl)-3-(N-methylpyrrolidin-2-ylmethyl)-1H-indole ;
(R)-5-carboxamido-3-(N-methylpyrrolidin-2-ylmethyl)-1H-indole ;
(R)-5-(2-methylsulfonylethyl)-3-(N-methylpyrrolidin-2-yl-methyl)-1H-indole ;
(R)-5-(2-methylsulfonamidoethyl)-3-(N-methylpyrrolidin-2-ylmethyl)-1H-indole;
(R)-5-(2-aminosulphonylethenyl)-3-(N-methylpyrrolidin-2-ylmethyl)-1H-indole ;
(R)-5-(2-aminosulphonylethyl)-3-(N-methylpyrrolidin-2-ylmethyl-1H-indole;
(R)-5-(2-N,N-dimethylaminosulphonylethyl)-3-(N-methylpyrrolidin-2-ylmethyl)-1H-indole ;
(R)-5-(2-phenylsulphonylethyl)-3-(N-methylpyrrolidin-2-ylmethyl)-1H-indole hemisuccinate;
(R)-5-(2-ethylsulphonylethyl)-3-(N-methylpyrrolidin-2-ylmethyl)-1H-indole hemisuccinate;
(R)-5-(2-phenylsulphonylethyl)-3-(N-methyipyrrolidin-2-ylmethyl)-1H-indole ;
(R)-5-(3-benzenecarbonylaminoprop-1-enyl)-3-(N-methylpyrrolidin-2-ylmethyl)-1H-indole ;
(R)-5-(2-(4-methylphenylsulphonyl)ethyl)-2-(N-methylpyrrolidin-2-ylmethyl)-1H-indole ;
(R)-5-(3-methylsulphonylaminoprop-1-enyl)-3-(N-methylpyrrolidin-2-ylmethyl)-1H-indole ;
(R)-5-(2-ethylsulphonylethyl)-3-(N-2-propylpyrrolidin-2-ylmethyl)-1H-indole;
(R)-5-(2-ethylsulphonylethyl)-3-(pyrrolidin-2-ylmethyl)-1H-indole ;
(R)-5-(2-(4-methylphenylsulphonyl)ethenyl)-3-(N-methylpyrrolidin-2-ylmethyl)-1H-indole; and
(R)-5-(2-methylsulfonamionethyl)-3-(N-methylpyrrolidin-2-ylmethyl)-1H-indole.

5. A pharmaceutical composition for treating a condition selected from hypertension, depression, anxiety, eating disorders, obesity, drug abuse, cluster headache, migraine, pain, and chronic paroxysmal hemicrania and headache associated with vascular disorders comprising an amount of a compound according to any one of claims 1 to 4 effective in treating such a condition and a pharmaceutically acceptable carrier.

6. A pharmaceutical composition for treating disorders arising from deficient serotonergic neurotransmission comprising an amount of a compound according to any one of claims 1 to 4 effective in treating such a disorder and a pharmaceutically acceptable carrier.

7. A compound of the formula (I), or a pharmaceutically acceptable salt or composition thereof, as claimed in any one of claims 1 to 4 and 5 and 6, respectively, for use as a medicament.

8. The use of a compound of the formula (I), or of a pharmaceutically acceptable salt or composition thereof, as claimed in any one of claims 1 to 4 and 5 and 6, respectively, for the manufacture of a medicament for treating a condition selected from hypertension, depression, anxiety, eating disorders, obesity, drug abuse, cluster headache, migraine, pain and chronic paroxysmal hemicrania and headache associated with vascular disorders.

9. The use of a compound of the formula (I), or of a pharmaceutically acceptable salt or composition thereof, as claimed in any one of claims 1 to 4 and 5 and 6, respectively, for the manufacture of a medicament for treating disorders arising from deficient serotonergic neurotransmission.

10. A compound of the formula 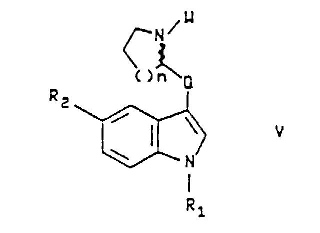 wherein W is -CO₂R₁₁ or R₃; Q is CH₂ or C=O; n is 0, 1 or 2; R₁ is hydrogen; R₂ is selected from hydrogen, halogen, cyano, OR₄, -(CH₂)ₘ-(C=O)NR₅R₆, -(CH₂)ₘ-SO₂NR₅R₆, -(CH₂)ₘ-NR₇(C=O)R₈, -(CH₂)ₘ-NR₇SO₂R₈, -(CH₂)ₘ-S(O)ₓR₈, -(CH₂)ₘ-NR₇(C=O)NR₅R₆, -(CH₂)ₘ-NR₇(C=O)OR₉, and -CH=CH(CH₂) _{y}R₁₀; x is 1 or 2; m is 0, 1, 2, or 3; R₃ is selected from hydrogen and C₁ to C₆ linear or branched alkyl; R₄ is selected from hydrogen, C₁ to C₆ alkyl, and aryl, R₅ and R₆ are independently selected from hydrogen, C₁ to C₆ alkyl, aryl, and C₁ to C₃ alkyl-aryl or R₅ and R₆ taken together form a 4, 5, or 6 membered ring; R₇ and R₈, are independently selected from hydrogen, C₁ to C₆ alkyl, aryl, and C₁ to C₃ alkyl-aryl; R₉ is selected from hydrogen, C₁ to C₆ alkyl, aryl, and C₁ to C₃ alkyl-aryl; R₁₀ is selected from -(C=O)NR₅R₆ and -SO₂NR₆R₅, wherein R₅ and R₆ are defined as above, and -NR₇(C=O)R₈, -NR₇SO₂R₈, -NR₇(C=O)NR₅R₆, -S(O)ₓR₈ and -NR₇(C=O)OR₉, wherein R₇, R₈, R₉ and x are defined as above; y is 0, 1, or 2; R₁₁ is selected from C₁ to C₆ alkyl, benzyl and aryl; and the above aryl groups and the aryl moieties of the above alkyl-aryl groups are independently selected from phenyl and substituted phenyl, wherein said substituted phenyl may be substituted with one to three groups selected from C₁ to C₄ alkyl, halogen, hydroxy, cyano, carboxamido, nitro, and C₁ to C₄ alkoxy, with the provisos that:
(i) when Q is CH₂, W is not R₃;
(ii) when R₁ and R₂ are hydrogen, Q is C=O and n is 2, W is not hydrogen or neo-pentyl; and
(iii) when R₁ and R₂ are hydrogen, Q is C=O and n is 1, W is not hydrogen or benzyloxycarbonyl.

11. The R enantiomer of a compound according to claim 10.

12. A compound according to claim 10, said compound being a compound of the fomula 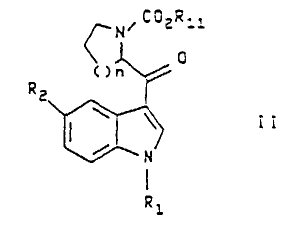 wherein n, R₁, R₂ and R₁₁ are as defined in claim 10.

13. The R enantiomer of a compound according to claim 12.

14. A compound according to claim 12 wherein R₁ is hydrogen ; R₂ is -(CH₂)ₘ-SO₂NHR₅, -(CH₂)ₘ-NHSO₂R₈, -(CH₂)ₘ-SO₂R₈, -(CH₂)ₘ-(C=O)NHR₅ or -(CH₂)ₘ-NH(C=O)R₈; m is 0, 1, 2, or 3; R₅ is hydrogen, C₁ to C₆ alkyl, aryl, or C₁ to C₃ alkyl-aryl; R₁₁ is selected from C₁ to C₅ alkyl, benzyl and aryl; and the above aryl groups and the aryl moieties of the above alkylaryl groups are independently selected from phenyl and substituted phenyl, wherein said substituted phenyl may be substitted with one to three groups selected from C₁ to C₄ alkyl, halogen, hydroxy, cyano, carboxamido, nitro, and C₁ to C₄ alkoxy.

15. A compound according to claim 1, said compound being a compound of the formula 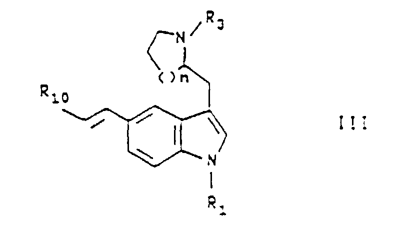 wherein n, R₁, R₃ and R₁₀ are as defined in claim 1.

16. The R enantiomer of a compound according to claim 15.

17. A compound according to claim 15 wherein R₁ is hydrogen; R₃ is hydrogen or methyl; and R₁₀ is -SO₂NHR₅, NHSO₂R₈, -SO₂R₈, -(C=O)NHR₅ or -NH(C=O)R₈, wherein R₅ and R₈ are as defined in claim 1.

18. A process for preparing a compound of the formula 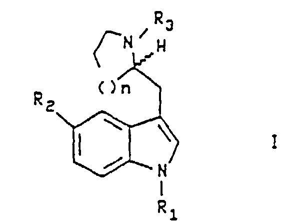 wherein n is O, 1, or 2; R₁ is hydrogen; R₂ is selected from hydrogen, halogen, cyano, OR₄, -(CH₂)ₘ-(C=O)NR₅R₆, -(CH₂)ₘ-SO₂NR₅R₆, -(CH₂)ₘ-NR₇(C=O)R₈, -(CH₂)ₘ-NR₇SO₂R₈, -(CH₂)ₘ-S(O)ₓR₈, -(CH₂)ₘ-NR₇(C=O)NR₅R₆, -(CH₂)ₘ-NR₇(C=O)OR₉, and -CH=CH(CH₂)_{y}R₁₀; R₃ is selected from hydrogen and C₁ to C₆ linear or branched alkyl; R₄ is selected from hydrogen, C₁ to C₆ alkyl, and aryl; R₅ and R₆ are independently selected from hydrogen, C₁ to C₆ alkyl, aryl, and C₁ to C₃ alkyl-aryl or R₅ and R₆ taken together form a 4, 5, or 6 membered ring; R₇ and R₈ are independently selected from hydrogen, C₁ to C₆ alkyl, aryl, and C₁ to C₃ alkyl-aryl; R₉ is selected from hydrogen, C₁ to C₆ alkyl, aryl, and C₁ to C₃ alkyl-aryl; R₁₀ is selected from -(C=O)NR₅R₆ and -SO₂NR₅R₆, wherein R₅ and R₆ are defined as above, and -NR₇(C=O)R₈, -NR₇SO₂R₈, -NR₇(C=O)NR₅R₆, -S(O)ₓR₈ and -NR₇(C=O)OR₉, wherein R₇, R₈, and R₉ are as defined above; y is 0, 1, or 2; x is 1 or 2; n is 0, 1, 2, or 3; and the above aryl groups and the aryl moieties of the above alkylaryl groups are independently selected from phenyl and substituted phenyl, wherein said substituted phenyl may be substituted with one to three groups selected from C₁ to C₄ alkyl, halogen, hydroxy, cyano, carboxamido, nitro, and C₁ to C₄ alkoxy, with the provisos that:
(i) when R₁ and R₂ are hydrogen and n is 2, R₃ is not hydrogen or C₁-C₆ linear or branched alkyl when the compound of the formula (I) is a free base;
(ii) when R₁ and R₂ are hydrogen, R₃ is methyl and n is 2, the compound of the formula (I) is not a pharmaceutically acceptable acid addition salt thereof;
(iii) when R₁, R₂ and R₃ are hydrogen and n is 2, the pharmaceutically acceptable salt of the compound of the formula (I) is not the hydrochloride or picrate;
(iv) when R₁ is hydrogen, R₂ is hydroxy and R₃ is methyl, n is not 2 when the compound of the formula (I) is a free base or the pharmaceutically acceptable salt is the picrate or ½ oxalate; and
(v) when R₁ and R₃ are hydrogen and R₂ is hydroxy, n is not 2 when the compound of the formula (I) is a free base or the pharmaceutically acceptable salt is the picrate or hydrogensuccinate,
comprising
(a) reducing a compound of the formula 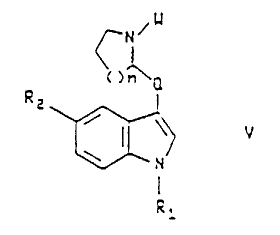 where Q is CH₂ or C=O, W is -CO₂R₁₁ or R₃, and R₁₁ is C₁ to C₆ alkyl, benzyl, or aryl, and R₁, R₂, and R₁₁ are as defined above;
(b) where R₂ is -CH=CH(CH₂)_{y}R₁₀ and R₁₀ is as defined above by reacting a compound of formula I where R₃ is H or C₁ to C₆ linear or branched alkyl and R₂ is halogen and R₁ is defined as above with a compound of the formula CH₂=CH(CH₂)_{y}R₁₀ where R₁₀ is as defined above using transition metal catalysis; or
(c) reacting the compound of formula I where R₃ is hydrogen and R₁ and R₂ are as defined above with a compound of the formula R₃-Z where R₃ is C₁ to C₆ linear or branched alkyl and Z is halogen and base; and,
if desired, converting a compound of formula I to a pharmaceutically acceptable salt thereof.

19. The process according to claim 18 wherein the compound of formula I is an R enantiomer.

20. A process according to claim 18 wherein for the compound of formula I, R₁ is hydrogen; R₂ is -(CH₂)ₘ-SO₂NHR₅, -(CH₂)ₘ-NHSO₂R₈, -(CH₂)ₘ-SO₂R₈, -(CH₂)ₘ-(C=O)NHR₅, or -(CH₂)ₘ-NH(C=O)R₈; R₃ is hydrogen or methyl ; and m, R₅ and R₈ are as defined in claim 18.

21. A process according to claim 18, said compound of formula I being selected from:
(a)-5-methoxy-3-(N-methylpyrrolidin-2-ylmethyl)-1H-indole;
(R)-5-bromo-3-(N-methylpyrrolidin-2-ylmethyl)-1H-indole;
(R)-5-(2-ethylsulfonylethyl)-3-(N-methylpyrrolidin-2-ylmethyl)-1H-indole;
(R)-5-(2-methylaminosulfonylethyl)-2-(N-methylpyrrolidin-2-ylmethyl)-1H-indole ;
(R)-5-(2-methylaminosulfonylethyl)-2-(pyrrolidin-2-ylmethyl)-1H-indole;
(R)-5-(methylaminosulfonylmethyl)-3-(N-methylpyrrolidin-2-ylmethyl)-1H-indole;
(R)-5-carboxamido-3-(-methylpyrrolidin-2-ylmethyl)-1H-indole;
(R)-5-(2-methylsulfonylethyl)-3-(N-methylpyrrolidin-2-yl-methyl)-1H-indole;
(R)-5-(2-methylsulfonamidoethyl)-3-(N-methylpyrrolidin-2-ylmethyl)-1H-indole;
(R)-5-(2-aminosulphonylethenyl)-2-(N-methylpyrrolidin-2-ylmethyl)-1H-indole;
(R)-5-(2-aminosulphonylethyl)-3-(N-methylpyrrolidin-2-ylmethyl)-1H-indole;
(R)-5-(2-N,N-dimethylaminosulphonylethyl)-3-(N-methylpyrrolidin-2-ylmethyl)-1H-indole;
(R)-5-(2-phenylsulphonylethyl)-3-(N-methylpyrrolidin-2-ylmethyl)-1H-indole hemisuccinate;
(R)-5-(2-ethylsulphonylethyl)-3-(N-methylpyrrolidin-2-ylmethyl)-1H-indole hemisuccinate;
(R)-5-(2-phenylsulphonylethyl)-3-(N-methylpyrrolidin-2-ylmethyl)-1H-indole;
(R)-5-(3-benzenecarbonylaminoprop-1-enyl)-3-(N-methylpyrrolidin-2-ylmethyl)-1H-indole ;
(R)-5(2-(4-methylphenylsulphonyl)ethyl)-3-(N-methylpyrrolidin-2-ylmethyl)-1H-indole;
(R)-5-(3-methylsulphonylaminoprop-1-enyl)-3-(N-methylpyrrolidin-2-ylmethyl)-1H-indole;
(R)-5-(2-ethylsulphonylethyl)-3-(N-2-propylpyrrolidin-2-ylmethyl)-1H-indole ;
(R)-5-(2-ethylsulphonylethyl)-3-(pyrrolidin-2-ylmethyl)-1H-indole;
(R)-5-(2-(4-methylphenylsulphonyl)ethenyl)-3-(N-methylpyrrolidin-2-ylmethyl)-1H-indole; and
(R)-5-(2-methylsulfonanidomethyl)-3-(N-methylpyrrolidin-2-ylmethyl-1H-indole.

22. A process for preparing a compound of the formula 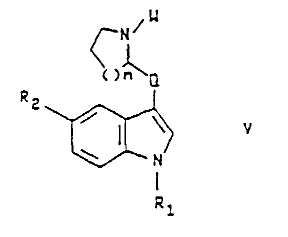 wherein W is -CO₂R₁₁; Q is C=0; n is 1; R₁ is hydrogen; R₂ is selected from hydrogen, halogen, cyano, OR₄, -(CH₂)ₘ-(C=O)NR₅R₆, -(CH₂)ₘ-SO₂NR₅R₆, -(CH₂)ₘ-NR₇(C=O)R₈, -(CH₂)ₘ-NR₇SO₂R₈, -(CH₂)ₘ-S(O)ₓR₈, -(CH₂)ₘ-NR₇(C=O)NR₅R₆, -(CH₂)ₘ-NR₇(C=O)OR₉, and -CH=CH(CH₂)_{y}R₁₀; x is 1 or 2; m is 0, 1, 2, or 3; R₄ is selected from hydrogen, C₁ to C₆ alkyl, and aryl, R₅ and R₆ are independently selected from hydrogen, C₁ to C₅ alkyl aryl, and C₁ to C₃ alkyl-aryl or R₅ and R₆ taken together form a 4, 5, or 6 membered ring; R₇ and R₈ are independently selected from hydrogen, C₁ to C₆ alkyl, aryl, and C₁ to C₃ alkyl-aryl; R₉ is selected from hydrogen, C₁ to C₆ alkyl, aryl, and C₁ to C₃ alkyl-aryl; R₁₀ is selected from -(C=O)NR₅R₆ and -SO₂NR₅R₆, wherein R₅ and R₆ are defined as above, and -NR₇(C=O)R₈, -NR₇SO₂R₈, -NR₇(C=O)NR₅R₆, -S(O)ₓR₈ and -NR₇(C=O)OR₉, wherein R₇, R₈, R₉ and x are defined as above; y is 0, 1 or 2; R₁₁ is selected from C₁ to C₆ alkyl, benzyl and aryl; and the above aryl groups and the aryl moieties of the above alkyl-aryl groups are independently selected from phenyl and substituted phenyl, wherein said substituted phenyl may be substituted with one to three groups selected from C₁ to C₄ alkyl, halogen, hydroxy, cyano, carboxamido, nitro, and C₁ to C₄ alkoxy, with the proviso that when R₁ and R₂ are hydrogen, R₁₁ is not benzyl, comprising reacting a compound of the formula 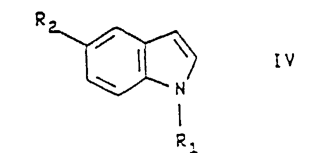 wherein R₁ and R₂ are as defined above with an acid chloride of a compound of the formula N-CO₂R₁₁-proline with base.

23. The process according to claim 22 wherein the compound of formula V is an R enantiomer.

24. A process according to claim 22 or 23 wherein for the compound of formula V R₁ is hydrogen; R₂ is -(CH₂)ₘ-SO₂NHR₅, -(CH₂)ₘ-NHSO₂R₈, -(CH₂)ₘ-SO₂R₈, -(CH₂)ₘ-(C=O)NHR₅ or -(CH₂)ₘ-NH(C=O)R₈; m is 0, 1, 2 or 3; R₅ is hydrogen, C₁ to C₆ alkyl, aryl or C₁ to C₃ alkyl-aryl; R₁₁ is selected from C₁ to C₆ alkyl, benzyl and aryl; and the above aryl groups and the aryl moieties of the above alkylaryl groups are independently selected from phenyl and substituted phenyl, wherein said substituted phenyl may be substituted with one to three groups selected from C₁ to C₄ alkyl, halogen, hydroxy, cyano, carboxamido, nitro, and C₁ to C₄ alkoxy.

25. A process for the preparation of a compound of the formula: 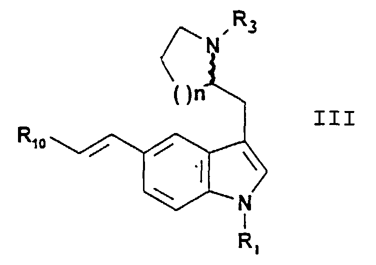 wherein R₁ and R₁₀ are as defined in claim 22, n is 0,1 or 2 and R₃ is selected from hydrogen and C₁ to C₆ linear or branched alkyl, comprising reacting a compound of the formula 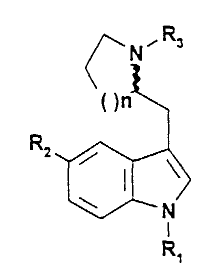 wherein R₂ is halogen and R₁, R₃ and n are as defined above, with a compound of the formula:
CH₂=CHR₁₀
where R₁₀ is as defined above, using transition metal catalysis.

26. The process according to claim 25 wherein the compound of formula III is an R enantiomer.

27. A process according to claim 25 or 26 wherein for the compound of formula III R₁ is hydrogen; R₃ is hydrogen or methyl; and R₁₀ is -SO₂NHR₅, NHSO₂R₈, -SO₂R₈, -(C=0)NHR₅ or -NH(C=0)R₈, wherein R₅ and R₈ are as defined in claim 22.

28. A compound according to claim 1 which is (R)-5-(methylaminosulfonylmethyl)-3-(N-methylpyrrolidin-2-ylmethyl)-1H-indole.

29. A compound according to claim 1 which is (R)-5-(2-phenylsulphonylethyl)-3-(N-methylpyrrolidin-2-ylmethyl)-1H-indole.

30. A compound according to claim which is (R)-5-(2-phenylsulphonylethyl)-3-(N-methylpyrrolidin-2-ylmethyl)-1H-indole hemisuccinate.

31. A compound according to claim which is (R)-5-(methylaminosulfonylmethyl)-3-(pyrrolidin-2-ylmethyl)-1H-indole.

32. A composition according to claim 5 or 6 where the compound of the formula (I) is as claimed in any one of claims 28 to 31.

33. A compound for use as a medicament as claimed in claim 7 wherein the compound of the formula (I) is as claimed in any one of claims 28 to 31.

34. Use as claimed in claim 8 or 9 wherein the compound of the formula (I) is as claimed in any one of claims 28 to 31.

35. A process according to claim 18, said compound of formula (I) being (R)-5-(methylaminosulfonylmethyl)-3-(N-methylpyrrolidin-2-ylmethyl)-1H-indole.

36. A process according to claim 18, said compound of formula (I) being (R)-5-(2-phenylsulphonylethyl)-3-(N-methylpyrrolidin-2-ylmethyl)-1H-indole.

37. A process according to claim 18, said compound of formula (I) being (R)-5-(2-phenylsulphonylethyl)-3-(N-methylpyrrolidin-2-ylmethyl)-1H-indole hemisuccinate.

38. A process according to claim 18, said compound of the formula (I) being (R)-5-(methylaminosulfonylmethyl)-3-pyrrolidin-2-ylmethyl)-1H-indole.

## Patentansprüche

1. Verbindung der Formel 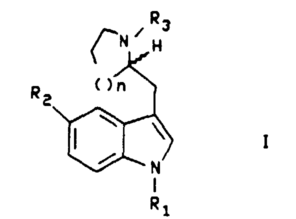 wobei n 0, 1 oder 2 ist; R₁ Wasserstoff ist; R₂ ausgewählt wird aus Wasserstoff, Halogen, Cyano, OR₄, -(CH₂)ₘ-(C=O)NR₅R₆, (CH₂)ₘ-SO₂NR₅R₆, -(CH₂)ₘ-NR₇(C=O)R₈, -(CH₂)ₘ-NR₇SO₂R₈, -(CH₂)ₘ-S(O)ₓR₈, -(CH₂)ₘ-NR₇(C=O)NR₅R₆, -(CH₂)ₘ-NR₇(C=O)OR₉ und -CH=CH(CH₂)_{y}R₁₀; R₃ ausgewählt wird aus Wasserstoff und C₁ bis C₆ linearem oder verzweigtem Alkyl; R₄ ausgewählt wird aus Wasserstoff, C₁ bis C₆ Alkyl und Aryl; R₅ und R₆ unabhängig voneinander ausgewählt werden aus Wasserstoff, C₁ bis C₆ Alkyl, Aryl und C₁ bis C₃ Alkyl-Aryl oder R₅ und R₆ zusammengenommen einen 4-, 5- oder 6-gliedrigen Ring bilden; R₇ und R₈ unabhängig voneinander ausgewählt werden aus Wasserstoff, C₁ bis C₆ Alkyl, Aryl und C₁ bis C₃ Alkyl-Aryl; R₉ ausgewählt wird aus Wasserstoff, C₁ bis C₆ Alkyl, Aryl und C₁ bis C₃ Alkyl-Aryl; R₁₀ ausgwählt wird aus -(C=O)NR₅R₆ und -SO₂NR₅R₆, wobei R₅ und R₆ wie vorstehend definiert sind, und -NR₇(C=O)R₈, NR₇SO₂R₈, -NR₇(C=O)NR₅R₆, -S(O)ₓR₈ und -NR₇(C=O)OR₉, wobei R₇, R₈ und R₉ wie vorstehend definiert sind; y 0, 1 oder 2 ist; x 1 oder 2 ist; m 0, 1, 2 oder 3 ist; und die vorstehenden Aryl-Gruppen und die Arylanteile der vorstehenden Alkyl-Aryl-Gruppen unabhängig voneinander ausgewählt werden aus Phenyl und substituiertem Phenyl, wobei das substituierte Phenyl mit einer bis drei Gruppen ausgewählt aus C₁ bis C₄ Alkyl, Halogen, Hydroxy, Cyano, Carboxamido, Nitro und C₁ bis C₄ Alkoxy substituiert sein kann, und die pharmazeutisch verträglichen Salze davon, mit den Maßgaben, daß:
(i) R₃ nicht Wasserstoff oder C₁-C₆ lineares oder verzweigtes Alkyl ist, wenn R₁ und R₂ Wasserstoff sind, n 2 ist und die Verbindung der Formel (I) eine freie Base ist;
(ii) die Verbindung der Formel (I) kein pharmazeutisch verträgliches Säureadditionssalz ist, wenn R₁ und R₂ Wasserstoff sind, R₃ Methyl und n 2 ist;
(iii) das pharmazeutisch verträgliche Salz der Verbindung der Formel (I) nicht das Hydrochlorid oder Pikrat ist, wenn R₁, R₂ und R₃ Wasserstoff sind und n 2 ist,;
(iv) n nicht 2 ist, wenn R₁ Wasserstoff, R₂ Hydroxy und R₃ Methyl ist und die Verbindung der Formel (I) eine freie Base oder das pharmazeutisch verträgliche Salz das Pikrat oder ½ Oxalat ist; und
(v) n nicht 2 ist, wenn R₁ und R₃ Wasserstoff sind und R₂ Hydroxy ist und die Verbindung der Formel (I) eine freie Base oder das pharmazeutisch verträgliche Salz das Pikrat oder Wasserstoffsuccinat ist.

2. R-Enantiomer einer Verbindung nach Anspruch 1.

3. Verbindung nach Anspruch 1, wobei R₁ Wasserstoff ist; R₂ -(CH₂)ₘ-SO₂NHR₅, -(CH₂)ₘ-NHSO₂R₈, -(CH₂)ₘ-SO₂R₈, -(CH₂)ₘ-(C=O)NHR₅ oder -(CH₂)ₘ-NH(C=O)R₈ ist; R₃ Wasserstoff oder Methyl ist; und m, R₅ und R₈ wie in Anspruch 1 definiert sind.

4. Verbindung nach Anspruch 1, wobei die Verbindung ausgewählt wird aus:
(R)-5-Methoxy-3-(N-methylpyrrolidin-2-ylmethyl)-1H-indol;
(R)-5-Brom-3-(N-methylpyrrolidin-2-ylmethyl)-1H-indol;
(R)-5-(2-Ethylsulfonylethyl)-3-(N-methylpyrrolidin-2-ylmethyl)-1H-indol;
(R)-5-(2-Methylaminosulfonylethyl)-3-(N-methylpyrrolidin-2-ylmethyl)-1H-indol;
(R)-5-(2-Methylaminosulfonylethyl)-3-(pyrrolidin-2-ylmethyl)-1H-indol;
(R)-5-(Methylaminosulfonylmethyl)-3-(N-methylpyrrolidin-2-ylmethyl)-1H-indol;
(R)-5-Carboxamido-3-(N-methylpyrrolidin-2-ylmethyl)-1H-indol;
(R)-5-(2-Methylsulfonylethyl)-3-(N-methylpyrrolidin-2-ylmethyl)-1H-indol;
(R)-5-(2-Methylsulfonamidoethyl)-3-(N-methylpyrrolidin-2-ylmethyl)-1H-indol;
(R)-5-(2-Aminosulfonylethenyl)-3-(N-methylpyrrolidin-2-ylmethyl)-1H-indol;
(R)-5-(2-Aminosulfonylethyl)-3-(N-methylpyrrolidin-2-ylmethyl)-1H-indol;
(R)-5-(2-N,N-Dimethylaminosulfonylethyl)-3-(N-methylpyrrolidin-2-ylmethyl)-1H-indol;
(R)-5-(2-Phenylsulfonylethyl)-3-(N-methylpyrrolidin-2-ylmethyl)-1H-indolhemisuccinat;
(R)-5-(2-Ethylsufonylethyl)-3-(N-methylpyrrolidin-2-ylmethyl)-1H-indolhemisuccinat;
(R)-5-(2-Phenylsulfonylethyl)-3-(N-methylpyrrolidin-2-ylmethyl)-1H-indol;
(R)-5-(3-Benzolcarbonylaminoprop-1-enyl)-3-(N-methylpyrrolidin-2-ylmethyl)-1H-indol;
(R)-5-(2-(4-Methylphenylsulfonyl)ethyl)-3-(N-methylpyrrolidin-2-ylmethyl)-1H-indol;
(R)-5-(3-Methylsulfonylaminoprop-1-enyl)-3-(N-methylpyrrolidin-2-ylmethyl)-1H-indol;
(R)-5-(2-Ethylsulfonylethyl)-3-(N-2-propylpyrrolidin-2-ylmethyl)-1H-indol;
(R)-5-(2-Ethylsulfonylethyl)-3-(pyrrolidin-2-ylmethyl)-1H-indol;
(R)-5-(2-(4-Methylphenylsulfonyl)ethenyl)-3-(N-methylpyrrolidin-2-ylmethyl)-1H-indol; und
(R)-5-(2-Methylsulfonamidomethyl)-3-(N-methylpyrrolidin-2-ylmethyl)-1H-indol.

5. Arzneimittel zur Behandlung eines Zustands ausgewählt aus Bluthochdruck, Depression, Angstzu-ständen, Eßstörungen, Fettleibigkeit, Arzneimittel- und Drogenmißbrauch, Horton's Syndrom, Migräne, Schmerzzuständen und chronischer, anfallartiger Hemikranie und Kopfschmerzen in Zusammenhang mit vasculären Störungen, umfassend eine Menge einer Verbindung nach einem der Ansprüche 1 bis 4, die zur Behandlung eines solchen Zustands wirksam ist, und einen pharmazeutisch verträglichen Träger.

6. Arzneimittel zur Behandlung von Störungen, die durch eine fehlerhafte, serotonergische Neurotransmission hervorgerufen werden, umfassend eine Menge einer Verbindung nach einem der Ansprüche 1 bis 4, die zur Behandlung einer derartigen Störung wirksam ist, und einen pharmazeutisch verträglichen Träger.

7. Verbindung der Formel (I) oder ein pharmazeutisch verträgliches Salz oder ein Mittel davon nach einem der Ansprüche 1 bis 4 bzw. 5 und 6, zur Verwendung als Medikament.

8. Verwendung einer Verbindung der Formel (I) oder eines pharmazeutisch verträglichen Salzes oder eines Mittels davon nach einem der Ansprüche 1 bis 4 bzw. 5 und 6, zur Herstellung eines Medikaments zur Behandlung eines Zustands ausgewählt aus Bluthochdruck, Depression, Angstzuständen, Eßstörungen, Fettleibigkeit, Arzneimittel- und Drogenmißbrauch, Horton's Syndrom, Migräne, Schmerzzuständen und chronischer, anfallartiger Hemikranie und Kopfschmerzen im Zusammenhang mit vasculären Störungen.

9. Verwendung einer Verbindung der Formel (I) oder eines pharmazeutisch verträglichen Salzes oder eines Mittles davon nach einem der Ansprüche 1 bis 4 bzw. 5 und 6 zur Herstellung eines Medikaments zur Behandlung von Störungen, die durch eine fehlerhafte, serotonergische Neurotransmission hervorgerufen werden.

10. Verbindung der Formel 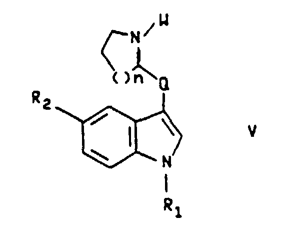 wobei W -CO₂R₁₁ oder R₃ ist; Q CH₂ oder C=O ist; n 0, 1 oder 2 ist; R₁ Wasserstoff ist; R₂ ausgewählt wird aus Wasserstoff, Halogen, Cyano, OR₄, -(CH₂)ₘ-(C=O)NR₅R₆, -(CH₂)ₘ-SO₂NR₅R₆, (CH₂)ₘ-NR₇(C=O)R₈, -(CH₂)ₘ-NR₇SO₂R₈, -(CH₂)ₘ-S(O)ₓR₈, -(CH₂)ₘ-NR₇(C=O)NR₅R₆, -(CH₂)ₘ-NR₇(C=O)OR₉ und -CH=CH(CH₂)_{y}R₁₀; x 1 oder 2 ist; m 0, 1, 2 oder 3 ist; R₃ ausgewählt wird aus Wasserstoff und C₁ bis C₆ linearem oder verzweigtem Alkyl; R₄ ausgewählt wird aus Wasserstoff, C₁ bis C₆ Alkyl und Aryl; R₅ und R₆ unabhängig voneinander ausgewählt werden aus Wasserstoff, C₁ bis C₆ Alkyl, Aryl und C₁ bis C₃ Alkyl-Aryl oder R₅ und R₆ zusammengenommen einen 4-, 5- oder 6-gliedrigen Ring bilden; R₇ und R₈ unabhängig voneinander ausgewählt werden aus Wasserstoff, C₁ bis C₆ Alkyl, Aryl und C₁ bis C₃ Alkyl-Aryl; R₉ ausgewählt wird aus Wasserstoff, C₁ bis C₆ Alkyl, Aryl und C₁ bis C₃ Alkyl-Aryl; R₁₀ ausgwählt wird aus -(C=O)NR₅R₆ und -SO₂NR₅R₆, wobei R₅ und R₆ wie vorstehend definiert sind, und -NR₇(C=O)R₈, NR₇SO₂R₈, -NR₇(C=O)NR₅R₆, -S(O)ₓR₈ und -NR₇(C=O)OR₉, wobei R₇, R_{8,} R₉ und x wie vorstehend definiert sind; y 0, 1 oder 2 ist; R₁₁ ausgewählt wird aus C₁ bis C₆ Alkyl, Benzyl und Aryl; und die vorstehenden Aryl-Gruppen und die Arylanteile der vorstehenden Alkyl-Aryl-Gruppen unabhängig voneinander ausgewählt werden aus Phenyl und substituiertem Phenyl, wobei das substituierte Phenyl mit einer bis drei Gruppen ausgewählt aus C₁ bis C₄ Alkyl, Halogen, Hydroxy, Cyano, Carboxamido, Nitro und C₁ bis C₄ Alkoxy substituiert sein kann, mit den Maßgaben, daß:
(i) W nicht R₃ ist, wenn Q CH₂ ist;
(ii) W nicht Wasserstoff oder Neopentyl ist, wenn R₁ und R₂ Wasserstoff sind, Q C=O ist und n 2 ist; und
(iii) W nicht Wasserstoff oder Benzyloxycarbonyl ist, wenn R₁ und R₂ Wasserstoff sind, Q C=O ist und n 1 ist.

11. R-Enantiomer einer Verbindung nach Anspruch 10.

12. Verbindung nach Anspruch 10, wobei die Verbindung eine Verbindung der Formel 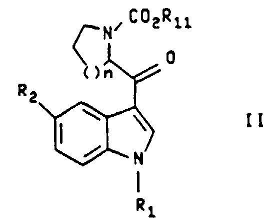 ist, wobei n, R₁, R₂ und R₁₁ wie in Anspruch 10 definiert sind.

13. R-Enantiomer einer Verbindung nach Anspruch 12.

14. Verbindung nach Anspruch 12, wobei R₁ Wasserstoff ist; R₂ -(CH₂)ₘ-SO₂NHR₅, -(CH₂)ₘ-NHSO₂R₈, -(CH₂)ₘ-SO₂R₈, -(CH₂)ₘ-(C=O)NHR₅ oder -(CH₂)ₘ-NH(C=O)R₈ ist; m 0, 1, 2 oder 3 ist; R₅ Wasserstoff, C₁ bis C₆ Alkyl, Aryl oder C₁ bis C₃ Alkyl-Aryl ist; R₁₁ ausgewählt wird aus C₁ bis C₆ Alkyl, Benzyl und Aryl; und die vorstehenden Arylgruppen und die Arylanteile der vorstehenden Alkyl-Aryl-Gruppen unabhängig voneinander ausgewählt werden aus Phenyl und substituiertem Phenyl, wobei das substituierte Phenyl mit einer bis drei Gruppen ausgewählt aus C₁ bis C₄ Alkyl, Halogen, Hydroxy, Cyano, Carboxamido, Nitro und C₁ bis C₄ Alkoxy substituiert sein kann.

15. Verbindung nach Anspruch 1, wobei die Verbindung eine Verbindung der Formel 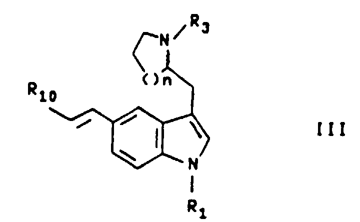 ist, wobei n, R₁, R₃ und R₁₀ wie in Anspruch 1 definiert sind.

16. R-Enantiomer einer Verbindung nach Anspruch 15.

17. Verbindung nach Anspruch 15, wobei R₁ Wasserstoff ist; R₃ Wasserstoff oder Methyl ist; und R₁₀ -SO₂NHR₅, NHSO₂R₈, -SO₂R₈, -(C=O)NHR₅ oder -NH(C=O)R₈ ist, wobei R₅ und R₈ wie in Anspruch 1 definiert sind.

18. Verfahren zur Herstellung einer Verbindung der Formel 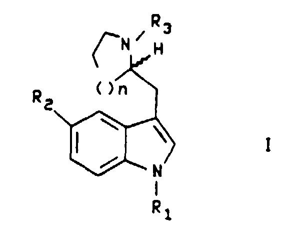 wobei n 0, 1 oder 2 ist; R₁ Wasserstoff ist; R₂ ausgewählt wird aus Wasserstoff, Halogen, Cyano, OR_{4,} -(CH₂)ₘ-(C=O)NR₅R₆, -(CH₂)ₘ-SO₂NR₅R₆, -(CH₂)ₘ-NR₇(C=O)R₈, (CH₂)ₘ-NR₇SO₂R₈, -(CH₂)ₘ-S(O)ₓR₈, -(CH₂)ₘ-NR₇(C=O)NR₅R₆, -(CH₂)ₘ-NR₇(C=O)OR₉ und -CH=CH(CH₂)_{y}R₁₀; R₃ ausgewählt wird aus Wasserstoff und C₁ bis C₆ linearem oder verzweigtem Alkyl; R₄ ausgewählt wird aus Wasserstoff, C₁ bis C₆ Alkyl und Aryl; R₅ und R₆ unabhängig voneinander ausgewählt werden aus Wasserstoff, C₁ bis C₆ Alkyl, Aryl und C₁ bis C₃ Alkyl-Aryl oder R₅ und R₆ zusammengenommen einen 4-, 5- oder 6-gliedrigen Ring bilden; R₇ und R₈ unabhängig voneinander ausgewählt werden aus Wasserstoff, C₁ bis C₆ Alkyl, Aryl und C₁ bis C₃ Alkyl-Aryl; R₉ ausgewählt wird aus Wasserstoff, C₁ bis C₆ Alkyl, Aryl und C₁ bis C₃ Alkyl-Aryl; R₁₀ ausgwählt wird aus -(C=O)NR₅R₆ und -SO₂NR₅R₆, wobei R₅ und R₆ wie vorstehend definiert sind, und -NR₇(C=O)R₈, -NR₇SO₂R₈, -NR₇(C=O)NR₅R₆, -S(O)ₓR₈ und -NR₇(C=O)OR₉, wobei R₇, R_{8,} und R₉ wie vorstehend definiert sind; y 0, 1 oder 2 ist; x 1 oder 2 ist; m 0, 1, 2 oder 3 ist; und die vorstehenden Aryl-Gruppen und die Arylanteile der vorstehenden Alkyl-Aryl-Gruppen unabhängig voneinander ausgewählt werden aus Phenyl und substituiertem Phenyl, wobei das substituierte Phenyl mit einer bis drei Gruppen ausgewählt aus C₁ bis C₄ Alkyl, Halogen, Hydroxy, Cyano, Carboxamido, Nitro und C₁ bis C₄ Alkoxy substituiert sein kann, mit den Maßgaben, daß:
(i) R₃ nicht Wasserstoff oder C₂-C₆ lineares oder verzweigtes Alkyl ist, wenn R₁ und R₂ Wasserstoff sind, n 2 ist und die Verbindung der Formel (I) eine freie Base ist;
(ii) die Verbindung der Formel (I) kein pharmazeutisch verträgliches Säureadditionssalz ist, wenn R₁ und R₂ Wasserstoff sind, R₃ Methyl ist und n 2 ist;
(iii) das pharmazeutisch verträgliche Salz der Verbindung der Formel (I) kein Hydrochlorid oder Pikrat ist, wenn R_{1,} R₂ und R₃ Wasserstoff sind und n 2 ist;
(iv) n nicht 2 ist, wenn R₁ Wasserstoff, R₂ Hydroxy und R₃ Methyl ist und die Verbindung der Formel (I) eine freie Base oder das pharmazeutisch verträgliche Salz das Pikrat oder ½ Oxalat ist; und
(v) n nicht 2 ist, wenn R₁ und R₃ Wasserstoff sind, R₂ Hydroxy ist und die Verbindung der Formel (I) eine freie Base oder das pharmazeutisch verträgliche Salz ein Pikrat oder Hydrogensuccinat ist,
umfassend
(a) Reduzieren einer Verbindung der Formel 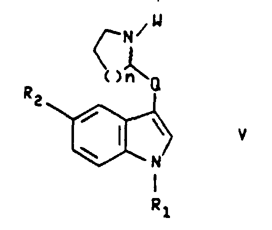 wobei Q CH₂ oder C=O ist, W -CO₂R₁₁ oder R₃ ist und R₁₁ C₁ bis C₆ Alkyl, Benzyl oder Aryl ist und R₁, R₂ und R₁₁ wie vorstehend definiert sind;
(b) wenn R₂ -CH=CH(CH₂)_{y}R₁₀ ist und R₁₀ wie vorstehend definiert ist, Umsetzen einer Verbindung der Formel I, wobei R₃ H oder C₁ bis C₆ lineares oder verzweigtes Alkyl ist und R₂ Halogen ist und R₁ wie vorstehend definiert ist, mit einer Verbindung der Formel CH₂=CH(CH₂)_{y}R₁₀, wobei R₁₀ wie vorstehend definiert ist unter Anwendung einer Übergangsmetallkatalyse; oder
(c) Umsetzen einer Verbindung der Formel I, wobei R₃ Wasserstoff ist und R₁ und R₂ wie vorstehend definiert sind, mit einer Verbindung der Formel R₃-Z, wobei R₃ C₁ bis C₆ lineares oder verzweigtes Alkyl ist und Z Halogen ist und einer Base; und,
wenn gewünscht, Überführen der Verbindung der Formel I in ein pharmazeutisch verträgliches Salz davon.

19. Verfahren nach Anspruch 18, wobei die Verbindung der Formel I ein R-Enantiomer ist.

20. Verfahren nach Anspruch 18, wobei in der Verbindung der Formel I R₁ Wasserstoff ist; R₂ -(CH₂)ₘ-SO₂NHR₅, -(CH₂)ₘ-NHSO₂R₈, -(CH₂)ₘ-SO₂R₈, -(CH₂)ₘ-(C=O)NHR₅ oder -(CH₂)ₘ-NH(C=N)R₈ ist; R₃ Wasserstoff oder Methyl ist; und m, R₅ und R₈ wie in Anspruch 18 definiert sind.

21. Verfahren nach Anspruch 18, wobei die Verbindung der Formel I ausgewählt wird aus:
(R)-5-Methoxy-3-(N-methylpyrrolidin-2-ylmethyl)-1H-indol;
(R)-5-Brom-3-(N-methylpyrrolidin-2-ylmethyl)-1H-indol;
(R)-5-(2-Ethylsulfonylethyl)-3-(N-methylpyrrolidin-2-ylmethyl)-1H-indol;
(R)-5-(2-Methylaminosulfonylethyl)-3-(N-methylpyrrolidin-2-ylmethyl)-1H-indol;
(R)-5-(2-Methylaminosulfonylethyl)-3-(pyrrolidin-2-ylmethyl)-1H-indol;
(R)-5-(Methylaminosulfonylmethyl)-3-(N-methylpyrrolidin-2-ylmethyl)-1H-indol;
(R)-5-Carboxamido-3-(N-methylpyrrolidin-2-ylmethyl)-1H-indol;
(R)-5-(2-Methylsulfonylethyl)-3-(N-methylpyrrolidin-2-ylmethyl)-1H-indol;
(R)-5-(2-Methylsulfonamidoethyl)-3-(N-methylpyrrolidin-2-ylmethyl)-1H-indol;
(R)-5-(2-Aminosulfonylethenyl)-3-(N-methylpyrrolidin-2-ylmethyl)-1H-indol;
(R)-5-(2-Aminosulfonylethyl)-3-(N-methylpyrrolidin-2-ylmethyl)-1H-indol;
(R)-5-(2-N,N-Dimethylaminosulfonylethyl)-3-(N-methylpyrrolidin-2-ylmethyl)-1H-indol;
(R)-5-(2-Phenylsulfonylethyl)-3-(N-methylpyrrolidin-2-ylmethyl)-1H-indolhemisuccinat;
(R)-5-(2-Ethylsufonylethyl)-3-(N-methylpyrrolidin-2-ylmethyl)-1H-indolhemisuccinat;
(R)-5-(2-Phenylsulfonylethyl)-3-(N-methylpyrrolidin-2-ylmethyl)-1H-indol;
(R)-5-(3-Benzolcarbonylaminoprop-1-enyl)-3-(N-methylpyrrolidin-2-ylmethyl)-1H-indol;
(R)-5-(2-(4-Methylphenylsulfonyl)ethyl)-3-(N-methylpyrrolidin-2-ylmethyl)-1H-indol;
(R)-5-(3-Methylsulfonylaminoprop-1-enyl)-3-(N-methylpyrrolidin-2-ylmethyl)-1H-indol;
(R)-5-(2-Ethylsulfonylethyl)-3-(N-2-propylpyrrolidin-2-ylmethyl)-1H-indol;
(R)-5-(2-Ethylsulfonylethyl)-3-(pyrrolidin-2-ylmethyl)-1H-indol;
(R)-5-(2-(4-Methylphenylsulfonyl)ethenyl)-3-(N-methylpyrrolidin-2-ylmethyl)-1H-indol; und
(R)-5-(2-Methylsulfonamidomethyl)-3-(N-methylpyrrolidin-2-ylmethyl)-1H-indol.

22. Verfahren zur Herstellung einer Verbindung der Formel 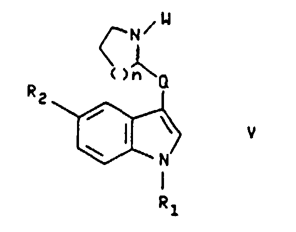 wobei W -CO₂R₁₁ ist; Q C=O ist; n 1 ist; R₁ Wasserstoff ist; R₂ ausgewählt wird aus Wasserstoff, Halogen, Cyano, OR₄, -(CH₂)ₘ-(C=O)NR₅R₆, -(CH₂)ₘ-SO₂NR₅R₆, -(CH₂)ₘ-NR₇(C=O)R₈, -(CH₂)ₘ-NR₇SO₂R₈, -(CH₂)ₘ-S(O)ₓR₈, -(CH₂)ₘ-NR₇(C=O)NR₅R₆, -(CH₂)ₘ-NR₇(C=O)OR₉ und -CH=CH(CH₂)_{y}R₁₀; x 1 oder 2 ist; m 0, 1, 2 oder 3 ist; R₄ ausgewählt wird aus Wasserstoff, C₁ bis C₆ Alkyl und Aryl, R₅ und R₆ unabhängig voneinander ausgewählt werden aus Wasserstoff, C₁ bis C₆ Alkyl, Aryl und C₁ bis C₃ Alkyl-Aryl oder R₅ und R₆ zusammengenommen einen 4-, 5- oder 6-gliedrigen Ring bilden; R₇ und R₈ unabhängig voneinander ausgewählt werden aus Wasserstoff, C₁ bis C₆ Alkyl, Aryl und C₁ bis C₃ Alkyl-Aryl; R₉ ausgewählt wird aus Wasserstoff, C₁ bis C₆ Alkyl, Aryl und C₁ bis C₃ Alkyl-Aryl; R₁₀ ausgwählt wird aus -(C=O)NR₅R₆ und -SO₂NR₅R₆, wobei R₅ und R₆ wie vorstehend definiert sind, und -NR₇(C=O)R₈, -NR₇SO₂R₈, -NR₇(C=O)NR₅R₆, -S(O)ₓR₈ und -NR₇(C=O)OR₉, wobei R₇, R_{8,} R₉ und x wie vorstehend definiert sind; y 0, 1 oder 2 ist; R₁₁ ausgewählt wird aus C₁ bis C₆ Alkyl, Benzyl und Aryl; und die vorstehenden Aryl-Gruppen und die Arylanteile der vorstehenden Alkyl-Aryl-Gruppen unabhängig voneinander ausgewählt werden aus Phenyl und substituiertem Phenyl, wobei das substituierte Phenyl mit einer bis drei Gruppen ausgewählt aus C₁ bis C₄ Alkyl, Halogen, Hydroxy, Cyano, Carboxamido, Nitro und C₁ bis C₄ Alkoxy substituiert sein kann, mit der Maßgabe, daß R₁₁ nicht Benzyl ist, wenn R₁ und R₂ Wasserstoff sind, umfassend das Umsetzen einer Verbindung der Formel 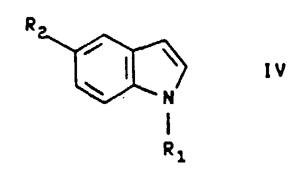 wobei R₁ und R₂ wie vorstehend definiert sind, mit einem Säurechlorid einer Verbindung der Formel N-CO₂R₁₁-Prolin mit einer Base.

23. Verfahren nach Anspruch 22, wobei die Verbindung der Formel V ein R-Enantiomer ist.

24. Verfahren nach Anspruch 22 oder 23, wobei in der Verbindung der Formel V R₁ Wasserstoff ist; R₂ -(CH₂)ₘ-SO₂NHR₅, -(CH₂)ₘ-NHSO₂R₈, -(CH₂)ₘ-SO₂R₈, -(CH₂)ₘ-(C=O)NHR₅ oder -(CH₂)ₘ-NH(C=O)R₈ ist; m 0, 1, 2 oder 3 ist; R₅ Wasserstoff, C₁ bis C₆ Alkyl, Aryl oder C₁ bis C₃ Alkyl-Aryl ist; R₁₁ ausgewählt wird aus C₁ bis C₆ Alkyl, Benzyl und Aryl; und die vorstehenden Aryl-Gruppen und die Aryl-Anteile der vorstehenden Alkyl-Aryl-Gruppen unabhängig voneinander ausgewählt werden aus Phenyl und substituiertem Phenyl, wobei das substituierte Phenyl mit einer bis drei Gruppen ausgewählt aus C₁ bis C₄ Alkyl, Halogen, Hydroxy, Cyano, Carboxamido, Nitro, und C₁ bis C₄ Alkoxy substituiert sein kann.

25. Verfahren zur Herstellung einer Verbindung der Formel: 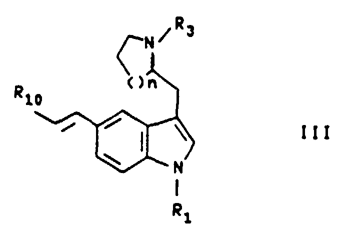 wobei R₁ und R₁₀ wie in Anspruch 22 definiert sind, n 0, 1 oder 2 ist und R₃ ausgewählt wird aus Wasserstoff und C₁ bis C₆ linearem oder verzweigtem Alkyl, umfassend das Umsetzen einer Verbindung der Formel 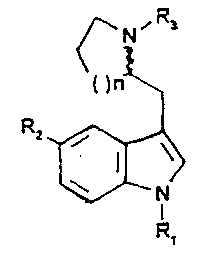 wobei R₂ Halogen ist und R₁, R₃ und n wie vorstehend definiert sind, mit einer Verbindung der Formel
CH₂=CHR₁₀
wobei R₁₀ wie vorstehend definiert ist, unter Anwendung einer Übergangsmetallkatalyse.

26. Verfahren nach Anspruch 25, wobei die Verbindung der Formel III ein R-Enantiomer ist.

27. Verfahren nach Anspruch 25 oder 26, wobei in der Verbindung der Formel III R₁ Wasserstoff ist; R₃ Wasserstoff oder Methyl ist; und R₁₀ -SO₂NHR₅, NHSO₂R₈, -SO₂R₈, -(C=O)NHR₅ oder -NH(C=O)R₈ ist, wobei R₅ und R₈ wie in Anspruch 22 definiert sind.

28. Verbindung nach Anspruch 1, wobei die Verbindung (R)-5-(Methylaminosulfonylmethyl)-3-(N-methylpyrrolidin-2-ylmethyl)-1H-indol ist.

29. Verbindung nach Anspruch 1, wobei die Verbindung (R)-5-(2-Phenylsulfonylethyl)-3-(N-methylpyrrolidin-2-ylmethyl)-1H-indol ist.

30. Verbindung nach Anspruch 1, wobei die Verbindung (R)-5-(2-Phenylsulfonylethyl)-3-(N-methylpyrrolidin-2-ylmethyl)-1H-indolhemisuccinat ist.

31. Verbindung nach Anspruch 1, wobei die Verbindung (R)-5-(Methylaminosulfonylmethyl)-3-(pyrrolidin-2-ylmethyl)-1H-indol ist.

32. Zusammensetzung nach Anspruch 5 oder 6, wobei die Verbindung der Formel (I) eine Verbindung nach einem der Ansprüche 28 bis 31 ist.

33. Verbindung zur Verwendung als Medikament nach Anspruch 7, wobei die Verbindung der Formel (I) eine Verbindung nach einem der Ansprüche 28 bis 31 ist.

34. Verwendung nach Anspruch 8 oder 9, wobei die Verbindung der Formel (I) eine Verbindung nach einem der Ansprüche 28 bis 31 ist.

35. Verfahren nach Anspruch 18, wobei die Verbindung der Formel (I) (R)-5-(Methylaminosulfonylmethyl)-3-(N-methylpyrrolidin-2-ylmethyl)-1H-indol ist.

36. Verfahren nach Anspruch 18, wobei die Verbindung der Formel (I) (R)-5-(2-Phenylsulfonylethyl)-3-(N-methylpyrrolidin-2-ylmethyl)-1H-indol ist.

37. Verfahren nach Anspruch 18, wobei die Verbindung der Formel (I) (R)-5-(2-Phenylsulfonylethyl)-3-(N-methylpyrrolidin-2-ylmethyl)-1H-indolhemisuccinat ist.

38. Verfahren nach Anspruch 18, wobei die Verbindung der Formel (I) (R)-5-(Methylaminosulfonylmethyl)-3-(pyrrolidin-2-ylmethyl)-1H-indol ist.

## Revendications

1. Composé de formule 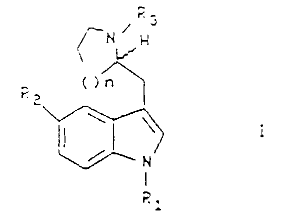 dans laquelle n a la valeur 0, 1 ou 2 ; R₁ représente de l'hydrogène ; R₂ est choisi entre l'hydrogène, un halogène, des groupes cyano, OR₄, -(CH₂)ₘ-(C=O) NR₅R₆, - (CH₂)ₘ-SO₂NR₅R₆, -(CH₂)ₘ-NR₇(C=O) R₈, (CH₂)ₘ-NR₇SO₂R₈, - (CH₂)ₘ-S(O)ₓR₈, -(CH₂)ₘ-NR₇(C=O)NR₅R₆, -(CH₂)ₘ-NR₇(C=O)OR₉ et -CH=CH(CH₂)_{y}R₁₀ ; R₃ est choisi entre l'hydrogène et un groupe alkyle linéaire ou ramifié en C₁ à C₆ ; R₄ est choisi entre l'hydrogène, un groupe alkyle en C₁ à C₆ et un groupe aryle ; R₅ et R₆ sont choisis indépendamment entre l'hydrogène, des groupes alkyle en C₁ à C₆, aryle et (alkyle en C₁ à C₃)-aryle ou bien R₅ et R₆ forment ensemble un noyau à 4, 5 ou 6 chaînons ; R₇ et R₈ sont choisis indépendamment entre l'hydrogène, des groupes alkyle en C₁ à C₆, aryle et (alkyle en C₁ à C₃)-aryle ; R₉ est choisi entre l'hydrogène, des groupes alkyle en C₁ à C₆, aryle et (alkyle en C₁ à C₃)-aryle ; R₁₀ est choisi entre des groupes -(C=O)NR₅R₆ et -SO₂NR₅R₆ où R₅ et R₆ sont tels que définis ci-dessus, et des groupes -NR₇(C=O)R₈, -NR₇SO₂R₈, -NR₇(C=O)NR₅R₆, -S(O)ₓR₈ et -NR₇(C=O)OR₉, dans lesquels R₇, R₈ et R₉ sont tels que définis ci-dessus ; y a la valeur 0, 1 ou 2 ; x a la valeur 1 ou 2 ; m a la valeur 0, 1, 2 ou 3 ; et les groupes aryle ci-dessus et les fragments aryle des groupes alkylaryle ci-dessus sont choisis, indépendamment, entre un groupe phényle et un groupe phényle substitué, le groupe phényle substitué pouvant porter comme substituants un à trois groupes choisis entre des groupes alkyle en C₁ à C₄, halogéno, hydroxy, cyano, carboxamido, nitro et alkoxy en C₁ à C₄, et leurs sels acceptables du point de vue pharmaceutique, sous réserve que :
(i) lorsque R₁ et R₂ sont de l'hydrogène et n est égal à 2, R₃ ne soit pas de l'hydrogène ou un groupe alkyle linéaire ou ramifié en C₁ à C₆ lorsque le composé de formule (I) est une base libre ;
(ii) lorsque R₁ et R₂ sont de l'hydrogène, R₃ est un groupe méthyle et n est égal à 2, le composé de formule (I) ne soit pas un sel d'addition d'acide acceptable du point de vue pharmaceutique ;
(iii) lorsque R₁, R₂ et R₃ sont de l'hydrogène et n est égal à 2, le sel acceptable du point de vue pharmaceutique du composé de formule (I) ne soit pas le chlorhydrate ou le picrate ;
(iv) lorsque R₁ et de l'hydrogène, R₂ est un groupe hydroxy et R₃ est un groupe méthyle, n ne soit pas égal à 2 lorsque le composé de formule (I) est une base libre ou le sel pharmaceutiquement acceptable est le picrate ou l'hémi-oxalate ; et
(v) lorsque R₁ et R₃ sont de l'hydrogène et R₂ est un groupe hydroxy, n ne soit pas égal à 2 lorsque le composé de formule (I) est une base libre ou lorsque le sel pharmaceutiquement acceptable est le picrate ou l'hydrogénosuccinate.

2. L'énantiomère R d'un composé suivant la revendication 1.

3. Composé suivant la revendication 1, dans lequel R₁ est de l'hydrogène ; R₂ est un groupe -(CH₂)ₘ-SO₂NHR₅, -(CH₂)ₘ-NHSO₂R₈, -(CH₂)ₘ-SO₂R₈, -(CH₂)ₘ-(C=O)NHR₅ ou -(CH₂)ₘ-NH(C=O)R₈ ; R₃ est de l'hydrogène ou un groupe méthyle ; et m, R₅ et R₈ sont tels que définis dans la revendication 1.

4. Composé suivant la revendication 1, choisi entre :
le (R)-5-méthoxy-3-(N-méthylpyrrolidine-2-ylméthyl)-1H-indole;
le (R)-5-bromo-3-(N-méthylpyrrolidine-2-yl-méthyl)-1H-indole ;
le (R)-5-(2-éthylsulfonyléthyl)-3-(N-méthyl-pyrrolidine-2-ylméthyl)-1H-indole ;
le (R)-5-(2-méthylaminosulfonyléthyl)-3-(N-méthylpyrrolidine-2-ylméthyl)-1H-indole;
le (R)-5-(2-méthylaminosulfonyléthyl)-3-(pyrrolidine-2-ylméthyl)-1H-indole;
le (R)-5-(méthylaminosulfonylméthyl)-3-(N-méthylpyrrolidine-2-ylméthyl)-1H-indole;
le (R)-5-carboxamido-3-(N-méthylpyrrolidine-2-yl-méthyl)-1H-indole ;
le (R)-5-(2-méthylsulfonyléthyl)-3-(N-méthylpyrrolidine-2-yl-méthyl)-1H-indole;
le (R)-(5-(2-méthylsulfonamidoéthyl)-3-(N-méthylpyrrolidine-2-ylméthyl)-1H-indole;
le (R)-5-(2-aminosulfononyléthényl)-3-(N-méthylpyrrolidine-2-ylméthyl)-1H-indole;
le (R)-5-(2-aminosulfonyléthyl)-3-(N-méthylpyrrolidine-2-ylméthyl)-1H-indole;
le (R)-5-(2-N,N-diméthylaminosulfonyléthyl)-3-(N-méthylpyrrolidine-2-ylméthyl)-1H-indole;
l'hémisuccinate de (R)-(5-(2-phénylsulfonyléthyl)-3-(N-méthylpyrrolidine-2-ylméthyl-1H-indole
l'hémisuccinate de (R)-5-(2-éthylsulfonyléthyl)-3-(N-méthylpyrrolidine-2-ylméthyl)-1H-indole;
le (R)-5-(2-phénylsulfonyléthyl)-3-(N-méthylpyrrolidine-2-ylméthyl)-1H-indole;
le (R)-5-(3-benzènecarbonylaminoprop-1-ényl)-3-(N-méthylpyrrolidine-2-ylméthyl)-1H-indole;
le (R)-5-(2-(4-méthylphénylsulfonyl)éthyl)-3-(N-méthylpyrrolidine-2-ylméthyl)-1H-indole;
le (R)-5-(3-méthylsulfonylaminoprop-1-ényl)-3-(N-méthylpyrrolidine-2-ylméthyl)-1H-indole ;
le (R)-5-(2-éthylsulfonyléthyl)-3-(N-2-propyl-pyrrolidine-2-ylméthyl)-1H-indole;
le (R)-5-(2-éthylsulfonyléthyl)-3-(pyrrolidine-2-ylméthyl)-1H-indole;
le (R)-5-(2-(4-méthylphénylsulfonyl)éthényl)-3-(N-méthylpyrrolidine-2-ylméthyl)-1H-indole; et
le (R)-5-(2-méthylsulfonamidométhyl)-3-(N-méthylpyrrolidine-2-ylméthyl)-1H-indole.

5. Composition pharmaceutique destinée au traitement d'une affection choisie entre l'hypertension, la dépression, l'anxiété, des troubles de l'alimentation, l'obésité, l'abus de médicaments, la céphalée vasculaire de Horton, la migraine, la douleur et l'hémicrânie paroxystique chronique et la céphalée associée à des troubles vasculaires, comprenant une quantité d'un composé suivant l'une quelconque des revendications 1 à 4 efficace pour le traitement d'une telle affection et un véhicule acceptable du point de vue pharmaceutique.

6. Composition pharmaceutique destinée au traitement de troubles provenant de la déficience de la neurotransmission sérotoninergique, comprenant une quantité d'un composé suivant l'une quelconque des revendications 1 à 4 efficace pour le traitement d'un tel trouble et un véhicule acceptable du point de vue pharmaceutique.

7. Composé de formule (I) ou sel pharmaceutiquement acceptable de ce composé ou composition le contenant, suivant l'une quelconque des revendications 1 à 4 et respectivement 5 et 6, destiné à être utilisé comme médicament.

8. Utilisation d'un composé de formule (I) ou d'un sel pharmaceutiquement acceptable de ce composé ou d'une composition le contenant suivant l'une quelconque des revendications 1 à 4 et respectivement 5 et 6, pour la préparation d'un médicament destiné au traitement d'une affection choisie entre l'hypertension, la dépression, l'anxiété, des troubles d'alimentation, l'obésité, l'abus de médicaments, la céphalée vasculaire de Horton, la migraine, la douleur et l'hémicrânie paroxystique chronique et la céphalée associée à des troubles vasculaires.

9. Utilisation d'un composé de formule (I) ou d'un sel pharmaceutiquement acceptable de ce composé ou d'une composition le contenant, suivant l'une quelconque des revendications 1 à 4 et respectivement 5 et 6, pour la préparation d'un médicament destiné au traitement de troubles résultant d'une déficience de la neurotransmission sérotoninergique.

10. Composé de formule 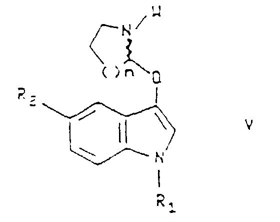 dans laquelle W est un groupe -CO₂R₁₁ ou R₃ ; Q est un groupe CH₂ ou C=O ; n a la valeur 0, 1 ou 2 ; R₁ est de l'hydrogène ; R₂ est choisi entre l'hydrogène, un halogène, des groupes cyano, OR₄, -(CH₂)ₘ-(C=O)NR₅R₆, -(CH₂)ₘ-SO₂NR₅R₆, -(CH₂)ₘ-NR₇(C=O)R₈, -(CH₂)ₘ-NR₇SO₂R₈, -(CH₂)ₘ-S(O)ₓR_{8,} -(CH₂)ₘ-NR₇(C=O)NR₅R₆, -(CH₂)ₘ-NR₇(C=O)OR₉ et -CH=CH(CH₂)_{y}R₁₀ ; x a la valeur 1 ou 2 ; m a la valeur 0, 1, 2 ou 3 ; R₃ est choisi entre l'hydrogène et un groupe alkyle en C₁ à C₆ linéaire ou ramifié ; R₄ est choisi entre l'hydrogène, un groupe alkyle en C₁ à C₆, un groupe aryle, R₅ et R₆ sont choisis indépendamment entre l'hydrogène, un groupe alkyle en C₁ à C₆, aryle et (alkyle en C₁ à C₃)-aryle ou bien R₅ et R₆ forment conjointement un noyau de 4, 5 ou 6 chaînons ; R₇ et R₈ sont choisis indépendamment entre l'hydrogène, un groupe alkyle en C₁ à C₆, aryle et (alkyle en C₁ à C₃)-aryle ; R₉ est choisi entre l'hydrogène, des groupes alkyle en C₁ à C₆, aryle et (alkyle en C₁ à C₃)-aryle ; R₁₀ est choisi entre des groupes -(C=O)NR₅R₆ et -SO₂NR₅R₆, où R₅ et R₆ sont tels que définis ci-dessus, et -NR₇(C=O)R₈, -NR₇SO₂R₈, -NR₇(C=O) NR₅R₆, -S(O)ₓR₈ et -NR₇(C=O)OR₉, où R₇, R₈, R₉ et x sont tels que définis ci-dessus ; y a la valeur 0, 1 ou 2 ; R₁₁ est choisi entre des groupes alkyle en C₁ à C₅, benzyle et aryle ; et les groupes aryle ci-dessus et les fragments aryle des groupes alkyl-aryle ci-dessus sont choisis indépendamment entre un groupe phényle et un groupe phényle substitué, le groupe phényle substitué pouvant porter un à trois groupes substituants choisis entre des groupes alkyle en C₁ à C₄, halogéno, hydroxy, cyano, carboxamido, nitro et alkoxy en C₁ à C₄, sous réserve que :
(i) lorsque Q est un groupe CH₂, W ne représente pas R₃ ;
(ii) lorsque R₁ et R₂ sont de l'hydrogène, Q est un groupe C=O et n est égal à 2, W ne soit pas de l'hydrogène ou un groupe néopentyle ; et
(iii) lorsque R₁ et R₂ sont de l'hydrogène, Q est un groupe C=O et n est égal à 1, W ne soit pas de l'hydrogène ou un groupe benzyloxycarbonyle.

11. L'énantiomère R d'un composé suivant la revendication 10.

12. Composé suivant la revendication 10, qui est un composé de formule 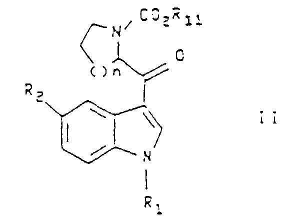 dans laquelle n, R₁, R₂ et R₁₁ sont tels que définis dans la revendication 10.

13. Enantiomère R d'un composé suivant la revendication 12.

14. Composé suivant la revendication 12, dans lequel R₁ est de l'hydrogène ; R₂ est un groupe -(CH₂)ₘ-SO₂NHR₅, -(CH₂)ₘ-NHSO₂R₈, -(CH₂)ₘ-SO₂R₈, -(CH₂)ₘ-(C=O)NHR₅ ou -(CH₂)ₘ-NH(C=O)R₈ ; m a la valeur 0, 1, 2 ou 3 ; R₅ est de l'hydrogène, un groupe alkyle en C₁ à C₆, aryle ou (alkyle en C₁ à C₃)-aryle ; R₁₁ est choisi entre des groupes alkyle en C₁ à C₅, benzyle et aryle ; et les groupes aryle ci-dessus et les fragments aryle des groupes alkylaryle ci-dessus sont choisis indépendamment entre un groupe phényle et un groupe phényle substitué, le groupe phényle substitué portant 1 à 3 groupes substituants choisis entre les groupes alkyle en C₁ à C₄, halogéno, hydroxy, cyano, carboxamido, nitro et alkoxy en C₁ à C₄.

15. Composé suivant la revendication 1, qui est un composé de formule 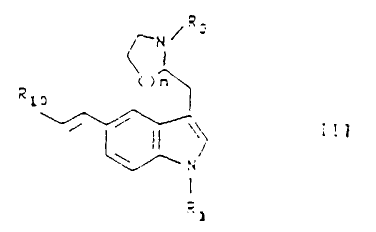 dans laquelle n, R₁, R₃ et R₁₀ sont tels que définis dans la revendication 1.

16. L'énantiomère R d'un composé suivant la revendication 15.

17. Composé suivant la revendication 15, dans lequel R₁ est de l'hydrogène ; R₃ est de l'hydrogène ou un groupe méthyle ; et R₁₀ est un groupe -SO₂NHR₅, NHSO₂R₈, -SO₂R₈, -(C=O)NHR₅ ou -NH(C=O)R₈, où R₅ et R₈ sont tels que définis dans la revendication 1.

18. Procédé de production d'un composé de formule 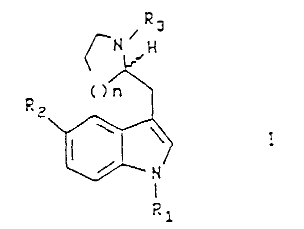 dans laquelle n a la valeur 0, 1 ou 2 ; R₁ représente de l'hydrogène ; R₂ est choisi entre l'hydrogène, un halogène, des groupes cyano, OR₄, -(CH₂)ₘ-(C=O)NR₅R₆, -(CH₂)ₘ-SO₂NR₅R₆ ; -(CH₂)ₘ-NR₇(C=O)R₈, -(CH₂)ₘNR₇SO₂R₈, -(CH₂)ₘ-S(O)ₓR₈, -(CH₂)ₘ-NR₇(C=O)NR₅R₆, -(CH₂)ₘ-NR₇(C=O)OR₉ et -CH=CH(CH₂)_{y}R₁₀ ; R₃ est choisi entre l'hydrogène et un groupe alkyle linéaire ou ramifié en C₁ à C₆ ; R₄ est choisi entre l'hydrogène, un groupe alkyle en C₁ à C₆ et un groupe aryle ; R₅ et R₆ sont choisis indépendamment entre l'hydrogène, des groupes alkyle en C₁ à C₆, aryle et (alkyle en C₁ à C₃)-aryle ou bien R₅ et R₆ forment ensemble un noyau à 4, 5 ou 6 chaînons ; R₇ et R₈ sont choisis indépendamment entre l'hydrogène, des groupes alkyle en C₁ à C₅, aryle et (alkyle en C₁ à C₃)-aryle ; R₆ est choisi entre l'hydrogène, des groupes alkyle en C₁ à C₆, aryle et (alkyle en C₁ à C₃)-aryle ; R₁₀ est choisi entre des groupes -(C=O)NR₅R₆ et -SO₂NR₅R₆, où R₅ et R₆ sont tels que définis ci-dessus, et des groupes -NR₇(C=O) R₈, -NR₇SO₂R₈, -NR₇(C=O) NR₅R₆, -S(O)ₓR₈ et -NR₇(C=O)OR₉, dans lesquels R₇, R₈ et R₉ sont tels que définis ci-dessus ; y a la valeur 0, 1 ou 2 ; x a la valeur 1 ou 2 ; m a la valeur 0, 1, 2 ou 3 ; et les groupes aryle ci-dessus et les fragments aryle des groupes alkylaryle ci-dessus sont choisis, indépendamment, entre un groupe phényle et un groupe phényle substitué, le groupe phényle substitué pouvant porter comme substituants un à trois groupes choisis entre des groupes alkyle en C₁ à C₄, halogéno, hydroxy, cyano, carboxamido, nitro et alkoxy en C₁ à C₄, sous réserve que :
(i) lorsque R₁ et R₂ sont de l'hydrogène et n est égal à 2, R₃ ne soit pas de l'hydrogène ou un groupe alkyle linéaire ou ramifié en C₁ à C₆ lorsque le composé de formule (I) est une base libre ;
(ii) lorsque R₁ et R₂ sont de l'hydrogène, R₃ est un groupe méthyle et n est égal à 2, le composé de formule (I) ne soit pas un sel d'addition d'acide acceptable du point de vue pharmaceutique ;
(iii) lorsque R₁, R₂ et R₃ sont de l'hydrogène et n est égal à 2, le sel acceptable du point de vue pharmaceutique du composé de formule (I) ne soit pas le chlorhydrate ou le picrate ;
(iv) lorsque R₁ et de l'hydrogène, R₂ est un groupe hydroxy et R₃ est un groupe méthyle, n ne soit pas égal à 2 lorsque le composé de formule (I) est une base libre ou le sel pharmaceutiquement acceptable est le picrate ou l'hémi-oxalate ; et
(v) lorsque R₁ et R₃ sont de l'hydrogène et R₂ est un groupe hydroxy, n ne soit pas égal à 2 lorsque le composé de formule (I) est une base libre ou lorsque le sel pharmaceutiquement acceptable est le picrate ou l'hydrogénosuccinate,
comprenant
(a) la réduction d'un composé de formule 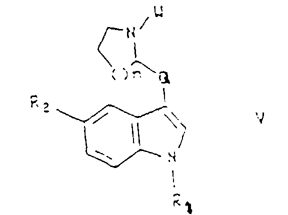 dans laquelle Q est un groupe CH₂ ou C=O, W est un groupe -CO₂R₁₁ ou R₃ et R₁₁ est un groupe alkyle en C₁ à C₆, benzyle ou aryle, et R₁, R₂ et R₁₁ sont tels que définis ci-dessus ;
(b) lorsque R₂ est un groupe -CH=CH(CH₂)_{y}R₁₀ et R₁₀ est tel que défini ci-dessus, la réaction d'un composé de formule (I) dans laquelle R₃ représente H ou un groupe alkyle linéaire ou ramifié en C₁ à C₆ et R₃ est un halogène, et R₁ est tel que défini ci-dessus, avec un composé de formule CH₂=CH(CH₂)_{y}R₁₀ dans laquelle R₁₀ est tel que défini ci-dessus, par catalyse avec un métal de transition ; ou bien
(c) la réaction du composé de formule (I) dans laquelle R₃ est de l'hydrogène et R₁ et R₂ sont tels que définis ci-dessus avec un composé de formule R₃-Z dans laquelle R₃ est un groupe alkyle linéaire ou ramifié en C₁ à C₆ et Z est un halogène, et une base ; et
le cas échéant, la transformation d'un composé de formule I en un sel pharmaceutiquement acceptable de ce composé.

19. Procédé suivant la revendication 18, dans lequel le composé de formule (I) est un R-énantiomère.

20. Procédé suivant la revendication 18, dans lequel, pour le composé de formule I, R₁ est de l'hydrogène ; R₂ est un groupe -(CH₂)ₘ-SO₂NHR₅, -(CH₂)ₘ-NHSO₂R₈, -(CH₂)ₘ-SO₂R₈, -(CH₂)ₘ-(C=O)NHR₅ ou -(CH₂)ₘ-NH(C=O)R₈; R₃ est de l'hydrogène ou un groupe méthyle ; et m, R₅ et R₈ sont tels que définis dans la revendication 18.

21. Procédé suivant la revendication 18, dans lequel le composé de formule (I) est choisi entre :
le (R)-5-méthoxy-3-(N-méthylpyrrolidine-2-ylméthyl)-1H-indole;
le (R)-5-bromo-3-(N-méthylpyrrolidine-2-yl-méthyl)-1H-indole;
le (R)-5-(2-éthylsulfonyléthyl)-3-(N-méthylpyrrolidine-2-ylméthyl)-1H-indole;
le (R)-5-(2-méthylaminosulfonyléthyl)-3-(N-méthylpyrrolidine-2-ylméthyl)-1H-indole;
le (R)-5-(2-méthylaminosulfonyléthyl)-3-(pyrrolidine-2-ylméthyl)-1H-indole;
le (R)-5-(méthylaminosulfonylméthyl)-3-(N-méthylpyrrolidine-2-ylméthyl)-1H-indole;
le (R)-5-carboxamido-3-(N-méthylpyrrolidine-2-yl-méthyl)-1H-indole;
le (R)-5-(2-méthylsulfonyléthyl)-3-(N-méthylpyrrolidine-2-yl-méthyl)-1H-indole;
le (R)-(5-(2-méthylsulfonamidoéthyl)-3-(N-méthylpyrrolidine-2-ylméthyl)-1H-indole;
le (R)-5-(2-aminosulfonyléthényl)-3-(N-méthylpyrrolidine-2-ylméthyl)-1H-indole;
le (R)-5-(2-aminosulfonyléthyl)-3-(N-méthylpyrrolidine-2-ylméthyl)-1H-indole;
le (R)-5-(2-N,N-diméthylaminosulfonyléthyl)-3-(N-méthylpyrrolidine-2-ylméthyl)-1H-indole;
l'hémisuccinate de (R)-(5-(2-phénylsulfononyléthyl)-3-(N-méthylpyrrolidine-2-ylméthyl)-1H-indole;
l'hémisuccinate de (R)-5-(2-éthylsulfonyléthyl)-3-(N-méthylpyrroidine-2-ylméthyl)-1H-indole;
le (R)-5-(2-phénylsulfonyléthyl)-3-(N-méthylpyrrolidine-2-ylméthyl)-1H-indole;
le (R)-5-(3-benzènecarbonylaminoprop-1-ényl)-3-(N-méthylpyrrolidine-2-ylméthyl)-1H-indole;
le (R)-5-(2-(4-méthylphénylsulfonyl)éthyl)-3-(N-méthylpyrrolidine-2-ylméthyl)-1H-indole;
le (R)-5-(3-méthylsulfonylaminoprop-1-ényl)-3-(N-méthylpyrrolidine-2-ylméthyl)-1H-indole;
le (R)-5-(2-éthylsulfonyléthyl)-3-(N-2-propylpyrrolidine-2-ylméthyl)-1H-indole;
le (R)-5-(2-éthylsulfonyléthyl)-3-(pyrrolidine-2-ylméthyl)-1H-indole;
le (R)-5-(2-(4-méthylphénylsulfonyl)éthényl)-3-(N-méthylpyrrolidine-2-ylméthyl)-1H-indole; et
le (R)-5-(2-méthylsulfonamidométhyl)-3-(N-méthylpyrrolidine-2-ylméthyl)-1H-indole.

22. Procédé de production d'un composé de formule 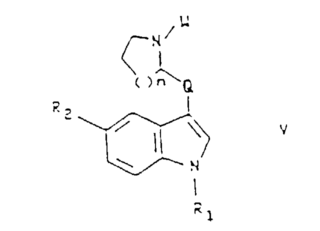 dans laquelle W est un groupe -CO₂R₁₁ ou R₃ ; Q est un groupe C=O ; n = 1 ; R₁ est de l'hydrogène ; R₂ est choisi entre l'hydrogène, un halogène, des groupes cyano, OR₄, -(CH₂)ₘ-(C=O)NR₅R₆, -(CH₂)ₘ-SO₂NR₅R₆, -(CH₂)ₘ-NR₇(C=O)R₈, -(CH₂)ₘ-NR₇SO₂R₈, -(CH₂)ₘ-S(O)ₓR₈, -(CH₂)ₘ-NR₇(C=O)NR₅R₆, -(CH₂)ₘ-NR₇(C=O)OR₉ et -CH=CH(CH₂)_{y}R₁₀ ; x a la valeur 1 ou 2 ; m a la valeur 0, 1, 2 ou 3 ; R₄ est choisi entre l'hydrogène et un groupe alkyle en C₁ à C₆ et un groupe aryle, R₅ et R₆ sont choisis indépendamment entre l'hydrogène, un groupe alkyle en C₁ à C₆, aryle et (alkyle en C₁ à C₃)-aryle ou bien R₅ et R₆ forment conjointement un noyau de 4, 5 ou 6 chaînons ; R₇ et R₈ sont choisis indépendamment entre l'hydrogène, un groupe alkyle en C₁ à C₆, aryle et (alkyle en C₁ à C₃)aryle ; R₉ est choisi entre l'hydrogène, des groupes alkyle en C₁ à C₆, aryle et (alkyle en C₁ à C₃)-aryle ; R₁₀ est choisi entre des groupes -(C=O)NR₅R₆ et -SO₂NR₅R₆, où R₅ et R₆ sont tels que définis ci-dessus, et -NR₇(C=O)R₈, -NR₇SO₂R₈, -NR₇(C=O)NR₅R₆, -S(O)ₓR₈ et -NR₇(C=O)OR₉, où R₇, R₈, R₉ et x sont tels que définis ci-dessus ; y a la valeur 0, 1 ou 2 ; R₁₁ est choisi entre des groupes alkyle en C₁ à C₆, benzyle et aryle ; et les groupes aryle ci-dessus et les fragments aryle des groupes alkyl-aryle ci-dessus sont choisis indépendamment entre un groupe phényle et un groupe phényle substitué, le groupe phényle substitué pouvant porter un à trois groupes substituants choisis entre des groupes alkyle en C₁ à C₄, halogéno, hydroxy, cyano, carboxamido, nitro et alkoxy en C₁ à C₄, sous réserve que R₁ et R₂ sont de l'hydrogène, R₁₁ ne soit pas un groupe benzyle, comprenant la réaction d'un composé de formule 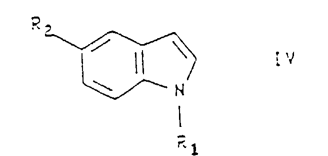 dans laquelle R₁ et R₂ sont tels que définis ci-dessus, avec un chlorure d'acide d'un composé de formule N-CO₂R₁₁-proline avec une base.

23. Procédé suivant la revendication 22, dans lequel le composé de formule V est un R-énantiomère.

24. Procédé suivant la revendication 22 ou 23, dans lequel, pour le composé de formule V, R₁ est l'hydrogène ; R₂ est un groupe -(CH₂)ₘ-SO₂NHR₅, -(CH₂)ₘNHSO₂R₈, -(CH₂)ₘ-SO₂R₈, -(CH₂)ₘ-(C=O) ou NHR₅ ou -(CH₂)ₘ-NH(C=O)R₈ ; m a la valeur 0, 1, 2 ou 3 ; R₅ est de l'hydrogène, un groupe alkyle en C₁ à C₅, aryle ou (alkyle en C₁ à C₃)-aryle ; R₁₁ est choisi entre des groupes alkyle en C₁ à C₆, benzyle et aryle ; et les groupes aryle ci-dessus et les fragments aryle des groupes alkylaryle ci-dessus sont choisis indépendamment entre un groupe phényle et un groupe phényle substitué, ce groupe phényle substitué pouvant porter un à trois groupes substituants choisis entre des groupes alkyle en C₁ à C₄, halogéno, hydroxy, cyano, carboxamido, nitro et alkoxy en C₁ à C₄.

25. Procédé de production d'un composé de formule 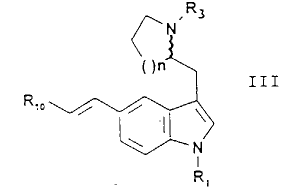 dans laquelle R₁ et R₁₀ sont tels que définis dans la revendication 22, n a la valeur 0, 1 ou 2 et R₃ est choisi entre l'hydrogène et un groupe alkyle linéaire ou ramifié en C₁ à C₆, comprenant la réaction d'un composé de formule 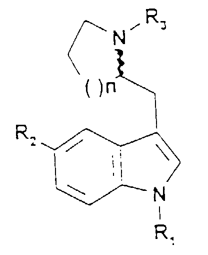 dans laquelle R₂ est un halogène et R₁, R₃ et n sont tels que définis ci-dessus, avec un composé de formule :
CH₂=CHR₁₀
dans laquelle R₁₀ est tel que défini ci-dessus, par catalyse avec un métal de transition.

26. Procédé suivant la revendication 25, dans lequel le composé de formule III est un R-énantiomère.

27. Procédé suivant la revendication 25 ou 26, dans lequel, pour le composé de formule III, R₁ est de l'hydrogène ; R₃ est de l'hydrogène ou un groupe méthyle ; et R₁₀ est un groupe -SO₂NHR₅, NHSO₂R₈, -SO₂R₈, -(C=O)NHR₅ et -NH(C=O)R₈, où R₅ et R₈ sont tels que définis dans la revendication 22.

28. Composé suivant la revendication 1, qui est le (R)-5-(méthylaminosulfonyméthyl)-3-(N-méthylpyrrolidine-2-ylméthyl)-1H-indole.

29. Composé suivant la revendication 1, qui est le (R)-5-(2-phénylsulfonyléthyl)-3-(N-méthylpyrrolidine-2-ylméthyl)-1H-indole.

30. Composé suivant la revendication 1, qui est l'hémi-succinate de (R)-5-(2-phénylsulfonyléthyl)-3-(N-méthylpyrrolidine-2-ylméthyl)-1H-indole.

31. Composé suivant la revendication 1, qui est le (R)-5-(méthylaminosulfonylméthyl)-3-(pyrrodine-2-yl-méthyl)-1H-indole.

32. Composition suivant la revendication 5 ou 6, dans lequel le composé de formule (I) est tel que défini selon l'une quelconque des revendications 28 à 31.

33. Composé destiné à être utilisé comme médicament suivant la revendication 7, dans lequel le composé de formule (I) est tel que défini dans l'une quelconque des revendications 28 à 31.

34. Utilisation suivant la revendication 8 ou 9, dans laquelle le composé de formule (I) est tel que défini dans l'une quelconque des revendications 28 à 31.

35. Procédé suivant la revendication 18, dans lequel le composé de formule (I) est le (R)-5-(méthylaminosulfonylméthyl)-3-(N-méthylpyrrolidine-2-ylméthyl)-1H-indole.

36. Procédé suivant la revendication 18, dans lequel le composé de formule (I) est le (R)-5-(2-phénylsulfonyléthyl)-3-(N-méthylpyrrolidine-2-ylméthyl)-1H-indole.

37. Procédé suivant la revendication 18, dans lequel le composé de formule (I) est l'hémisuccinate de (R)-5-(2-phénylsulfonyléthyl)-3-(N-méthylpyrrolidine-2-ylméthyl)-1H-indole.

38. Procédé suivant la revendication 18, dans lequel le composé de formule (I) est le (R)-5-(méthylaminosulfonylméthyl)-3-pyrrolidine-2-ylméthyl)-1H-indole.
